# EUROPEAN PATENT SPECIFICATION

(11) **EP 2 558 575 B1**
(45) Date of publication and mention of the grant of the patent: **16.12.2015**
(21) Application number: 11769489.3
(22) Date of filing: 13.04.2011
(51) Int. Cl.: C07K 14/47, G01N 33/58, C12N 15/90, G01N 33/50

(54) **METHODS FOR GENERATING ENDOGENOUSLY TAGGED PROTEIN**
VERFAHREN ZUR ERZEUGUNG EINES ENDOGEN MARKIERTEN PROTEINS
PROCÉDÉ DE GÉNÉRATION D'UNE PROTÉINE MARQUÉE DE MANIÈRE ENDOGÈNE

(30) Priority: 12.01.2011 US 431957 P; 01.11.2010 US 408856 P; 07.10.2010 US 390668 P; 23.07.2010 US 367017 P; 13.04.2010 US 323698 P; 13.04.2010 US 323719 P; 13.04.2010 US 323702 P
(43) Date of publication of application: 20.02.2013
(73) Proprietor: Sigma-Aldrich Co. LLC, St Louis MO 63103 (US)
(72) Inventor: MALKOV, Dmitry, St. Louis Missouri 63103 (US); ZENSER, Nathan, St. Louis Missouri 63103 (US); VASSAR, Deborah, St. Louis Missouri 63103 (US); ZHANG, Fan, St. Louis Missouri 63103 (US); ZHANG, Hongyi, St. Louis Missouri 63103 (US)
(74) Representative: Turner, Rhiannon Rosalind
(86) International application number: PCT/US2011/032218
(87) International publication number: WO 2011/130346

(56) References cited:
- US-A1- 2002 004 491
- US-A1- 2008 305 519
- US-A1- 2010 009 352
- US-A1- 2010 047 805
- US-A1- 2010 047 805
- MILLER JEFFREY C ET AL: "An improved zinc-finger nuclease architecture for highly specific genome editing", NATURE BIOTECHNOLOGY, NATURE PUBLISHING GROUP, NEW YORK, NY, US, vol. 25, no. 7, 1 July 2007 (2007-07-01), pages 778-785, XP002465119, ISSN: 1087-0156, DOI: 10.1038/NBT1319
- URNOV F D ET AL: "Highly efficient endogenous human gene correction using designed zinc-finger nucleases", NATURE: INTERNATIONAL WEEKLY JOURNAL OF SCIENCE, NATURE PUBLISHING GROUP, UNITED KINGDOM, vol. 435, no. 7042, 2 June 2005 (2005-06-02), pages 646-651, XP002411069, ISSN: 0028-0836, DOI: 10.1038/NATURE03556
- PORTEUS M H ET AL: "GENE TARGETING USING ZINC FINGER NUCLEASES", NATURE BIOTECHNOLOGY, NATURE PUBLISHING GROUP, NEW YORK, NY, US, vol. 23, no. 8, 1 August 2005 (2005-08-01) , pages 967-973, XP002467422, ISSN: 1087-0156, DOI: 10.1038/NBT1125

### SEQUENCE LISTING

<110> SIGMA-ALDRICH CO.
   MALKOV, Dmitry
   ZENSER, Nathan
   VASSAR, Deborah L
   ZHANG, Fan
   ZHANG, Hongyi
<120> METHODS FOR GENERATING ENDOGENOUSLY TAGGED PROTEIN
<130> 047497-424729
<150> US 61/323,702
   <151> 2010-04-13
<150> US 61/323,719
   <151> 2010-04-13
<150> US 61/323,698
   <151> 2010-04-13
<150> US 61/367,017
   <151> 2010-07-23
<150> US 61/390,668
   <151> 2010-10-07
<150> US 61/408,856
   <151> 2010-11-01
<150> US 61/431,957
   <151> 2011-01-12
<160> 29
<170> PatentIn version 3.5
<210> 1
   <211> 15
   <212> DNA
   <213> Homo sapiens
<400> 1
   cttcgcctcc taatc 15
<210> 2
   <211> 15
   <212> DNA
   <213> Homo sapiens
<400> 2
   cactatggtg agtaa 15
<210> 3
   <211> 80
   <212> DNA
   <213> Homo sapiens
<400> 3
   cacaccaaca actgcccacc tcacaggaga aggaggaaga ggagggcatc tcgcaggagt 60
   cctcggagga ggagcagtga 80
<210> 4
   <211> 1197
   <212> DNA
   <213> Artificial Sequence
<220>
   <223> SYNTHESIZED
<400> 4
   ccgcccctcc gcagccgcta cttaagaggc tccagcgccg gccccgccct agtgcgttac 60
   ttacctcgac tcttagcttg tcggggacgg taaccgggac ccggtgtctg ctcctgtcgc 120
   cttcgcctcc taatccctag ccactatggg taccgcaagc gggggcgagg agctgttcgc 180
   cggcatcgtg cccgtgctga tcgagctgga cggcgacgtg cacggccaca agttcagcgt 240
   gcgcggcgag ggcgagggcg acgccgacta cggcaagctg gagatcaagt tcatctgcac 300
   caccggcaag ctgcccgtgc cctggcccac cctggtgacc accctctgct acggcatcca 360
   gtgcttcgcc cgctaccccg agcacatgaa gatgaacgac ttcttcaaga gcgccatgcc 420
   cgagggctac atccaggagc gcaccatcca gttccaggac gacggcaagt acaagacccg 480
   cggcgaggtg aagttcgagg gcgacaccct ggtgaaccgc atcgagctga agggcaagga 540
   cttcaaggag gacggcaaca tcctgggcca caagctggag tacagcttca acagccacaa 600
   cgtgtacatc cgccccgaca aggccaacaa cggcctggag gctaacttca agacccgcca 660
   caacatcgag ggcggcggcg tgcagctggc cgaccactac cagaccaacg tgcccctggg 720
   cgacggcccc gtgctgatcc ccatcaacca ctacctgagc actcagacca agatcagcaa 780
   ggaccgcaac gaggcccgcg accacatggt gctcctggag tccttcagcg cctgctgcca 840
   cacccacggc atggacgagc tgtacagggc cgctggggat cccactatgg tgagtaagcc 900
   gtgcggctcc cggctgcttt cagggaagca gggaaaagcg agccggcggg gcgctggggc 960
   cctgtataca gccgggaagg gctggcctca gagccgtccg tttggagggc ggaaaacgag 1020
   gcgagaggcc agggcgggag tggtgagacc tcggtgtgtg taaatagcgg gggcccggaa 1080
   aggtcgaggg gcgccaggat ttcttctcgg actctggaag ggatgggggg ctcgggctgc 1140
   cctccgccgt atccggagct ctcttttgtc gcgtaactgt gtcctgggtg cggtccc 1197
<210> 5
   <211> 1198
   <212> DNA
   <213> Artificial Sequence
<220>
   <223> SYNTHESIZED
<400> 5
   gcccctccgc agccgctact taagaggctc cagcgccggc cccgccctag tgcgttactt 60
   acctcgactc ttagcttgtc ggggacggta accgggaccc ggtgtctgct cctgtcgcct 120
   tcgcctccta atccctagcc actatgggta ccgcagtgtc taagggcgaa gagctgatta      180
   aggagaacat gcacatgaag ctgtacatgg agggcaccgt gaacaaccac cacttcaagt      240
   gcacatccga gggcgaaggc aagccctacg agggcaccca gaccatgaga atcaaggtgg      300
   tcgagggcgg ccctctcccc ttcgccttcg acatcctggc taccagcttc atgtacggca      360
   gcagaacctt catcaaccac acccagggca tccccgactt ctttaagcag tccttccctg      420
   agggcttcac atgggagaga gtcaccacat acgaagacgg gggcgtgctg accgctaccc      480
   aggacaccag cctccaggac ggctgcctca tctacaacgt caagatcaga ggggtgaact      540
   tcccatccaa cggccctgtg atgcagaaga aaacactcgg ctgggaggcc aacaccgaga      600
   tgctgtaccc cgctgacggc ggcctggaag gcagaagcga catggccctg aagctcgtgg      660
   gcgggggcca cctgatctgc aacttcaaga ccacatacag atccaagaaa cccgctaaga      720
   acctcaagat gcccggcgtc tactatgtgg accacagact ggaaagaatc aaggaggccg      780
   acaaagagac ctacgtcgag cagcacgagg tggctgtggc cagatactgc gacctcccta      840
   gcaaactggg gcacaaactt aatgccgctg gggatcccac tatggtgagt aagccgtgcg      900
   gctcccggct gctttcaggg aagcagggaa aagcgagccg gcggggcgct ggggccctgt      960
   atacagccgg gaagggctgg cctcagagcc gtccgtttgg agggcggaaa acgaggcgag     1020
   aggccagggc gggagtggtg agacctcggt gtgtgtaaat agcgggggcc cggaaaggtc     1080
   gaggggcgcc aggatttctt ctcggactct ggaagggatg gggggctcgg gctgccctcc     1140
   gccgtatccg gagctctctt ttgtcgcgta actgtgtcct gggtgcggtc cctcgagt       1198
<210> 6
   <211> 19
   <212> DNA
   <213> Homo sapiens
<400> 6
   cccctccgca gccgctact 19
<210> 7
   <211> 19
   <212> DNA
   <213> Homo sapiens
<400> 7
   cccctccgca gccgctact 19
<210> 8
   <211> 1198
   <212> DNA
   <213> Artificial Sequence
<220>
   <223> SYNTHESIZED
<400> 8
   gcccctccgc agccgctact taagaggctc cagcgccggc cccgccctag tgcgttactt 60
   acctcgactc ttagcttgtc ggggacggta accgggaccc ggtgtctgct cctgtcgcct 120
   tcgcctccta atccctagcc actatgggta ccgcagtgtc taagggcgaa gagctgatta 180
   aggagaacat gcacatgaag ctgtacatgg agggcaccgt gaacaaccac cacttcaagt 240
   gcacatccga gggcgaaggc aagccctacg agggcaccca gaccatgaga atcaaggtgg 300
   tcgagggcgg ccctctcccc ttcgccttcg acatcctggc taccagcttc atgtacggca 360
   gcagaacctt catcaaccac acccagggca tccccgactt ctttaagcag tccttccctg 420
   agggcttcac atgggagaga gtcaccacat acgaagacgg gggcgtgctg accgctaccc 480
   aggacaccag cctccaggac ggctgcctca tctacaacgt caagatcaga ggggtgaact 540
   tcccatccaa cggccctgtg atgcagaaga aaacactcgg ctgggaggcc aacaccgaga 600
   tgctgtaccc cgctgacggc ggcctggaag gcagaagcga catggccctg aagctcgtgg 660
   gcgggggcca cctgatctgc aacttcaaga ccacatacag atccaagaaa cccgctaaga 720
   acctcaagat gcccggcgtc tactatgtgg accacagact ggaaagaatc aaggaggccg 780
   acaaagagac ctacgtcgag cagcacgagg tggctgtggc cagatactgc gacctcccta 840
   gcaaactggg gcacaaactt aatgccgctg gggatcccac tatggtgagt aagccgtgcg 900
   gctcccggct gctttcaggg aagcagggaa aagcgagccg gcggggcgct ggggccctgt 960
   atacagccgg gaagggctgg cctcagagcc gtccgtttgg agggcggaaa acgaggcgag 1020
   aggccagggc gggagtggtg agacctcggt gtgtgtaaat agcgggggcc cggaaaggtc 1080
   gaggggcgcc aggatttctt ctcggactct ggaagggatg gggggctcgg gctgccctcc 1140
   gccgtatccg gagctctctt ttgtcgcgta actgtgtcct gggtgcggtc cctcgagt 1198
<210> 9
   <211> 15
   <212> DNA
   <213> Homo sapiens
<400> 9
   agctacagca gcttg 15
<210> 10
   <211> 15
   <212> DNA
   <213> Homo sapiens
<400> 10
   cggtacctgg agcag 15
<210> 11
   <211> 16
   <212> DNA
   <213> Homo sapiens
<400> 11
   ttcctctctc tcccac 16
<210> 12
   <211> 15
   <212> DNA
   <213> Homo sapiens
<400> 12
   aggaaggatt cgcac 15
<210> 13
   <211> 18
   <212> DNA
   <213> Homo sapiens
<400> 13
   gtcgtcgaca acggctcc 18
<210> 14
   <211> 19
   <212> DNA
   <213> Homo sapiens
<400> 14
   tgcaaggccg gcttcgcgg 19
<210> 15
   <211> 47
   <212> DNA
   <213> Homo sapiens
<400> 15
   ccagagtacc tgggtctgga cgtgccagtg tgaaccagaa ggccaag 47
<210> 16
   <211> 1191
   <212> DNA
   <213> Artificial Sequence
<220>
   <223> SYNTHESIZED
<400> 16
   ggacgcctcc gaccagtgtt tgccttttat ggtaataacg cggccggccc ggcttccttt      60
   gtccccaatc tgggcgcgcg ccggcgcccc ctggcggcct aaggactcgg cgcgccggaa     120
   gtggccaggg cgggggcgac ctcggctcac agcgcgcccg gctattctcg cagctcacca     180
   tgggatccgg gggcgaggag ctgttcgccg gcatcgtgcc cgtgctgatc gagctggacg     240
   gcgacgtgca cggccacaag ttcagcgtgc gcggcgaggg cgagggcgac gccgactacg     300
   gcaagctgga gatcaagttc atctgcacca ccggcaagct gcccgtgccc tggcccaccc     360
   tggtgaccac cctctgctac ggcatccagt gcttcgcccg ctaccccgag cacatgaaga     420
   tgaacgactt cttcaagagc gccatgcccg agggctacat ccaggagcgc accatccagt     480
   tccaggacga cggcaagtac aagacccgcg gcgaggtgaa gttcgagggc gacaccctgg     540
   tgaaccgcat cgagctgaag ggcaaggact tcaaggagga cggcaacatc ctgggccaca     600
   agctggagta cagcttcaac agccacaacg tgtacatccg ccccgacaag gccaacaacg     660
   gcctggaggc taacttcaag acccgccaca acatcgaggg cggcggcgtg cagctggccg     720
   accactacca gaccaacgtg cccctgggcg acggccccgt gctgatcccc atcaaccact     780
   acctgagcac tcagaccaag atcagcaagg accgcaacga ggcccgcgac cacatggtgc     840
   tcctggagtc cttcagcgcc tgctgccaca cccacggcat ggacgagctg tacagggccg     900
   gctccggtac cgatgatgat atcgccgcgc tcgtcgtcga caacggcagc ggcatgtgca     960
   aggccggctt cgcgggcgac gatgcccccc gggccgtctt cccctccatc gtggggcgcc    1020
   ccaggcacca ggtaggggag ctggctgggt ggggcagccc cgggagcggg cgggaggcaa    1080
   gggcgctttc tctgcacagg agcctcccgg tttccggggt gggggctgcg cccgtgctca    1140
   gggcttcttg tcctttcctt cccagggcgt gatggtgggc atgggtcaga a             1191
<210> 17
   <211> 1192
   <212> DNA
   <213> Artificial Sequence
<220>
   <223> SYNTHESIZED
<400> 17
   ggacgcctcc gaccagtgtt tgccttttat ggtaataacg cggccggccc ggcttccttt 60
   gtccccaatc tgggcgcgcg ccggcgcccc ctggcggcct aaggactcgg cgcgccggaa 120
   gtggccaggg cgggggcgac ctcggctcac agcgcgcccg gctattctcg cagctcacca 180
   tgggatccgt gtctaagggc gaagagctga ttaaggagaa catgcacatg aagctgtaca 240
   tggagggcac cgtgaacaac caccacttca agtgcacatc cgagggcgaa ggcaagccct 300
   acgagggcac ccagaccatg agaatcaagg tggtcgaggg cggccctctc cccttcgcct 360
   tcgacatcct ggctaccagc ttcatgtacg gcagcagaac cttcatcaac cacacccagg 420
   gcatccccga cttctttaag cagtccttcc ctgagggctt cacatgggag agagtcacca 480
   catacgaaga cgggggcgtg ctgaccgcta cccaggacac cagcctccag gacggctgcc 540
   tcatctacaa cgtcaagatc agaggggtga acttcccatc caacggccct gtgatgcaga 600
   agaaaacact cggctgggag gccaacaccg agatgctgta ccccgctgac ggcggcctgg 660
   aaggcagaag cgacatggcc ctgaagctcg tgggcggggg ccacctgatc tgcaacttca 720
   agaccacata cagatccaag aaacccgcta agaacctcaa gatgcccggc gtctactatg 780
   tggaccacag actggaaaga atcaaggagg ccgacaaaga gacctacgtc gagcagcacg 840
   aggtggctgt ggccagatac tgcgacctcc ctagcaaact ggggcacaaa cttaatgccg 900
   gctccggtac cgatgatgat atcgccgcgc tcgtcgtcga caacggcagc ggcatgtgca 960
   aggccggctt cgcgggcgac gatgcccccc gggccgtctt cccctccatc gtggggcgcc 1020
   ccaggcacca ggtaggggag ctggctgggt ggggcagccc cgggagcggg cgggaggcaa 1080
   gggcgctttc tctgcacagg agcctcccgg tttccggggt gggggctgcg cccgtgctca 1140
   gggcttcttg tcctttcctt cccagggcgt gatggtgggc atgggtcaga ag 1192
<210> 18
   <211> 15
   <212> DNA
   <213> Homo sapiens
<400> 18
   cctcgccgcc ccgct 15
<210> 19
   <211> 16
   <212> DNA
   <213> Homo sapiens
<400> 19
   gccgcccgcc atggcg 16
<210> 20
   <211> 37
   <212> DNA
   <213> Homo sapiens
<400> 20
   cctcgccgcc ccgctgtctc cgccgcccgc catggcg 37
<210> 21
   <211> 1996
   <212> DNA
   <213> Artificial Sequence
<220>
   <223> SYNTHESIZED
<400> 21
   ccctgagcct ggtccgggaa ccgcccagcc gggagggccg agctgacggt tgcccaaggg 60
   ccagatttta aatttacagg cccggccccc gaaccgccga agcgcgctgc ctgctcccca 120
   ttggcccatg gtagtcacgt ggaggcgccg gggcgtgccg gccatgttgg ggagtgcggc 180
   gccgcggccc gcgccacctc cgccccccgc ggcttgcctc cagcccgccc ctcccggccc 240
   tcctcccccc gcccgccgct ccgtgcagcc tgagaggaaa caaagtgctg cgagcaggag 300
   acggcggcgg cgcgaaccct gctgggcctc cagtcaccct cgtcttgcat tttcccgcgt 360
   gcgtgtgtga gtgggtgtgt gtgttttctt acaaagggta tttcgcgatc gatcgattga 420
   ttcgtagttc ccccccgcgc gcctttgccc tttgtgctgt aatcgagctc ccgccatccc 480
   aggtgcttct ccgttcctct aaacgccagc gtctggacgt gagcgcaggt cgccggtttg 540
   tgccttcggt ccccgcttcg ccccctgccg tcccctcctt atcacggtcc cgctcgcggc 600
   ctcgccgccc cgctgtctcc gccgcccgcc atgggatccg ggggcgagga gctgttcgcc 660
   ggcatcgtgc ccgtgctgat cgagctggac ggcgacgtgc acggccacaa gttcagcgtg 720
   cgcggcgagg gcgagggcga cgccgactac ggcaagctgg agatcaagtt catctgcacc 780
   accggcaagc tgcccgtgcc ctggcccacc ctggtgacca ccctctgcta cggcatccag 840
   tgcttcgccc gctaccccga gcacatgaag atgaacgact tcttcaagag cgccatgccc 900
   gagggctaca tccaggagcg caccatccag ttccaggacg acggcaagta caagacccgc 960
   ggcgaggtga agttcgaggg cgacaccctg gtgaaccgca tcgagctgaa gggcaaggac 1020
   ttcaaggagg acggcaacat cctgggccac aagctggagt acagcttcaa cagccacaac 1080
   gtgtacatcc gccccgacaa ggccaacaac ggcctggagg ctaacttcaa gacccgccac 1140
   aacatcgagg gcggcggcgt gcagctggcc gaccactacc agaccaacgt gcccctgggc 1200
   gacggccccg tgctgatccc catcaaccac tacctgagca ctcagaccaa gatcagcaag 1260
   gaccgcaacg aggcccgcga ccacatggtg ctcctggagt ccttcagcgc ctgctgccac 1320
   acccacggca tggacgagct gtacagggga tctggatcag gtaccgcgac tgcgaccccc 1380
   gtgccgccgc ggatgggcag ccgcgctggc ggccccacca cgccgctgag ccccacgcgc 1440
   ctgtcgcggc tccaggagaa ggaggagctg cgcgagctca atgaccggct ggcggtgtac 1500
   atcgacaagg tgcgcagcct ggagacggag aacagcgcgc tgcagctgca ggtgacggag 1560
   cgcgaggagg tgcgcggccg tgagctcacc ggcctcaagg cgctctacga gaccgagctg 1620
   gccgacgcgc gacgcgcgct cgacgacacg gcccgcgagc gcgccaagct gcagatcgag 1680
   ctgggcaagt gcaaggcgga acacgaccag ctgctcctca agtgagtgct agctggcggc 1740
   cgcgttagcg ccaaggaggg gcgggggcgc aaccgcggcg accagctcac cgggttctgc 1800
   cgtggggagg gagcagaggc caggatgcac gcgtccttct gaaggaacag ggtctcggtc 1860
   tccggaaagg agaaagaatc tagagttcat agcggagcag gggtcgcgga gggggctcga 1920
   gctgtagcgc tggggggccg tgatgcccat ttctagattt tggatacccg ctgggacgtg 1980
   gtaagtgcgc gcctgg 1996
<210> 22
   <211> 15
   <212> DNA
   <213> Homo sapiens
<400> 22
   tacctgggtc tggac 15
<210> 23
   <211> 18
   <212> DNA
   <213> Homo sapiens
<400> 23
   agtgtgaacc agaaggcc 18
<210> 24
   <211> 69
   <212> DNA
   <213> Homo sapiens
<400> 24
   atggatgatg atatcgccgc gctcgtcgtc gacaacggct ccggcatgtg caaggccggc 60
ttcgcgggc 69
<210> 25
   <211> 18
   <212> DNA
   <213> Homo sapiens
<400> 25
   cacaccaaca actgccca 18
<210> 26
   <211> 15
   <212> DNA
   <213> Homo sapiens
<400> 26
   ggagaaggag gaaga 15
<210> 27
   <211> 51
   <212> DNA
   <213> Homo sapiens
<400> 27
   atggcccaat ggaatcagct acagcagctt gacacacggt acctggagca g 51
<210> 28
   <211> 3343
   <212> DNA
   <213> Artificial Sequence
<220>
   <223> SYNTHESIZED
<400> 28
<210> 29
   <211> 42
   <212> DNA
   <213> Homo sapiens
<400> 29
   tgtcgccttc gcctcctaat ccctagccac tatggtgagt aa 42

## Description

### CROSS-REFERENCE TO RELATED APPLICATIONS

This application claims the priority of US provisional application number 61/323,702, filed April 13, 2010, US provisional application number 61/323,719, filed April 13, 2010, US provisional application number 61/323,698, filed April 13, 2010, US provisional application number 61/367,017, filed July 23, 2010, US provisional application number 61/390,668, filed October 7, 2010, US provisional application number 61/408,856, filed November 1, 2010, and US provisional application number 61/431,957, filed January 12, 2011, each of which is hereby incorporated by reference in its entirety.

### FIELD OF THE INVENTION

The present disclosure relates to methods for tagging endogenous proteins.

### BACKGROUND OF THE INVENTION

Protein tagging is extensively used to provide a visual readout on the protein of interest in the cell. Among other uses, tagged proteins are used to study protein abundance and localization, transcriptional and translational regulation, post-translational modifications, protein-protein interactions, alternative splicing, knockdown of RNA and protein by RNAi and transcription factor binding sites. However, current methods of expressing tagged proteins in the cell result in distorted expression that does not reflect the expression pattern of the endogenous protein. This is because expression of tagged proteins often relies on heterologous promoters for expression. In addition, some tagged proteins are expressed ectopically from epigenetic vectors or vectors randomly integrated into the cell genome and are therefore not controlled by the endogenous regulatory pathways. Thus, there exists a strong need for a method that can direct specific integration into the chromosome of a cell to produce a tagged protein controlled by endogenous regulatory pathways.

### SUMMARY OF THE INVENTION

In one aspect, the present disclosure provides a method for tagging at least one endogenous protein. The method comprises a) introducing into a cell (i) at least one targeting endonuclease or nucleic acid encoding a targeting endonuclease, the targeting endonuclease binding a target site and able to cleave a cleavage site in a chromosomal sequence encoding the endogenous protein, and (ii) at least one donor polynucleotide comprising a tag sequence, the tag sequence being flanked by an upstream sequence and a downstream sequence, the upstream sequence and the downstream sequence sharing substantial sequence identity with either side of the cleavage site in the chromosomal sequence; and (b) maintaining the cell under conditions such that a double-stranded break introduced at the cleavage site by the targeting endonuclease is repaired by a homology-directed process such that the tag sequence in the donor polynucleotide is integrated in-frame into the chromosomal sequence encoding the endogenous protein chosen from actin, rubulin, lamin, human epidermal growth factor receptor (HER2) and high mobility group A protein (HMGA), wherein a tagged endogenous protein is produced.

In another aspect, the present disclosure provides a cell comprising at least one tag sequence integrated in-frame into a chromosomal sequence encoding an endogenous protein, such that the cell expresses at least one tagged endogenous protein.

In yet another aspect, the present disclosure provides a kit for monitoring the localization of an endogenous protein. The kit comprises a cell having at least one tag sequence integrated in-frame into a chromosomal sequence encoding an endogenous protein, such that the cell expresses at least one tagged endogenous protein.

Other aspects and iterations of the disclosure are described in more detail below.

### REFERENCE TO COLOR FIGURES

The application file contains at least one photograph executed in color. Copies of this patent application publication with color photographs will be provided by the Office upon request and payment of the necessary fee.

### BRIEF DESCRIPTION OF THE FIGURES

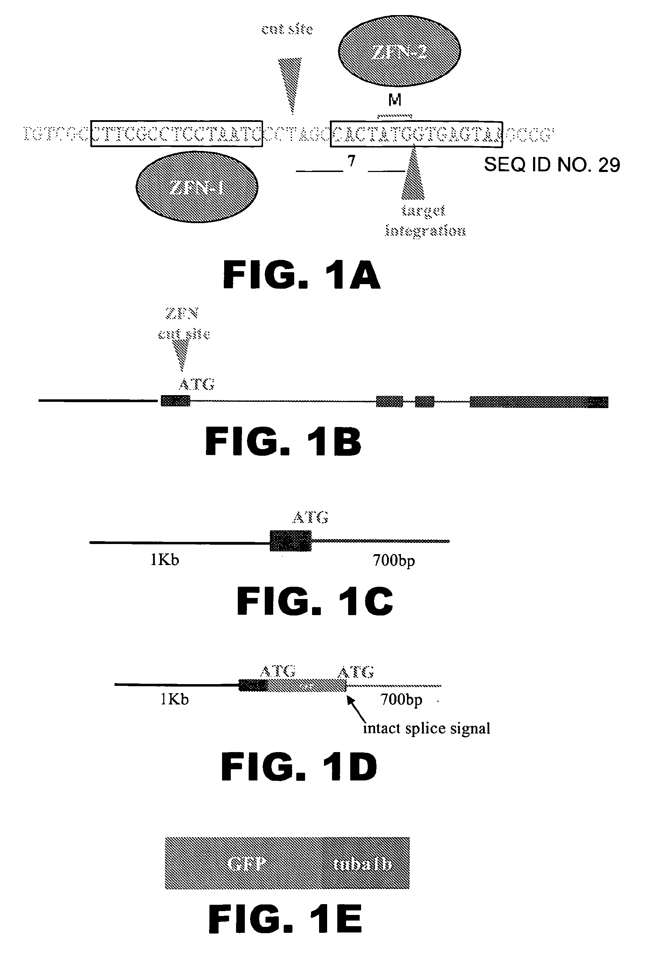

**Fig. 1** depicts the design of tag sequence integration at the TUBA1 B locus. (A) is a schematic showing the chromosome sequence (SEQ ID NO:29) at the target region for integration of the tag sequence, ZFN binding sites (boxed nucleotides) on the chromosome target region, the ZFN cut site (yellow arrow), and the tag sequence integration site (green arrow). (B) is a schematic depicting the TUBA1 B genomic target region showing the coding region (red), untranslated region (blue) and the ZFN cut site (yellow arrow). (C) is a schematic of the DNA fragment of the TUBA1B genomic region before integration. (D) is a schematic of the DNA fragment of the TUBA1 B genomic region with the GFP sequence integrated in-frame with the TUBA1 B coding sequence. (E) is a schematic of the endogenous α-tubulin protein fused to the GFP tag at the N-terminus created after successful integration of the tag sequence.
**Fig. 2** depicts the map of a donor plasmid comprising the GFP tag flanked by the genomic tubulin sequences.
**Fig. 3** depicts the DNA sequence (SEQ ID NO:4) of the TUBA1 B genomic region in U2OS cells demonstrating that GFP2 coding sequence was integrated into the tubulin coding region. The underlined text denotes the region sequenced, bold text indicates coding sequence of GFP2, italicized text indicates restriction site or linker, and bold and upper case text indicates the Met codon for splice junction.
**Fig. 4** depicts the DNA sequence (SEQ ID NO:5) of the TUBA1 B genomic region in U2OS cells demonstrating that RFP coding sequence was integrated into the tubulin coding region. The underlined text denotes the region sequenced, bold text indicates coding sequence of RFP, italicized text indicates restriction site or linker, and bold and upper case text indicates the Met codon for splice junction.
**Fig. 5** presents agarose gel electrophoresis analysis of junction PCR of 14 cell clones using primers specific for the targeted integration of GFP into the TUBA1 B locus. Molecular size markers and a GFP control are also shown.

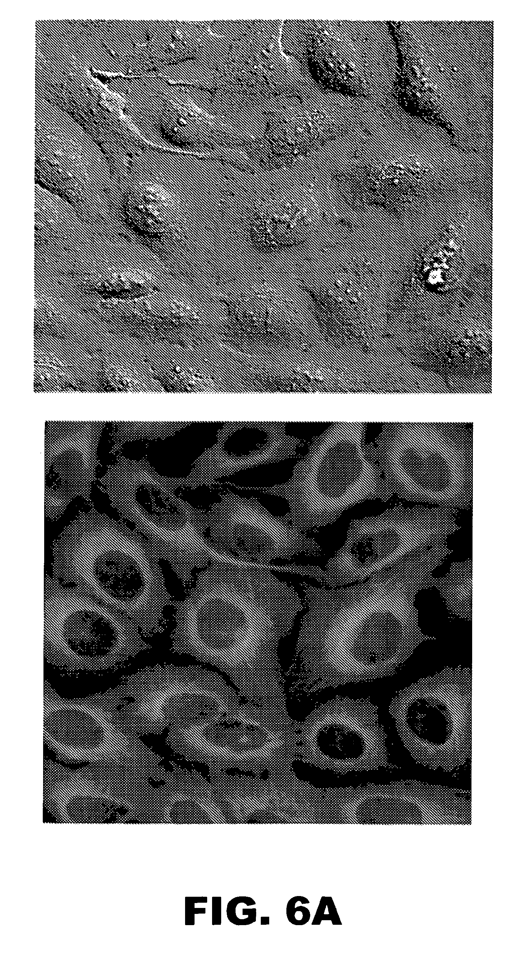

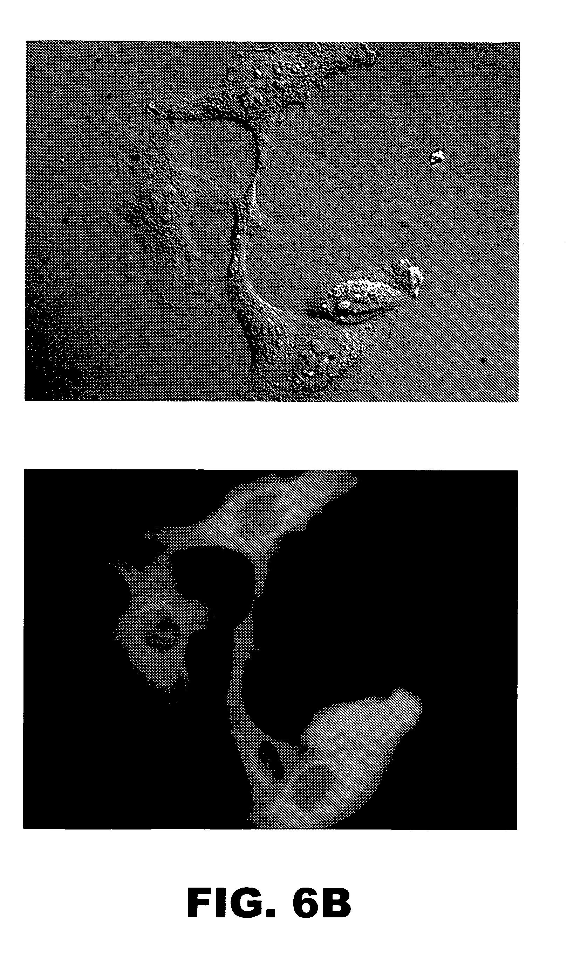

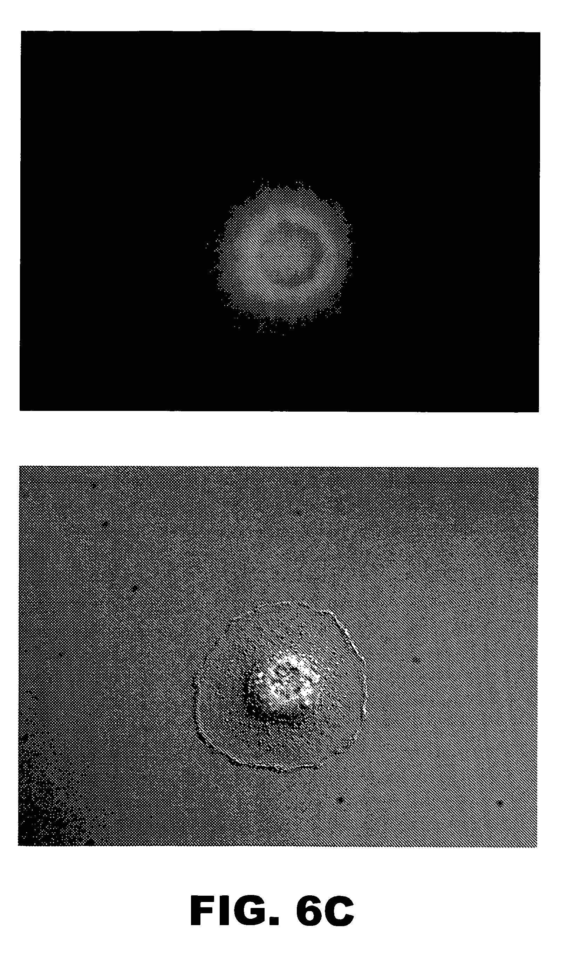

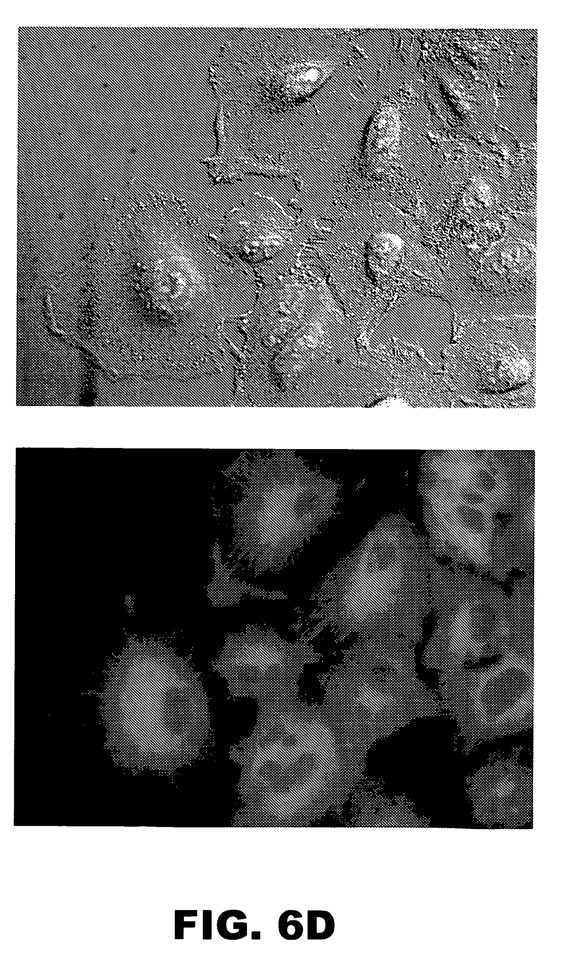

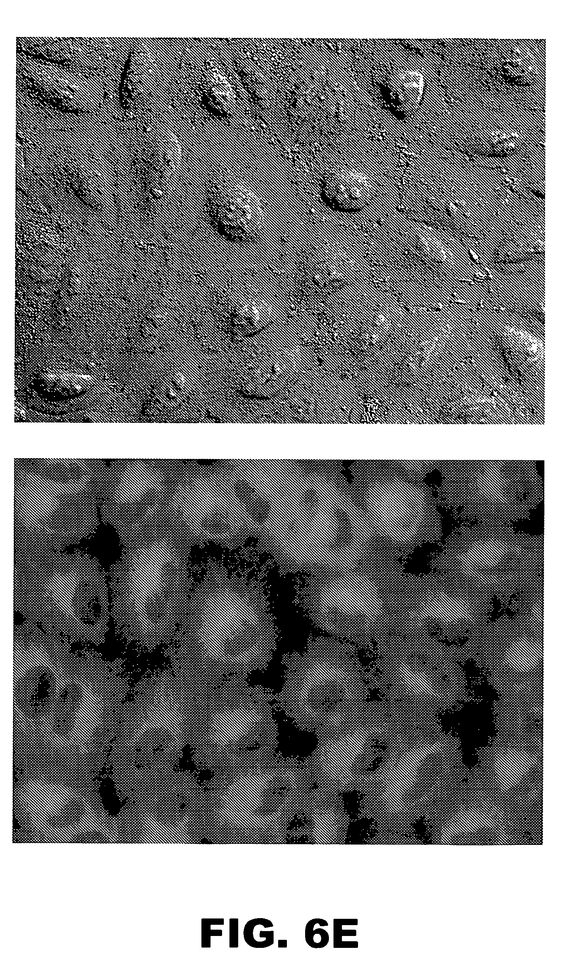

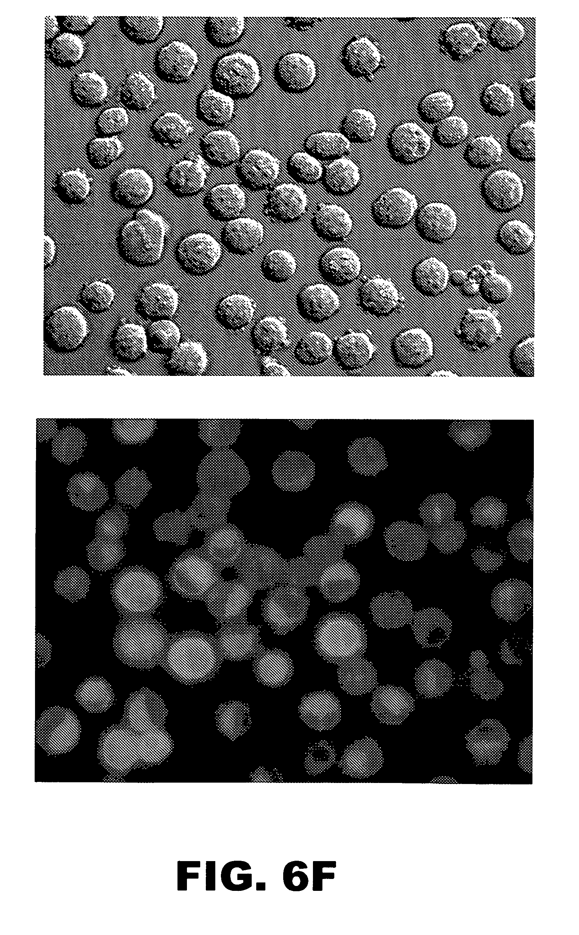

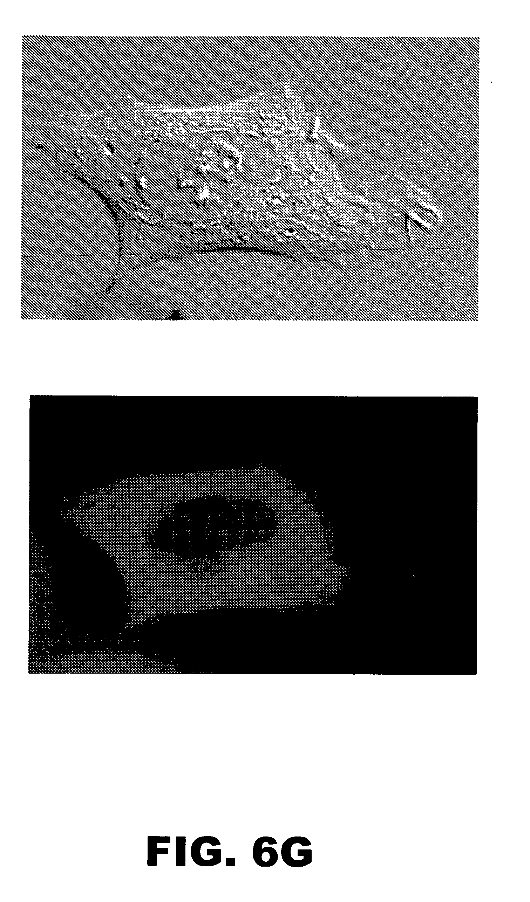

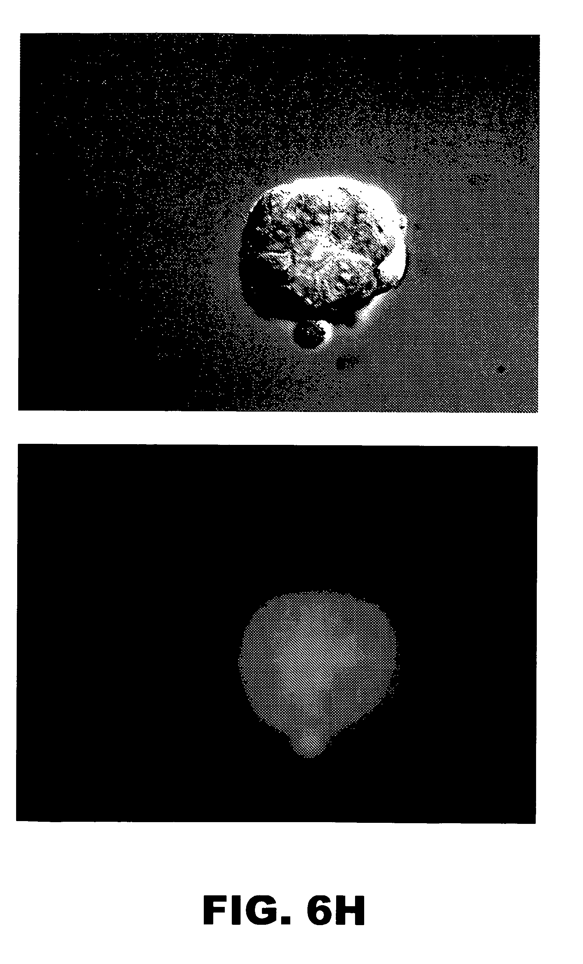

**Fig. 6** shows multiple examples of differential interference contrast (DIC) and fluorescence microscopy images of individual isolated cell clones expressing endogenous α-tubulin isoform 1 B protein tagged with GFP. (A) GFP-tagged α-tubulin isoform 1 B protein in U2OS cells, (B) GFP-tagged α-tubulin isoform 1 B protein in U2OS cells, (C) GFP-tagged α-tubulin isoform 1 B protein in U2OS cells, (D) GFP-tagged α-tubulin isoform 1 B protein in A549 cells, (E) GFP-tagged α-tubulin isoform 1 B protein in A549 cells, (F) GFP-tagged α-tubulin isoform 1 B protein in K562 cells, (G) GFP-tagged α-tubulin isoform 1 B protein in HEK293 cells, and (H) GFP-tagged α-tubulin isoform 1 B protein in HEK293T cells.
**Fig. 7** depicts the map of a donor plasmid comprising the RFP tag flanked by the genomic tubulin sequences.
**Fig. 8** shows the verification of the RFP integration into the TUBA1B region in MCF10a cell line. The integration was verified by genomic PCR and junction PCR using Tubulin primers. (A) Southern blotting showing presence of 1945 bp RFP/tubulin fusion band and (B) Genomic PCR showing the positive integration of RFP tag sequence into TUBA1 B in several clones (T.I. = targeted integration). The Wt MCF10a cell and U2SO cell line with RFP integration were used as controls.
**Fig. 9** depicts the confirmed sequence of TUBA1 B region in MCF10a cells demonstrating integration of RFP sequence (SEQ ID NO:8). The underlined text denotes the region sequenced, bold text indicates coding sequence of GFP2, italicized text indicates restriction site or linker, and bold and upper case text indicates the Met codon for splice junction.
**Fig. 10** depicts PCR verification of RFP integration into TUBA1 B locus of MCF10a cells, as well as RFP and GFP integration into same locus of U2OS cells. Wild-type band was 452 bp and targeted integrated (T.I.) band was 1190 bp
**Fig. 11** shows that the junctions at the site of insertion of RFP in MCF10a clone 5 were of the expected sizes. The expected size of the left junction is 453 bp and the expected size of the right junction is 4089 bp.
**Fig. 12** depicts the Western blotting detecting RFP and tubulin expression in the MCF10a clone 5 with the RFP tagged tubulin.
**Fig. 13** demonstrates that >99% of wild-type MCF10a cells lack red fluorescence, whereas >99% of MCF10a clone 5 cells comprising RFP tagged tubulin had red fluorescence.
**Fig. 14** depicts the phenotype stability of the transfected MCF10a cells comprising RFP-tagged tubulin. (A) Expression at P2 and (B) P18. DIC images on the left and fluorescent images on the right..
**Fig. 15** depicts the map of a donor plasmid comprising the GFP tag flanked by the genomic STAT3 sequences.
**Fig. 16** depicts a schematic showing the chromosome sequence (SEQ ID NO:27) at the STAT3 region for integration of the tag sequence, ZFN binding sites (yellow sequence) on the chromosome target region, the ZFN cut site (yellow arrow), and tag sequence integration site (green arrow). "M" symbolizes the amino acid start codon methionine.
**Fig. 17** depicts a Cel-1 assay confirming the efficacy of ZFNs in cutting the STAT3 chromosomal sequence at the intended site (third lane). Cel-1 results for donor polynucleotide control alone and ZFN with donor polynucleotide control are also shown.
**Fig. 18** presents agarose gel electrophoresis analysis of synthesized RNA encoding ZFNs specific for the STAT3 locus.
**Fig. 19** depicts cell sorter data for cells transfected with ZFNs and donor polynucleotide for integration of GFP into STAT3 locus (A). Also shown is cell sorter data for negative control cells (B).
**Fig. 20** depicts an agarose gel electrophoresis analysis of junction PCR of 2 different targeted regions in the genome: the ACTB region encoding β-actin was targeted with a tag sequence encoding either GFP or RFP, while STAT3 was targeted with a tag sequence encoding GFP. STAT3 was analyzed using 2 different junction primer sets ("primer 1" and "primer 2"). PCR confirmed integration within the actin locus, but not within the STAT3 locus. Molecular size markers and a GFP control are also shown.
**Fig. 21** depicts the map of a donor plasmid comprising the genomic MAPRE3 sequences flanking the GFP tag sequence.
**Fig. 22** depicts a Cel-1 assay showing the efficacy of a number of ZFN pairs in cutting the MAPRE3 chromosomal sequence at the N-terminus integration site. Lane 1 is a DNA size marker, lanes 2 and 11 are GFP control, and lanes 3 to 10 depict Cel-1 assay using various ZFN pairs shown above each lane.
**Fig. 23** depicts a Cel-1 assay showing the efficiency of a number of ZFN pairs in cutting the MAPRE3 targeted chromosomal sequence at the C-terminus integration site (lanes 4-7) and Cel-1 assay results of ZFN pairs in cutting the LMNB1 targeted chromosomal sequence (lanes 10-13). Lanes 1 and 2 are a DNA size marker, lanes 3 and 8 are GFP-MAPRE3 controls, and lanes 9 and 14 are GFP-Lamin controls.
**Fig. 24** presents agarose gel electrophoresis analysis of junction PCR at the MAPRE3 target site. Circles highlight possible integration of tag sequences.
**Fig. 25** depicts cell sorter analysis of cells transfected with ZFNs and donor polynucleotide for integrating GFP tag sequences into the MAPRE3 locus. (A) control cells transfected with donor polynucleotide alone, and (B) cells transfected with ZFN + donor polynucleotide.

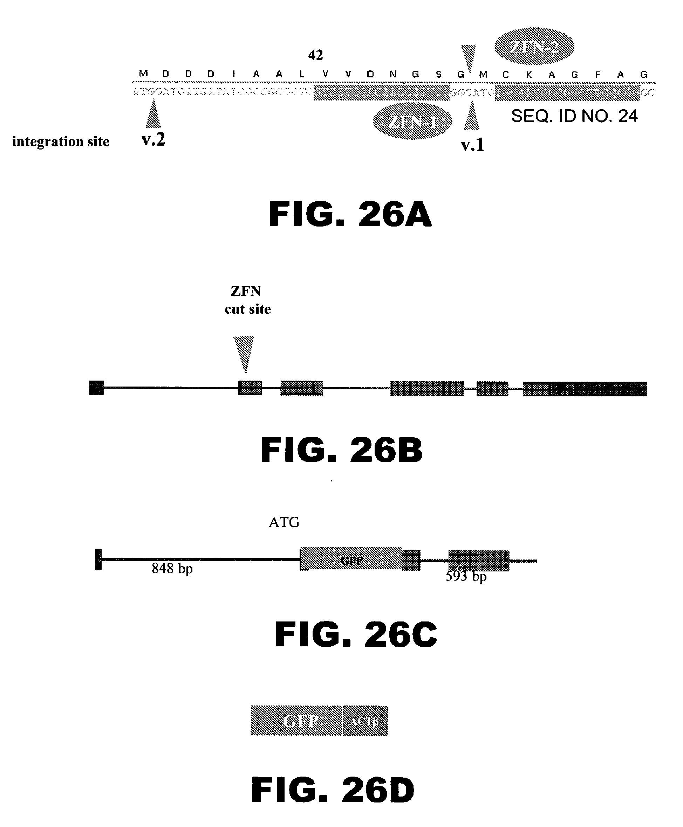

**Fig. 26** depicts the design of tag sequence integration at the ACTB locus. (A) is a schematic showing the chromosome sequence (SEQ ID NO:24) at the target region for integration of the tag sequence, ZFN binding sites (yellow sequence) on the chromosome target region, the ZFN cut site (yellow arrow), and the tag sequence integration sites (green, and green and yellow arrows). (B) is a schematic depicting the ACTB genomic target region showing the coding region (red), untranslated region (blue) and the ZFN cut site (yellow arrow). (C) is a schematic of the ACTB genomic region with the GFP sequence integrated in-frame with the ACTB coding sequence. (D) is a schematic of the endogenous β-actin protein fused to the GFP tag at the N-terminus created after successful integration of the tag sequence.
**Fig. 27** shows a Cel-1 assay screen for ZFNs targeting ACTB locus in K562 cells. Lane 1 is a marker, and numbers above the lanes refer to ZFN pairs.
**Fig. 28** depicts the map of a donor plasmid comprising the GFP tag flanked by the genomic ACTB sequences whose integration site is represented as "v.2" in **Fig. 26A****.**
**Fig. 29** shows fluorescence microscopy images of individual isolated cell clones expressing endogenous β-actin protein tagged with GFP. Well position is labeled above each image.
**Fig. 30** depicts the DNA sequence (SEQ ID NO:16) of the ACTB1 genomic region in U2OS cells demonstrating that GFP2 coding sequence was integrated into the Actin coding region The underlined text denotes the region sequenced, bold text indicates coding sequence of GFP2, italicized text indicates restriction site or linker, and bold and upper case text indicates the Met codon for splice junction.
**Fig. 31** depicts the DNA sequence (SEQ ID NO:17) of the ACTB1 genomic region in U2OS cells demonstrating that RFP coding sequence was integrated into the Actin coding region. The underlined text denotes the region sequenced, bold text indicates coding sequence of RFP, italicized text indicates restriction site or linker, and bold and upper case text indicates the Met codon for splice junction.
**Fig. 32** depicts the map of a donor plasmid for integrating the GFP tag sequences and exchanging the genomic sequence encoding the first 15 amino acids of the β-actin protein with a nucleic acid sequence encoding an alternate codon usage whose integration site is represented as "v.1" in **Fig. 26A****.**
**Fig. 33** is a schematic of the DNA fragment shown in **Fig. 32** of the ACTB genomic region in the donor polynucleotide used to replace the genomic sequence encoding the first 15 amino acids of the β-actin protein with a nucleic acid sequence encoding an alternate codon usage.

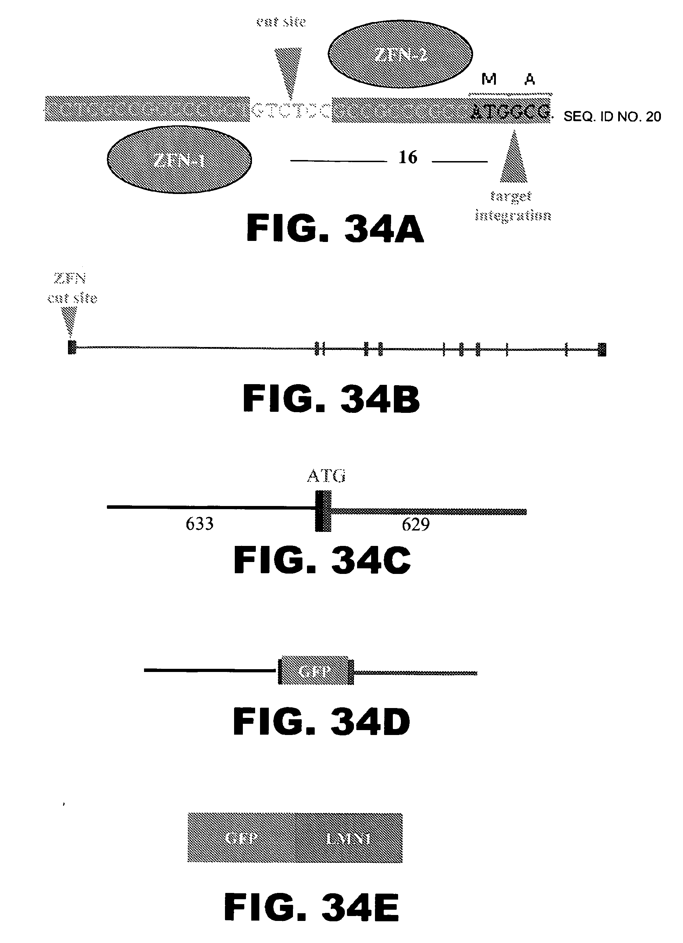

**Fig. 34** depicts the design of tag sequence integration at the LMNB1 locus. (A) is a schematic showing the chromosome sequence (SEQ ID NO:20) at the target region for integration of the tag sequence, ZFN binding sites (yellow sequence) on the chromosome target region, the ZFN cut site (yellow arrow), and the tag sequence integration site (green arrow). (B) is a schematic depicting the LMNB1 genomic target region showing the coding region (red), untranslated region (blue) and the ZFN cut site (yellow arrow). (C) is a schematic of the targeted site of integration in LMNB1 genomic region. (D) is a schematic of the LMNB1 genomic region in with the GFP sequence integrated into LMNB1 coding sequence. (E) is a schematic of the endogenous Lamin B1 protein fused to the GFP tag at the N-terminus created after successful integration of the tag sequence.
**Fig. 35** shows differential interference contrast (DIC) and fluorescence microscopy images of cells expressing endogenous Lamin B1 protein tagged with GFP.
**Fig. 36** depicts the DNA sequence of the LAMNB1 genomic region in U2OS cells demonstrating that RFP coding sequence was integrated into the Lamin coding region (SEQ ID NO:21). The underlined text denotes the region sequenced, bold text indicates coding sequence of GFP2, italicized text indicates restriction site or linker, and bold and upper case text indicates the start codon for splice junction.
**Fig. 37** shows images of iPS cells comprising RFP-tagged Lamin. (A) DIC image of the field of cells. (B) Red fluorescent image showing expression of lamin tagged with RFP. (C) Nuclei of cells stained with DAPI.
**Fig. 38** depicts the design of tag sequence (SEQ ID:15) integration at the ERBB2 locus. The schematic figure shows the chromosome sequence at the target region for integration of the tag sequence, ZFN binding sites on the chromosome target region, the ZFN cut site, and the tag sequence integration site.
**Fig. 39** depicts the map of a donor plasmid for integrating the GFP tag sequences. The GFP coding sequence is flanked by ERBB2 genomic sequences
**Fig. 40** depicts the Junction PCR of the left junction to confirm integration of GFP2 into the ERBB2 locus in SKOV3 cells.
**Fig. 41** shows expression of GFP-tagged HER2 in SKOV3 cells. Upper Images: DIC; lower Images fluorescent microscopy.
**Fig. 42** depicts the design of tag sequence integration at the HMGA locus. The schematic figure shows the chromosome sequence (SEQ ID NO:3) at the target region for integration of the tag sequence, ZFN binding sites on the chromosome target region, the ZFN cut site, and the tag sequence integration site, and the relevant location of coding region, untranslated region, and the insertion site of GFP in the HMGA locus.
**Fig. 43** depicts the map of a donor plasmid for integrating the GFP tag sequences. The GFP coding sequence is flanked by HMG1 chromosomal sequences.
**Fig. 44** depict (A) Genomic PCR and (B) Southern blotting (with a GFP probe) verification of the integration of GFP tag into HMGA1 locus in selected clones.
**Fig. 45** depicts the DNA sequence of the HMGA1 genomic region in U2OS cells demonstrating that GFP2 coding sequence was integrated into the HMGA coding region (SEQ ID NO:17). The underlined text denotes the region sequenced, bold text indicates coding sequence of GFP2, italicized text indicates restriction site or linker, and bold and upper case text indicates the start codon for splice junction.
**Fig. 46** shows images of U2OS cells expressing GFP-tagged HMGA1 protein. Left; DIC image; right: fluorescent image.

### DETAILED DESCRIPTION OF THE INVENTION

The present disclosure encompasses a method for tagging an endogenous protein in a cell. The method comprises contacting a cell with a targeting endonuclease and a donor polynucleotide comprising a tag sequence. The targeting endonuclease introduces a double stranded break at a specific site in the chromosomal sequence encoding the endogenous protein. The double stranded break induces cell DNA repair process that results in homologous recombination and repair of the double stranded break using a donor polynucleotide as a template. As a consequence, the tag sequence in the donor polynucleotide is integrated in-frame into the chromosome sequence encoding the endogenous protein. Because the tag sequence is integrated in-frame with the endogenous coding sequence, the endogenous protein comprises a tag sequence when it is produced.

Advantageously, as illustrated in the examples, the method may be utilized to express tagged proteins under the control of endogenous regulatory pathways reflecting the expression pattern of the endogenous protein.

The present disclosure also provides cells comprising at least one tag sequence integrated in-frame into a chromosomal sequence encoding an endogenous protein, such that the cell expressed at least one tagged endogenous protein. Also provided herein is a kit for monitoring the localization of at least one endogenous protein, wherein the kit comprise a cell having at least one tag sequence integrated in-frame into a chromosomal sequence encoding an endogenous protein.

### I. Cell Comprising Tagged Endogenous Protein(s)

One aspect of the present disclosure encompasses a cell comprising at least one tag sequence integrated in-frame into a chromosomal sequence encoding an endogenous protein, such that the cell expressed at least one tagged endogenous protein. Examples of suitable endogenous proteins are detailed below, as are examples of suitable tags.

### (a) endogenous protein

The term "endogenous protein" herein refers to a protein encoded by the genetic material of the cell. In general, any endogenous protein of interest may be tagged with a variety of tag sequences.

In one embodiment, the endogenous protein may be a tubulin protein. In various embodiments, the tubulin protein may be a human tubulin protein such as an α-tubulin protein encoded by the TUBA1A, TUBA1B, TUBA1C, TUBA3C, TUBA3D, TUBA3E, TUBA4A and TUBA8 genes; a β-tubulin protein encoded by the TUBB, TUBB1, TUBB2A, TUBB2B, TUBB2C, TUBB3, TUBB4, TUBB4Q and TUBB6 genes; a γ-tubulin protein encoded by the, TUBG1, TUBG2, TUBGCP2, TUBGCP3, TUBGCP4, TUBGCP5 and TUBGCP6 genes; a δ-tubulin protein encoded by the TUBD1 gene, or a ε-tubulin protein encoded by the TUBE1 gene. In an exemplary embodiment, the endogenous tubulin may be the human α-tubulin isoform 1 B protein encoded by the TUBA1B gene on human chromosome number 12 (accession number NM_006082).

In another embodiment, endogenous protein may be an actin protein. In some embodiment, the actin protein may be a human actin protein such as α-actin encoded by the ACTA1 gene, the β-actin protein encoded by the ACTB gene, or the γ-actin protein encoded by the ACTG1 gene. In an exemplary embodiment, the endogenous protein may be the human β-actin protein encoded by the ACTB gene on human chromosome 7 (accession number N_001101).

In yet another embodiment, endogenous protein may be a lamin protein. In certain embodiments, the lamin protein may be a human lamin protein such as B1 and B2 Lamins, expressed by the LMNB1 and LMNB2 genes, or Lamin A and C proteins, the splice variants of the LMNA gene. In an exemplary embodiment, the endogenous protein may be the human Lamin B1 protein encoded by the LMNB1 gene on human chromosome 5 (accession number NM_005573).

In still another embodiment, the endogenous protein may be human epidermal growth factor receptor 2 (HER2 protein) that is encoded by the ERBB2 gene. HER2 is a cell membrane surface-bound receptor tyrosine kinase and is involved in the signal transduction pathways leading to cell growth and differentiation. Amplification of the ERBB2 gene or overexpression of its protein product is associated with breast cancer, ovarian cancer and stomach cancer. The endogenous HER2 protein may be the human HER2 protein (UniProtKB/Swiss-Prot accession number: P04626).

In an alternative embodiment, the endogenous protein may be HMGA. HMGA refers to high mobility group of chromosomal proteins that regulate gene expression by changing the DNA conformation by binding to AT-rich regions. They are among the largest and best characterized group of non-histone nuclear proteins. HMGA1 gene regulates a diverse array of normal biological processes including cell growth, proliferation, differentiation and death. At least seven transcript variants encoding two different isoforms have been found for this gene. In some embodiments, the endogenous protein may be a human HMGA protein. Non-limiting examples of human HMGA proteins that may be used in the invention include HMGA isoform a and isoform b, expressed by the HMGA1 gene (accession number NM_145899).

In further embodiments, the endogenous protein may be a protein listed in TABLE A.

| **Table A Other endogenously tagged proteins** | | | | |
|---|---|---|---|---|
| | **Gene Symbol** | **Protein Name** | **Protein Symbol** | **Protein Accession No.** |
| 1 | HiF1a | Hypoxia-inducible factor-1 | HIF1 | Q16665 |
| 2 | VEGF(A, B, C) | vascular endothelial growth factor (A, B, C) | VEGFA, VEFGB, VEGFC | P15692, P49765, P49767 |
| 3 | GLUT1 (SLC2A1) | solute carrier family 2 (facilitated glucose transporter) | GTR1 | P11166 |
| 4 | LDHA | lactate dehydrogenase A | LDHA | P00338 |
| 5 | IL-1 (A, B) | Interleukin 1 (alpha, beta) | IL1A, IL1B | P01538, P01584 |
| 6 | IL-8 | Interleukin 8 | IL8 | P10145 |
| 7 | Cox-2 (PTGS2) | prostaglandin-endoperoxide synthase 2 | PTGS2 | P35354 |
| 8 | CCND1 | cyclinD1 | CCND1 | P24385 |
| 9 | CDKN1B (p27) | cyclin-dependent kinase inhibitor 1B | CDKN1B | P46527 |
| 10 | CREB1 | cAMP responsive element binding protein 1 | CREB1 | P16220 |
| 11 | Bcl2 | B-cell CLL/lymphoma 2 | BCL2 | P10415 |
| 12 | MDM2 | p53 binding protein | MDM2 | Q00987 |
| 13 | p70S6K (RPS6KB1) | ribosomal protein S6 kinase, 70kDa, polypeptide 1 | RPS6KB1 | P23443 |
| 14 | FKHR (FOXO1) | forkhead box O1 | FOXO1 | Q12778 |
| 15 | β-catenin (Ctnnb1) | catenin (cadherin-associated protein), beta 1 | CTNNB1 | P35222 |
| 16 | MMP7 | matrix metallopeptidase 7 (matrilysin, uterine) | MMP7 | P09237 |
| 17 | Vim | Vimentin | VIM | P08670 |
| 18 | BIRC5 | baculoviral IAP repeat-containing 5 (survivin variant 3 alpha) | BIRC5 | 015392 |
| 19 | CCND2 | Cyclin D2 | CCND2 | P30279 |
| 20 | BCLXL (BCL2L1) | BCL2-like 1 | BCL2L1 | Q07817 |
| 21 | p21 (CIP1,CDKN1A) | cyclin-dependent kinase inhibitor 1A (p21, Cip1) | CDKN1A | P38936 |
| 22 | STAT1 | signal transducer and activator of transcription 1 | STAT1 | P42224 |
| 23 | STAT2 | signal transducer and activator of transcription 2 | STAT2 | P52630 |
| 24 | STAT3 | signal transducer and activator of transcription 3 | STAT3 | P40763 |
| 25 | STAT4 (SLEB11) | signal transducer and activator of transcription 4 | STAT4 | Q14765 |
| 26 | EGFR (ERBB1) | epidermal growth factor receptor | EGFR | P00533 |
| 27 | SOCS1 | suppressor of cytokine signaling 1 | SOCS1 | 015524 |
| 28 | SOCS2 | suppressor of cytokine signaling 2 | SOCS2 | 014508 |
| 29 | SOCS3 | suppressor of cytokine signaling 3 | SOCS3 | 014543 |
| 30 | Viperin (RSAD2, cig5) | radical S-adenosyl methionine domain containing 2 (Viperin) | RSAD2 | Q8WXG1 |
| 31 | GLUT4 (SLC2A4) | solute carrier family 2 (facilitated glucose transporter), member 4 | GTR4 | P14672 |
| 32 | COL1A1 | collagen, type I, alpha 1 | COL1A1 | P02452 |
| 33 | PPARG | peroxisome proliferator-activated receptor gamma | PPARG | P37231 |
| 34 | SMAD3 | SMAD family member 3 | SMAD3 | P84022 |
| 35 | SMAD4 | SMAD family member 4 | SMAD4 | Q13485 |
| 36 | JNK (MAPK8) | mitogen-activated protein kinase 8 | MAPK8 | P45983 |
| 37 | TP53 | tumor protein p53 | TP53 | P04637 |
| 38 | NF-kB (NFKB1, p50) | nuclear factor of kappa light polypeptide gene enhancer in B-cells 1 | NFKB1 | P19838 |
| 39 | Notch 1 | Notch 1 | NOTC1 | P46531 |
| 40 | ATF-2 | activating transcription factor 2 | ATF2 | P15336 |
| 41 | c-JUN (Jun) | jun proto-oncogene | JUN | P05412 |
| 42 | AKT1 | v-akt murine thymoma viral oncoqene homolog 1 | AKT1 | P31749 |
| 43 | p38α (MAPK14) | mitogen-activated protein kinase 14 | MK14 | Q16539 |
| 44 | p38β (MAPK11) | mitogen-activated protein kinase 11 | MK11 | Q15759 |
| 45 | p38γ (MAPK12) | mitogen-activated protein kinase 12 | MK12 | P53778 |
| 46 | ERK (MAPK1) | mitogen-activated protein kinase 1 | MK01 | P28482 |
| 47 | AhR | aryl hydrocarbon receptor | AHR | P35869 |
| 48 | PXR | nuclear receptor subfamily 1, group I, member 2 | NR1L2 | 075469 |
| 49 | CAR | Nuclear receptor subfamily 1 group I member 3 | NR1L3 | Q14994 |
| 50 | CYP1A2 | cytochrome P450, family 1, subfamily A, polypeptide 2 | CP1A2 | P05177 |
| 51 | CYP3A4 | cytochrome P450, family 3, subfamily A, polypeptide 4 | CP3A4 | P08684 |
| 52 | CYP2B6 | cytochrome P450, family 2, subfamily B, polypeptide 6 | CP2B6 | P20813 |
| 53 | Nrf2 | Nuclear factor erythroid 2-related factor 2 | NF2L2 | Q16236 |
| 54 | Hmox1 | heme oxygenase (decycling) 1 | HMOX1 | P09601 |
| 55 | GSTA2 | glutathione S-transferase alpha 2 | GSTA2 | P09210 |
| 56 | Prdx1 | peroxiredoxin 1 | PRDX1 | Q06830 |
| 57 | Keap1 | kelch-like ECH-associated protein 1 | KEAP1 | Q14145 |
| 58 | Grp78 | G protein-coupled receptor 78 | GPR78 | Q96P69 |
| 59 | ATF4 | activating transcription factor 4 (tax-responsive enhancer element B67) | ATF4 | P18848 |
| 60 | ATF6 | activating transcription factor 6 | ATF6 | P18850 |
| 61 | XBP1 | X-box binding protein 1 | XBP1 | P17861 |
| 62 | Gadd45a | growth arrest and DNA-damage-inducible, alpha | GADD45A | P24522 |
| 63 | p21 | ribonuclease P/MRP 21 kDa subunit | RPP21 | Q9H633 |
| 64 | Bax | BCL2-associated X protein | BAX | Q07812 |
| 65 | RAD51c | DNA repair protein RAD51 homolog 3 | RA51C | 043502 |
| 66 | BTG2 | BTG family member 2 | BTG2 | P78543 |
| 67 | OATP1B1 | solute carrier organic anion transporter family, member 1B1 | OATP2 | Q9Y6L6 |
| 68 | OATP1B3 | solute carrier organic anion transporter family, member 1 B3 | OATP8 | Q9NPD5 |
| 69 | OAT1 | solute carrier family 22 (organic anion transporter), member 6 | OAT1 | Q4U2R8 |
| 70 | OAT3 | solute carrier family 22 (organic anion transporter), member 8 | OAT3 | Q8TCC7 |
| 71 | OCT2 | solute carrier family 22 (organic cation transporter), member 2 | OCT2 | 015244 |
| 72 | BSEP | ATP-binding cassette, sub-family B (MDR/TAP), member 11 | BSEP | 095342 |
| 73 | MATE1 | solute carrier family 47, member 1 | MATE1 | Q96FL8 |
| 74 | BCRP | Breast cancer resistance protein | BCRP | Q9UNQ0 |
| 75 | ABCB1 | Multidrug resistance protein 1 | MDR1 | P08183 |
| 76 | ABCC2 | ATP-binding cassette sub-family C member 2 | MRP2 | Q92887 |
| 77 | Pdk1 | 3-phosphoinositide-dependent protein kinase 1 | PDK1 | 015530 |
| 78 | HSF-1 | Heat shock factor 1 | HSF1 | Q00613 |
| 79 | HSP90(AA1, AB1) | Heat shock protein HSP 90 (-alpha, -beta) | HSP90 | P07900 P08238 |
| 80 | HSPA1A/1B | Heat shock 70 kDa protein 1A/1B | HSP70 | P08107 |
| 81 | HSPB1 | Heat shock protein beta-1 | Hsp27 | P04792 |
| 82 | p65 | Transcription factor p65 | TP65 | Q04206 |
| 83 | IL2 | Interleukin-2 | IL-2 | P60568 |
| 84 | NOS2 | Nitric oxide synthase, inducible | iNOS | P35228 |
| 85 | iCAM (1,2,3,4,5) | Intercellular adhesion molecule (1,2,3,4,5) | iCAM (1,2,3,4,5) | P05362, P13598, P32942, Q14773, Q9UMF0 |
| 86 | JUN | Transcription factor AP-1 | AP1 | P05412 |
| 87 | Fbx15 | F-box only protein 15 | FBX15 | Q8NCQ5 |
| 88 | TUBB3 | Tubulin beta-3 chain | TUBB3 | Q13509 |
| 89 | UCHL1 | Ubiquitin carboxyl-terminal hydrolase isozyme L1 | UCHL1 | P09936 |
| 90 | SERPIN1 | | | |
| 91 | SV2A | synaptic vesicle glycoprotein 2A | SV2A | Q7L0J3 |
| 92 | GRIA2 | glutamate receptor, ionotropic, AMPA 2 | GRIA2 | P42262 |
| 93 | MAP2 | microtubule-associated protein 2 | MAP2 | P11137 |
| 94 | GFAP | glial fibrillary acidic protein | GFAP | P14136 |
| 95 | PEA15 | phosphoprotein enriched in astrocytes 15 | PEA15 | Q15121 |
| 96 | PLP | proteolipid protein 1 | PLP | P60 |
| 97 | GALC | galactosylceramidase | GALC | P54803 |
| 98 | MBP | myelin basic protein | MBP | P02686 |
| 99 | CNP | 2',3'-cyclic nucleotide 3' phosphodiesterase | CNP | P09543 |
| 100 | Olig2 | Oligodendrocyte transcription factor 2 | Olig2 | Q13516 |
| 101 | NES | Nestin | Nestin | Q48681 |
| 102 | Sox2 | Transcription factor SOX-2 | SOX2 | P48431 |
| 103 | FoxG1B | Foxhead box protein G1 | FOXG1B | P55316 |
| 104 | Pax6 | Paired box protein Pax-6 | PAX6 | P26367 |
| 105 | TH | Tyrosine 3-monooxygenase | TH | P07101 |
| 106 | CLDN6 | Claudin-6 | CLDN6 | P56747 |
| 107 | GATA4 | Transcription factor GATA-4 | GATA4 | P43694 |
| 108 | PDX1 | Pancreas/duodenum homeobox protein 1 | PDX-1 | P52945 |
| 109 | Krt20 | Keratin, type I cytoskeletal 20 | KRT20 | P35900 |
| 110 | KLF4 | Krueppel-like factor 4 | KLF4 | 043474 |
| 111 | Sox17 | Transcription factor SOX-17 | Sox17 | Q9H6I2 |
| 112 | FoxA2 | Hepatocyte nuclear factor 3-beta | FOXA2 | Q9Y261 |
| 113 | CXCR4 | C-X-C chemokine receptor type 4 | CXCR4 | P61073 |
| 114 | HNF4A | Hepatocyte nuclear factor 4-alpha | HNF4 | P41235 |
| 115 | DPP4 | Dipeptidyl peptidase 4 | DPP4 | P27487 |
| 116 | AFM | Afamin | ALB2 | P43652 |
| 117 | KRT19 | Keratin, type I cytoskeletal 19 | KRT19 | P08727 |
| 118 | KRT18 | Keratin, type I cytoskeletal 18 | KRT18 | P05783 |
| 119 | CYP7A1 | Cholesterol 7-alpha-monooxygenase | CYP7A1 | P22680 |
| 120 | CYP3A4 | Cytochrome P450 3A4 | CYP3A4 | P08684 |
| 121 | Cyp2B6 | Cytochrome P450 2B6 | CYP2B6 | P20813 |
| 122 | PCK1 | Phosphoenolpyruvate carboxykinase, cytosolic [GTP] | PCK1 | P35558 |
| 123 | PCK2 | Phosphoenolpyruvate carboxykinase [GTP], mitochondrial | PCK2 | Q16822 |
| 124 | TAT | Tyrosine aminotransferase | TAT | P17735 |
| 125 | TDO2 | Tryptophan 2,3-dioxygenase | TDO | P48775 |
| 126 | GalC | Galactocerebrosidase | GALC | P54803 |
| 127 | Mafa | Transcription factor MafA | MAFA | Q8NHW3 |
| 128 | NEUROG3 | Neurogenin-3 | NGN-3 | Q9Y4Z2 |
| 129 | RUNX1 | Runt-related transcription factor 1 | RUNX1 | Q01196 |
| 130 | myb (c-myb) | Transcriptional activator Myb | c-Myb | P10242 |
| 131 | VAV1 | Proto-oncogene vav | VAV1 | P15498 |
| 132 | GATA1 | Erythroid transcription factor | GATA1 | P15976 |
| 133 | LCLAT1 | Lysocardiolipin acyltransferase 1 | LCLAT1 | Q6UWP7 |
| 134 | CD34 | Hematopoietic progenitor cell antigen CD34 | CD34 | P28906 |
| 135 | PTPRC | Protein tyrosine phosphatase receptor type C-associated protein | CD45 | Q14761 |
| 136 | MNX1 | Motor neuron and pancreas homeobox protein 1 | HOXHB9 | P50219 |
| 137 | CD34 | | | |
| 138 | ICA1 | Islet cell autoantigen 1 | ICAp69 | Q05084 |
| 139 | MYEF-2 | Myelin expression factor 2 | MYEF-2 | Q9P2K5 |
| 140 | ChAT | choline O-acetyltransferase | ChAT | P28329 |
| 141 | ISLET (ISL1) | Insulin gene enhancer protein ISL-1 | ISLET | P61371 |
| 142 | NKX2-5 | NK2 transcription factor related, locus 5 | NKX2-5 | P52952 |
| 143 | EHMT1 (Brachyury) | euchromatic histone-lysine N-methyltransferase 1 | EHMT1 | Q9H9B1 |
| 144 | MyH6 | myosin, heavy chain 6, cardiac muscle, alpha | MYH6 | P13533 |
| 145 | TNNT2 | troponin T type 2 (cardiac) | TNNT2 | P45379 |
| 146 | Mixl1 | Homeobox protein MIXL1 | MIXL | Q9H2W2 |
| 147 | MLC2a | Myosin regulatory light chain 2, atrial isoform | MLC-2a | Q01449 |
| 148 | MLC2v | Myosin regulatory light chain 2, ventricular/cardiac muscle isoform | MLC-2v | P10916 |
| 149 | HCN4 | Potassium/sodium hyperpolarization-activated cyclic nucleotide-gated channel 4 | HCN4 | Q9Y3Q4 |
| 150 | Hey1 | Hairy/enhancer-of-split related with YRPW motif protein 1 | CHF-2 | Q9Y5J3 |
| 151 | Hey2 | Hairy/enhancer-of-split related with YRPW motif protein 2 | CHF-1 | Q9UBP5 |
| 152 | Mesp1 | Mesoderm posterior protein 1 | Mesp1 | Q9BRJ9 |
| 153 | GRE (glucocorticoid response element) | | | |
| 1-46 Cell signaling pathway related genes | | | | |
| 47-86 ADEM/Toxicity related genes | | | | |
| 89-152 Regenerative medicine/Stem cells related genes | | | | |

### (b) tag sequence

The tag refers herein to a protein that is fused to the endogenous protein to create the tagged endogenous proteins. The tag sequence is fused in-frame to the endogenous protein coding sequence such that a fusion protein is generated. In-frame means that the open reading frame (ORF) of the chromosomal sequence encoding the protein is maintained after the insertion of the tag sequence. In-frame insertions occur when the number of inserted nucleotides is divisible by three, which may be achieved by adding a linker of any number of nucleotides to the tag protein encoding sequence as applicable. The endogenous protein may be tagged anywhere within the protein polypeptide sequence provided the function of the endogenous protein is not affected. Generally tagging is at the N- or C-terminus of the protein. The endogenous protein may be tagged, for example, at the N-terminus of the protein. Alternatively, the endogenous protein may be tagged at the C-terminus of the protein.

A tag sequence may be any peptide sequence encoded by a nucleic acid sequence. Tag sequence may encode a variety of tags including, but not limited to, epitope tags, affinity tags, reporters, or combinations thereof.

The tag may be, for example, an epitope tag. The epitope tag may comprise a random amino acid sequence, or a known amino acid sequence. A known amino acid sequence may have, for example, antibodies generated against it, or there may be no known antibodies generated against the sequence. The epitope tag may be an antibody epitope tag for which commercial antibodies are available. Non-limiting examples of suitable antibody epitope tags are myc, AcV5, AU1, AU5, E, ECS, E2, FLAG, HA, Maltose binding protein, nus, Softag 1, Softag 3, Strep, SBP, Glu-Glu, HSV, KT3, S, S1, T7, V5, VSV-G, 6xHis, BCCP, and calmodulin.

An exemplary tag may be a reporter. Suitable reporters are known in the art. Non-limiting examples of reporters include affinity tags, visual reporters or selectable-marker reporters. Non-limiting examples of affinity tags include chitin binding protein (CBP), thioredoxin (TRX), poly(NANP), tandem affinity purification (TAP) tag, and glutathione-S-transferase (GST). Visual reporters typically result in a visual signal, such as a color change in the cell, or fluorescence or luminescence of the cell. For instance, the reporter LacZ, which encodes β-galactosidase, will turn a cell blue in the presence of a suitable substrate, such as X-gal. Other non-limiting examples of visual reporters include a fluorescent protein, luciferase, alkaline phosphatase, beta-galactosidase, beta-lactamase, horseradish peroxidase, and variants thereof. Additionally, luciferase may be used. Selectable-marker reporters typically confer a selectable trait to the cell, such as drug resistance (e.g. antibiotic resistance).

An exemplary tag is a fluorescent protein visual reporter. Non limiting examples of fluorescent protein visual reporters include green fluorescent proteins (e.g., GFP, GFP-2, tagGFP, turboGFP, EGFP, Emerald, Azami Green, Monomeric Azami Green, CopGFP, AceGFP, ZsGreen1), yellow fluorescent proteins (e.g. YFP, EYFP, Citrine, Venus, YPet, PhiYFP, ZsYellow1), blue fluorescent proteins (e.g. EBFP, EBFP2, Azurite, mKalama1, GFPuv, Sapphire, T-sapphire), cyan fluorescent proteins (e.g. ECFP, Cerulean, CyPet, AmCyan1, Midoriishi-Cyan), red fluorescent proteins (mKate, mKate2, mPlum, DsRed monomer, mCherry, mRFP1, DsRed-Express, DsRed2, DsRed-Monomer, HcRed-Tandem, HcRed1, AsRed2, eqFP611, mRasberry, mStrawberry, Jred), and orange fluorescent proteins (mOrange, mKO, Kusabira-Orange, Monomeric Kusabira-Orange, mTangerine, tdTomato) or any other suitable fluorescent protein. Exemplary tags are a green fluorescent protein, or a red fluorescent protein.

Non-limiting examples also include circular permutations of green fluorescent proteins, in which the amino and carboxyl portions are interchanged and rejoined with a short spacer connecting the original termini, while still being fluorescent. These circular permutations of fluorescent protein have altered pKa values and orientations of the chromophore. Furthermore, certain locations within some fluorescent proteins tolerate insertion of entire proteins, and conformational changes in the insert can have profound effects on the fluorescence, such as enhancement or changed colors. For example, insertions of calmodulin or a zinc finger domain in place of Tyr-145 of a yellow mutant (EYFP, enhanced yellow fluorescent protein) of GFP result in indicator proteins whose fluorescence can be enhanced several fold upon metal binding. The calmodulin graft into enhanced yellow fluorescent protein can monitor cytosolic Ca²⁺ in single mammalian cells.

The endogenous protein may be, for example, fused to the tag through a peptide linker. The sequence of the linker peptide is chosen based on known structural and conformational contributions of peptide segments to allow for proper folding and prevent possible steric hindrance of the protein to be tagged and the tag polypeptide. Linker peptides are commonly used and known in the art, and may be from about 3 to about 40 amino acids in length.

The endogenous protein also may be tagged with more than one tag. For instance, an endogenous protein may be tagged with at least one, two, three, four, five, six, seven, eight, or nine tags. More than one tag may be expressed as a single polypeptide fused to an endogenous protein of interest. More than one tag fused to an endogenous protein may be expressed as a single polypeptide which is cleaved into the individual tag polypeptides after translation. By way of non-limiting example, 2A peptides of picornaviruses inserted between tag polypeptides or between tag polypeptide and the endogenous protein may result in the co-translational 'cleavage' of a tag and lead to expression of multiple proteins at equimolar levels.

In one exemplary embodiment, the cell expresses one endogenous protein that is tagged with a fluorescent protein. In another exemplary embodiment, the cell expresses two fluorescently tagged endogenous proteins. In still another exemplary embodiment, the cell expresses three fluorescently tagged endogenous proteins. In an additional embodiment, the cell expresses four or more tagged endogenous proteins.

### (c) cell type

In general, the cell will be a eukaryotic cell. Suitable cells include fungi or yeast, such as *Pichia pastoris* or *Saccharomyces cerevisiae;* insect cells, such as SF9 cells from *Spodoptera frugiperda* or S2 cells from *Drosophila melanogaster,* plant cells; and animal cells, such as mouse, rat, hamster, non-human primate, or human cells. Exemplary cells are mammalian. The mammalian cells may be primary cells. In general, any primary cell that is sensitive to double strand breaks may be used. The cells may be of a variety of cell types, e.g., fibroblast, myoblast, T or B cell, macrophage, epithelial cell, and so forth.

The mammalian cell may be a mammalian cell line cell. The cell line may be any established cell line or a primary cell line. The cell line may be adherent or non-adherent, or the cell line may be grown under conditions that encourage adherent, non-adherent or organotypic growth using standard techniques known to individuals skilled in the art. Non-limiting examples of suitable mammalian cell lines include Chinese hamster ovary (CHO) cells, monkey kidney CVI line transformed by SV40 (COS7); human embryonic kidney line 293; baby hamster kidney cells (BHK); mouse sertoli cells (TM4); monkey kidney cells (CVI-76); African green monkey kidney cells (VERO-76); human cervical carcinoma cells (HELA); canine kidney cells (MDCK); buffalo rat liver cells (BRL 3A); human lung cells (W138); human liver cells (Hep G2); mouse mammary tumor cells (MMT); rat hepatoma cells (HTC); HIH/3T3 cells, the human U2-OS osteosarcoma cell line, the human A549 cell line, the human K562 cell line, the human HEK293 cell line, the human HEK293T cell line, and TRI cells. For an extensive list of mammalian cell lines, those of ordinary skill in the art may refer to the American Type Culture Collection catalog (ATCC®, Mamassas, VA). In general, the cells may be of a variety of cell types, e.g., fibroblast, myoblast, T or B cell, macrophage, epithelial cell, and so forth. An exemplary cell line according to the present disclosure is the human U2OS osteosarcoma cell line. Alternative exemplary human cell lines the cell line are the A549 cell line, the K562 cell line cell line, the HEK293 cell line, and the HEK293T cell line cell line. Another exemplary human cell line is the MCF10a, a breast epithelial cancer cell line. Yet another exemplary human cell line is the SKOV3, an epithelial cell line. Alternative exemplary cell lines include iPS cells, which are induced pluripotent stem cells generated from fibroblasts or other cell types.

In still other embodiments, the cell may be a stem cell. Suitable stem cells include without limit embryonic stem cells, ES-like stem cells, fetal stem cells, adult stem cells, pluripotent stem cells, induced pluripotent stem cells, multipotent stem cells, oligopotent stem cells, and unipotent stem cells.

In further embodiments, the cell may be a one-cell embryo. The embryo may be a vertebrate or an invertebrate. Suitable vertebrates include mammals, birds, reptiles, amphibians, and fish. Examples of suitable mammals include without limit rodents, companion animals, livestock, and non-primates. Non-limiting examples of rodents include mice, rats, hamsters, gerbils, and guinea pigs. Suitable companion animals include but are not limited to cats, dogs, rabbits, hedgehogs, and ferrets. Non-limiting examples of livestock include horses, goats, sheep, swine, cattle, Ilamas, and alpacas. Suitable non-primates include but are not limited to capuchin monkeys, chimpanzees, lemurs, macaques, marmosets, tamarins, spider monkeys, squirrel monkeys, and vervet monkeys. Non-limiting examples of birds include chickens, turkeys, ducks, and geese. Alternatively, the animal may be an invertebrate such as an insect, a nematode, and the like. Non-limiting examples of insects include *Drosophila* and mosquitoes.

### II. Method for Tagged Endogenous Protein(s)

Another aspect of the present disclosure encompasses a method for tagging at least one endogenous protein in a cell. The method comprises using a targeting endonuclease to mediate integration of a tag sequence in-frame with an endogenous coding sequence. More specifically, the method comprises introducing into a cell at least one zinc finger nuclease or nucleic acid encoding a zinc finger nuclease and at least one donor polynucleotide. The donor polynucleotide comprises a tag sequence to be integrated in-frame into the endogenous chromosomal sequence, an upstream sequence and a downstream sequence flanking the tag sequence, wherein the upstream and downstream sequences share substantial sequence identity with either side of the cleavage site in the endogenous chromosomal sequence encoding the protein. The cells are then maintained under conditions such that a double-stranded break introduced at the cleavage site by the zinc finger nuclease is repaired by a homology-directed process such that the tag sequence in the donor polynucleotide is integrated in-frame into the chromosomal sequence encoding the endogenous protein. Cells generated by the method that express at least one tagged endogenous protein are detailed above in section (I). Components of the method are described in more detail below.

### (a) targeting endonuclease

The method comprises, in part, introducing into a cell at least one targeting endonuclease or nucleic acid encoding a targeting endonuclease. The targeting endonuclease may be a naturally-occurring protein or an engineered protein. In some embodiments, the targeting endonuclease may be a meganuclease or a homing endonuclease. In other embodiments, the targeting endonuclease may be a transcription activator-like effector (TALE)-nuclease. In preferred embodiments, the targeting endonuclease may be a zinc finger nuclease. Typically, a zinc finger nuclease comprises a DNA binding domain (i.e., zinc finger) and a cleavage domain (i.e., nuclease), which are described below.

### (i) zinc finger binding domain

Zinc finger binding domains may be engineered to recognize and bind to any nucleic acid sequence of choice. See, for example, Beerli et al. (2002) Nat. Biotechnol. 20:135-141; Pabo et al. (2001) Ann. Rev. Biochem. 70:313-340; Isalan et al. (2001) Nat. Biotechnol. 19:656-660; Segal et al. (2001) Curr. Opin. Biotechnol. 12:632-637; Choo et al. (2000) Curr. Opin. Struct. Biol. 10:411-416; Zhang et al. (2000) J. Biol. Chem. 275(43):33850-33860; Doyon et al. (2008) Nat. Biotechnol. 26:702-708; and Santiago et al. (2008) Proc. Natl. Acad. Sci. USA 105:5809-5814. An engineered zinc finger binding domain may have a novel binding specificity compared to a naturally-occurring zinc finger protein. Engineering methods include, but are not limited to, rational design and various types of selection. Rational design includes, for example, using databases comprising doublet, triplet, and/or quadruplet nucleotide sequences and individual zinc finger amino acid sequences, in which each doublet, triplet or quadruplet nucleotide sequence is associated with one or more amino acid sequences of zinc fingers which bind the particular triplet or quadruplet sequence. See, for example, U.S. Pat. Nos. 6,453,242 and 6,534,261, the disclosures of which are incorporated by reference herein in their entireties. As an example, the algorithm of described in US patent 6,453,242 may be used to design a zinc finger binding domain to target a preselected sequence. Alternative methods, such as rational design using a nondegenerate recognition code table may also be used to design a zinc finger binding domain to target a specific sequence (Sera et al. (2002) Biochemistry 41:7074-7081). Publically available web-based tools for identifying potential target sites in DNA sequences and designing zinc finger binding domains may be found at http://www.zincfingertools.org and http://bindr.gdcb.iastate.edu/ZiFiT/, respectively (Mandell et al. (2006) Nuc. Acid Res. 34:W516-W523; Sander et al. (2007) Nuc. Acid Res. 35:W599-W605).

A zinc finger binding domain may be designed to recognize and bind a DNA sequence ranging from about 3 nucleotides to about 21 nucleotides in length, or from about 8 to about 19 nucleotides in length. In general, the zinc finger binding domains of the zinc finger nucleases disclosed herein comprise at least three zinc finger recognition regions (i.e., zinc fingers). In one embodiment, the zinc finger binding domain may comprise four zinc finger recognition regions. In another embodiment, the zinc finger binding domain may comprise five zinc finger recognition regions. In still another embodiment, the zinc finger binding domain may comprise six zinc finger recognition regions. A zinc finger binding domain may be designed to bind to any suitable target DNA sequence. See for example, U.S. Pat. Nos. 6,607,882; 6,534,261 and 6,453,242, the disclosures of which are incorporated by reference herein in their entireties.

Exemplary methods of selecting a zinc finger recognition region may include phage display and two-hybrid systems, and are disclosed in U.S. Pat. Nos. 5,789,538; 5,925,523; 6,007,988; 6,013,453; 6,410,248; 6,140,466; 6,200,759; and 6,242,568; as well as WO 98/37186; WO 98/53057; WO 00/27878; WO 01/88197 and GB 2,338,237, each of which is incorporated by reference herein in its entirety. In addition, enhancement of binding specificity for zinc finger binding domains has been described, for example, in WO 02/077227.

Zinc finger binding domains and methods for design and construction of fusion proteins (and polynucleotides encoding same) are known to those of skill in the art and are described in detail in U.S. Patent Application Publication Nos. 20050064474 and 20060188987, each incorporated by reference herein in its entirety. Zinc finger recognition regions and/or multi-fingered zinc finger proteins may be linked together using suitable linker sequences, including for example, linkers of five or more amino acids in length. See, U.S. Pat. Nos. 6,479,626; 6,903,185; and 7,153,949, the disclosures of which are incorporated by reference herein in their entireties, for non-limiting examples of linker sequences of six or more amino acids in length. The zinc finger binding domain described herein may include a combination of suitable linkers between the individual zinc fingers of the protein.

In some embodiments, the zinc finger nuclease may further comprise a nuclear localization signal or sequence (NLS). A NLS is an amino acid sequence which facilitates targeting the zinc finger nuclease protein into the nucleus to introduce a double stranded break at the target sequence in the chromosome. Nuclear localization signals are known in the art. See, for example, Makkerh et al. (1996) Current Biology 6:1025-1027.

An exemplary zinc finger DNA binding domain recognizes and binds a sequence having at least about 80% sequence identity to a sequence chosen from SEQ ID NO:1, 2, 13, 14, 18, 19, 22, 23, 25 and 26. In other embodiments, the sequence identity may be about 81%, 82%, 83%, 84%, 85%, 86%, 87%, 88%, 89%, 90%, 91%, 92%, 93%, 94%, 95%, 96%, 97%, 98%, 99%, or 100%.

### (ii) cleavage domain

A zinc finger nuclease also includes a cleavage domain. The cleavage domain portion of the zinc finger nucleases disclosed herein may be obtained from any endonuclease or exonuclease. Non-limiting examples of endonucleases from which a cleavage domain may be derived include, but are not limited to, restriction endonucleases and homing endonucleases. See, for example, 2002-2003 Catalog, New England Biolabs, Beverly, Mass.; and Belfort et al. (1997) Nucleic Acids Res. 25:3379-3388 or www.neb.com. Additional enzymes that cleave DNA are known (e.g., S1 Nuclease; mung bean nuclease; pancreatic DNase I; micrococcal nuclease; yeast HO endonuclease). See also Linn et al. (eds.) Nucleases, Cold Spring Harbor Laboratory Press, 1993. One or more of these enzymes (or functional fragments thereof) may be used as a source of cleavage domains.

A cleavage domain also may be derived from an enzyme or portion thereof, as described above, that requires dimerization for cleavage activity. Two zinc finger nucleases may be required for cleavage, as each nuclease comprises a monomer of the active enzyme dimer. Alternatively, a single zinc finger nuclease may comprise both monomers to create an active enzyme dimer. As used herein, an "active enzyme dimer" is an enzyme dimer capable of cleaving a nucleic acid molecule. The two cleavage monomers may be derived from the same endonuclease (or functional fragments thereof), or each monomer may be derived from a different endonuclease (or functional fragments thereof).

When two cleavage monomers are used to form an active enzyme dimer, the recognition sites for the two zinc finger nucleases are preferably disposed such that binding of the two zinc finger nucleases to their respective recognition sites places the cleavage monomers in a spatial orientation to each other that allows the cleavage monomers to form an active enzyme dimer, e.g., by dimerizing. As a result, the near edges of the recognition sites may be separated by about 5 to about 18 nucleotides. For instance, the near edges may be separated by about 5, 6, 7, 8, 9, 10, 11, 12, 13, 14, 15, 16, 17 or 18 nucleotides. It will however be understood that any integral number of nucleotides or nucleotide pairs may intervene between two recognition sites (e.g., from about 2 to about 50 nucleotide pairs or more). The near edges of the recognition sites of the zinc finger nucleases, such as for example those described in detail herein, may be separated by 6 nucleotides. In general, the site of cleavage lies between the recognition sites.

Restriction endonucleases (restriction enzymes) are present in many species and are capable of sequence-specific binding to DNA (at a recognition site), and cleaving DNA at or near the site of binding. Certain restriction enzymes (e.g., Type IIS) cleave DNA at sites removed from the recognition site and have separable binding and cleavage domains. For example, the Type IIS enzyme Fokl catalyzes double-stranded cleavage of DNA, at 9 nucleotides from its recognition site on one strand and 13 nucleotides from its recognition site on the other. See, for example, U.S. Pat. Nos. 5,356,802; 5,436,150 and 5,487,994; as well as Li et al. (1992) Proc. Natl. Acad. Sci. USA 89:4275-4279; Li et al. (1993) Proc. Natl. Acad. Sci. USA 90:2764-2768; Kim et al. (1994a) Proc. Natl. Acad. Sci. USA 91:883-887; Kim et al. (1994b) J. Biol. Chem. 269:31, 978-31, 982. Thus, a zinc finger nuclease may comprise the cleavage domain from at least one Type IIS restriction enzyme and one or more zinc finger binding domains, which may or may not be engineered. Exemplary Type IIS restriction enzymes are described for example in International Publication WO 07/014,275, the disclosure of which is incorporated by reference herein in its entirety. Additional restriction enzymes also contain separable binding and cleavage domains, and these also are contemplated by the present disclosure. See, for example, Roberts et al. (2003) Nucleic Acids Res. 31:418-420.

An exemplary Type IIS restriction enzyme, whose cleavage domain is separable from the binding domain, is Fokl. This particular enzyme is active as a dimer (Bitinaite et al. (1998) Proc. Natl. Acad. Sci. USA 95: 10, 570-10, 575). Accordingly, for the purposes of the present disclosure, the portion of the Fokl enzyme used in a zinc finger nuclease is considered a cleavage monomer. Thus, for targeted double-stranded cleavage using a Fokl cleavage domain, two zinc finger nucleases, each comprising a Fokl cleavage monomer, may be used to reconstitute an active enzyme dimer. Alternatively, a single polypeptide molecule containing a zinc finger binding domain and two Fokl cleavage monomers may also be used.

In certain embodiments, the cleavage domain may comprise one or more engineered cleavage monomers that minimize or prevent homodimerization, as described, for example, in U.S. Patent Publication Nos. 20050064474, 20060188987, and 20080131962, each of which is incorporated by reference herein in its entirety. By way of non-limiting example, amino acid residues at positions 446, 447, 479, 483, 484, 486, 487, 490, 491, 496, 498, 499, 500, 531, 534, 537, and 538 of Fokl are all targets for influencing dimerization of the Fokl cleavage half-domains. Exemplary engineered cleavage monomers of Fokl that form obligate heterodimers include a pair in which a first cleavage monomer includes mutations at amino acid residue positions 490 and 538 of Fokl and a second cleavage monomer that includes mutations at amino-acid residue positions 486 and 499.

Thus, in one embodiment, a mutation at amino acid position 490 replaces Glu (E) with Lys (K); a mutation at amino acid residue 538 replaces Iso (I) with Lys (K); a mutation at amino acid residue 486 replaces Gln (Q) with Glu (E); and a mutation at position 499 replaces Iso (I) with Lys (K). Specifically, the engineered cleavage monomers may be prepared by mutating positions 490 from E to K and 538 from I to K in one cleavage monomer to produce an engineered cleavage monomer designated "E490K:I538K" and by mutating positions 486 from Q to E and 499 from I to L in another cleavage monomer to produce an engineered cleavage monomer designated "Q486E:I499L." The above described engineered cleavage monomers are obligate heterodimer mutants in which aberrant cleavage is minimized or abolished. Engineered cleavage monomers may be prepared using a suitable method, for example, by site-directed mutagenesis of wild-type cleavage monomers (Fokl) as described in U.S. Patent Publication No. 20050064474 (see Example 5).

The zinc finger nuclease described above may be engineered to introduce a double stranded break at the targeted site of integration. The double stranded break may be at the targeted site of integration, or it may be up to 1, 2, 3, 4, 5, 10, 15, 20, 25, 30, 35, 40, 45, 50, 100, or 1000 nucleotides away from the site of integration. In some embodiments, the double stranded break may be up to 1, 2, 3, 4, 5, 10, 15, or 20 nucleotides away from the site of integration. In other embodiments, the double stranded break may be up to 10, 15, 20, 25, 30, 35, 40, 45, or 50 nucleotides away from the site of integration. In yet other embodiments, the double stranded break may be up to 50, 100, or 1000 nucleotides away from the site of integration.

### (iv) additional methods for targeted cleavage

Any nuclease having a target site in a chromosomal sequence may be used in the methods disclosed herein. For example, homing endonucleases and meganucleases have very long recognition sequences, some of which are likely to be present, on a statistical basis, once in a human-sized genome. Any such nuclease having a unique target site in a cellular genome may be used instead of, or in addition to, a zinc finger nuclease, for targeted cleavage of a cell chromosome.

Non-limiting examples of homing endonucleases include I-SceI, I-CeuI, PI-PspI, PI-Sce, I-SceIV, I-CsmI, I-PanI, I-SceII, I-PpoI, I-SceIII, I-CreI, I-Tevl, I-Tevll and I-TevIII. The recognition sequences of these enzymes are known in the art. See also U.S. Pat. No. 5,420,032; U.S. Pat. No. 6,833,252; Belfort et al. (1997) Nucleic Acids Res. 25:3379-3388; Dujon et al. (1989) Gene 82:115-118; Perler et al. (1994) Nucleic Acids Res. 22, 1125-1127; Jasin (1996) Trends Genet. 12:224-228; Gimble et al. (1996) J. Mol. Biol. 263:163-180; Argast et al. (1998) J. Mol. Biol. 280:345-353 and the New England Biolabs catalogue.

Although the cleavage specificity of most homing endonucleases is not absolute with respect to their recognition sites, the sites are of sufficient length that a single cleavage event per mammalian-sized genome may be obtained by expressing a homing endonuclease in a cell containing a single copy of its recognition site. It has also been reported that the specificity of homing endonucleases and meganucleases may be engineered to bind non-natural target sites. See, for example, Chevalier et al. (2002) Molec. Cell 10:895-905; Epinat et al. (2003) Nucleic Acids Res. 31:2952-2962; Ashworth et al. (2006) Nature 441:656-659; Paques et al. (2007) Current Gene Therapy 7:49-66.

### (v) nucleic acid encoding a zinc finger nuclease

The zinc finger nuclease may be introduced into the cell as a nucleic acid that encodes the zinc finger nuclease. The nucleic acid encoding a zinc finger nuclease may be DNA or RNA. In one embodiment, the nucleic acid encoding a zinc finger nuclease may DNA. For example, plasmid DNA comprising a zinc finger nuclease coding sequence may be introduced into the cell. In another embodiment, the nucleic acid encoding a zinc finger nuclease may be RNA or mRNA. When the nucleic acid encoding a zinc finger nuclease is mRNA, the mRNA molecule may be 5' capped. Similarly, when the nucleic acid encoding a zinc finger nuclease is mRNA, the mRNA molecule may be polyadenylated. Thus, a nucleic acid according to the method may be a capped and polyadenylated mRNA molecule encoding a zinc finger nuclease. Methods for capping and polyadenylating mRNA are known in the art.

### (b) donor polynucleotide

The method for integrating the tag sequence in-frame into a targeted chromosomal sequence further comprises introducing into the cell at least one donor polynucleotide comprising the tag sequence. A donor polynucleotide comprises not only the tag sequence, as detailed above in section (l)(b), but also comprises an upstream sequence and a downstream sequence. The upstream and downstream sequences flank the tag sequence in the donor polynucleotide. Furthermore, the upstream and downstream sequences share substantial sequence identity with either side of the site of integration in the endogenous chromosomal sequence.

The upstream and downstream sequences in the donor polynucleotide are selected to promote recombination between the targeted chromosomal sequence and the donor polynucleotide. The upstream sequence, as used herein, refers to a nucleic acid sequence that shares sequence similarity with the chromosomal sequence upstream of the targeted site of integration. Similarly, the downstream sequence refers to a nucleic acid sequence that shares sequence similarity with the chromosomal sequence downstream of the targeted site of integration. The upstream and downstream sequences in the donor polynucleotide may have about 75%, 80%, 85%, 90%, 95%, or 100% sequence identity with the targeted chromosomal sequence. In other embodiments, the upstream and downstream sequences in the donor polynucleotide may have about 95%, 96%, 97%, 98%, 99%, or 100% sequence identity with the targeted chromosomal sequence. In an exemplary embodiment, the upstream and downstream sequences in the donor polynucleotide may have about 99% or 100% sequence identity with the targeted chromosomal sequence.

An upstream or downstream sequence may comprise from about 20 bp to about 2500 bp. In one embodiment, an upstream or downstream sequence may comprise about 50, 100, 200, 300, 400, 500, 600, 700, 800, 900, 1000, 1100, 1200, 1300, 1400, 1500, 1600, 1700, 1800, 1900, 2000, 2100, 2200, 2300, 2400, or 2500 bp. An exemplary upstream or downstream sequence may comprise about 200 bp to about 2000 bp, about 600 bp to about 1000 bp, or more particularly about 700 bp to about 1000 bp.

Typically, the donor polynucleotide will be DNA. The donor polynucleotide may be a DNA plasmid, a bacterial artificial chromosome (BAC), a yeast artificial chromosome (YAC), a viral vector, a linear piece of DNA, a PCR fragment, a naked nucleic acid, or a nucleic acid complexed with a delivery vehicle such as a liposome or poloxamer. In one embodiment, the donor polynucleotide comprising the tag sequence may be a DNA plasmid. In another embodiment, the donor polynucleotide comprising the tag sequence may be a BAC.

One of skill in the art would be able to construct a donor polynucleotide as described herein using well-known standard recombinant techniques (see, for example, Sambrook et al., 2001 and Ausubel et al., 1996).

### (c) delivery to the cell

The method comprises introducing the targeting endonuclease or nucleic acid encoding the targeting endonuclease and the donor polynucleotide into a cell. Suitable cells are detailed above in section (l)(c).

Suitable delivery methods include microinjection, electroporation, sonoporation, biolistics, calcium phosphate-mediated transfection, cationic transfection, liposome transfection, dendrimer transfection, heat shock transfection, nucleofection transfection, magnetofection, lipofection, impalefection, optical transfection, proprietary agent-enhanced uptake of nucleic acids, and delivery via liposomes, immunoliposomes, virosomes, or artificial virions. In one embodiment, the molecules may be introduced into a cell by nucleofection. In another embodiment the molecules may be introduced into the by microinjection. The molecules may be microinjected into the nucleus or the cytoplasm of the cell.

The ratio of the donor polynucleotide comprising the tag sequence to the targeting endonuclease or nucleic acid encoding the targeting endonuclease can and will vary. In preferred embodiment, the targeting endonuclease may be a zinc finger nuclease. In general, the ratio of the donor polynucleotide to the zinc finger nuclease molecule may range from about 1:10 to about 10:1. In various embodiments, the ratio of donor polynucleotide to zinc finger nuclease molecules may be about 1:10, 1:9, 1:8, 1:7, 1:6, 1:5, 1:4, 1:3, 1:2, 1:1,2:1,3:1,4:1, 5:1, 6:1, 7:1, 8:1, 9:1, or 10:1. In one embodiment, the ratio may be about 1:1.

In embodiments in which more than one targeting endonuclease molecule and more than one donor polynucleotide are introduced into a cell, the molecules may be introduced simultaneously or sequentially. For example, targeting endonuclease molecules, each specific for a distinct recognition sequence, as well as the corresponding donor polynucleotides, may be introduced at the same time. Alternatively, each targeting endonuclease molecule, as well as the corresponding donor polynucleotide, may be introduced sequentially.

### (d) culturing the cell

The method further comprises maintaining the cell under appropriate conditions such that the targeting endonuclease-mediated integration may occur. The cell may be cultured using standard procedures to allow expression of the targeting endonuclease, if necessary. Standard cell culture techniques are described, for example, in Santiago et al. (2008) PNAS 105:5809-5814; Moehle et al. (2007) PNAS 104:3055-3060; Urnov et al. (2005) Nature 435:646-651; and Lombardo et al (2007) Nat. Biotechnology 25:1298-1306. Those of skill in the art appreciate that methods for culturing cells are known in the art and can and will vary depending on the cell type. Routine optimization may be used, in all cases, to determine the best techniques for a particular cell type.

In embodiments in which the cell is a one-cell embryo, the embryo may be cultured *in vitro* (e.g., in cell culture). Typically, the embryo is cultured at an appropriate temperature and in appropriate media with the necessary O₂/CO₂ ratio to allow the expression of the zinc finger nuclease. Suitable non-limiting examples of media include M2, M16, KSOM, BMOC, and HTF media. A skilled artisan will appreciate that culture conditions can and will vary depending on the species of embryo. Routine optimization may be used, in all cases, to determine the best culture conditions for a particular species of embryo. In some instances, the embryo also may be cultured *in vivo* by transferring the embryo into the uterus of a female host. Generally speaking the female host is from the same or similar species as the embryo. Preferably, the female host is pseudo-pregnant. Methods of preparing pseudo-pregnant female hosts are known in the art. Additionally, methods of transferring an embryo into a female host are known. Culturing an embryo *in vivo* permits the embryo to develop and may result in a live birth of an animal derived from the embryo.

During this step of the process, the targeting endonuclease (which in some case is expressed from the introduced nucleic acid) recognizes, binds, and cleaves the target sequence in the chromosome. The double-stranded break introduced by the targeting endonuclease is repaired, via homologous recombination with the donor polynucleotide, such that the tag sequence of the donor polynucleotide is integrated in-frame into the chromosomal location. The donor polynucleotide may be physically integrated or, alternatively, the donor polynucleotide may be used as a template for repair of the break, resulting in the integration of the tag sequence as well as all or part of the upstream and downstream sequences of the donor polynucleotide into the chromosome. A skilled artisan will appreciate that methods for culturing of cells are known in the art and can and will vary depending on the cell type. Routine optimization may be used, in all cases, to determine the best techniques for a particular cell type.

### (e) multiple integrations

A further embodiment of the above invention comprises performing a method of the invention serially, such that a cell is developed with more than one targeted integration such that more than one endogenous protein is tagged. For instance, a cell with a first targeted integration may then be used in a method of the invention to create a second targeted integration. The same process may be repeated to create a cell with three, four, five, six, seven, eight, nine, ten or more than ten targeted integrations.

Alternatively, a cell with multiple integrations may be developed by introducing more than one targeting endonuclease, each specific for a distinct site of integration, and introducing a corresponding number of donor polynucleotides. Each donor polynucleotide would comprise a nucleic acid sequence to be integrated and an upstream and downstream sequence homologous to the chromosomal site of integration as detailed above. The number of targeting endonucleases and corresponding donor polynucleotides injected into a cell may be two, three, four, five or more than five.

### III. Kit for tagging endogenous protein.

The present disclosure also encompasses a kit for monitoring the localization of at least one endogenous protein in a cell. The kit comprises a cell having at least one tag sequence integrated in-frame into a chromosomal sequence encoding an endogenous protein, such that the cell expresses at least one tagged endogenous protein. The cell may be a mammalian cell. Preferably, the cell is a human cell. The human cell may be a cell line cell chosen from a human U2OS cell, a human MCF10A, a human SKOV3, or a human iPS. The tagged endogenous protein may be chosen from tubulin, actin, lamin, HER2, and HMGA. Alternatively, the kit may express at least one tagged endogenous protein chosen from those listed in TABLE A. In preferred embodiments, the tag of the endogenous protein may be a fluorescent protein chosen from a green fluorescent protein, a blue fluorescent protein, a cyan fluorescent protein, a yellow fluorescent protein, an orange fluorescent protein, and a red fluorescent protein. Exemplary tags are green fluorescent and red fluorescent proteins.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

When introducing elements of the present disclosure or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

A "gene," as used herein, refers to a DNA region (including exons and introns) encoding a gene product, as well as all DNA regions which regulate the production of the gene product, whether or not such regulatory sequences are adjacent to coding and/or transcribed sequences. Accordingly, a gene includes, but is not necessarily limited to, promoter sequences, terminators, translational regulatory sequences such as ribosome binding sites and internal ribosome entry sites, enhancers, silencers, insulators, boundary elements, replication origins, matrix attachment sites, and locus control regions.

A "heterologous protein" is a protein that is not native (i.e., foreign) to the cell or organism of interest.

The terms "nucleic acid" and "polynucleotide" refer to a deoxyribonucleotide or ribonucleotide polymer, in linear or circular conformation, and in either single- or double-stranded form. For the purposes of the present disclosure, these terms are not to be construed as limiting with respect to the length of a polymer. The terms can encompass known analogs of natural nucleotides, as well as nucleotides that are modified in the base, sugar and/or phosphate moieties (e.g., phosphorothioate backbones). In general, an analog of a particular nucleotide has the same base-pairing specificity; i.e., an analog of A will base-pair with T.

The terms "polypeptide" and "protein" are used interchangeably to refer to a polymer of amino acid residues.

The term "recombination" refers to a process of exchange of genetic information between two polynucleotides. For the purposes of this disclosure, "homologous recombination" refers to the specialized form of such exchange that takes place, for example, during repair of double-strand breaks in cells. This process requires sequence similarity between the two polynucleotides, uses a "donor" or "exchange" molecule to template repair of a "target" molecule (i.e., the one that experienced the double-strand break), and is variously known as "non-crossover gene conversion" or "short tract gene conversion," because it leads to the transfer of genetic information from the donor to the target. Without being bound by any particular theory, such transfer can involve mismatch correction of heteroduplex DNA that forms between the broken target and the donor, and/or "synthesis-dependent strand annealing," in which the donor is used to resynthesize genetic information that will become part of the target, and/or related processes. Such specialized homologous recombination often results in an alteration of the sequence of the target molecule such that part or all of the sequence of the donor polynucleotide is incorporated into the target polynucleotide.

The term "sequence identity" refers to the extent in which two nucleotide sequences are invariant, i.e., the two sequences have the same nucleotide at the same position. Sequence identity is generally expressed as a percentage. Two nucleotide sequences that are identical in sequence and length have 100% sequence identity.

As used herein, the terms "target site" or "target sequence" refer to a nucleic acid sequence that defines a portion of a chromosomal sequence to be edited and to which a zinc finger nuclease is engineered to recognize and bind, provided sufficient conditions for binding exist.

Techniques for determining nucleic acid and amino acid sequence identity are known in the art. Typically, such techniques include determining the nucleotide sequence of the mRNA for a gene and/or determining the amino acid sequence encoded thereby, and comparing these sequences to a second nucleotide or amino acid sequence. Genomic sequences can also be determined and compared in this fashion. In general, identity refers to an exact nucleotide-to-nucleotide or amino acid-to-amino acid correspondence of two polynucleotides or polypeptide sequences, respectively. Two or more sequences (polynucleotide or amino acid) can be compared by determining their percent identity. The percent identity of two sequences, whether nucleic acid or amino acid sequences, is the number of exact matches between two aligned sequences divided by the length of the shorter sequences and multiplied by 100. An approximate alignment for nucleic acid sequences is provided by the local homology algorithm of Smith and Waterman, Advances in Applied Mathematics 2:482-489 (1981). This algorithm can be applied to amino acid sequences by using the scoring matrix developed by Dayhoff, Atlas of Protein Sequences and Structure, M. O. Dayhoff ed., 5 suppl. 3:353-358, National Biomedical Research Foundation, Washington, D.C., USA, and normalized by Gribskov, Nucl. Acids Res. 14(6):6745-6763 (1986). An exemplary implementation of this algorithm to determine percent identity of a sequence is provided by the Genetics Computer Group (Madison, Wis.) in the "BestFit" utility application. Other suitable programs for calculating the percent identity or similarity between sequences are generally known in the art, for example, another alignment program is BLAST, used with default parameters. For example, BLASTN and BLASTP can be used using the following default parameters: genetic code=standard; filter=none; strand=both; cutoff=60; expect=10; Matrix=BLOSUM62; Descriptions=50 sequences; sort by=HIGH SCORE; Databases=non-redundant, GenBank+EMBL+DDBJ+PDB+GenBank CDS translations+Swiss protein+Spupdate+PIR. Details of these programs can be found on the GenBank website. With respect to sequences described herein, the range of desired degrees of sequence identity is approximately 80% to 100% and any integer value therebetween. Typically the percent identities between sequences are at least 70-75%, preferably 80-82%, more preferably 85-90%, even more preferably 92%, still more preferably 95%, and most preferably 98% sequence identity.

Alternatively, the degree of sequence similarity between polynucleotides can be determined by hybridization of polynucleotides under conditions that allow formation of stable duplexes between regions that share a degree of sequence identity, followed by digestion with single-stranded-specific nuclease(s), and size determination of the digested fragments. Two nucleic acid, or two polypeptide sequences are substantially similar to each other when the sequences exhibit at least about 70%-75%, preferably 80%-82%, more-preferably 85%-90%, even more preferably 92%, still more preferably 95%, and most preferably 98% sequence identity over a defined length of the molecules, as determined using the methods above. As used herein, substantially similar also refers to sequences showing complete identity to a specified DNA or polypeptide sequence. DNA sequences that are substantially similar can be identified in a Southern hybridization experiment under, for example, stringent conditions, as defined for that particular system. Defining appropriate hybridization conditions is within the skill of the art. See, e.g., Sambrook et al., supra; Nucleic Acid Hybridization: A Practical Approach, editors B. D. Hames and S. J. Higgins, (1985) Oxford; Washington, D.C.; IRL Press).

Selective hybridization of two nucleic acid fragments can be determined as follows. The degree of sequence identity between two nucleic acid molecules affects the efficiency and strength of hybridization events between such molecules. A partially identical nucleic acid sequence will at least partially inhibit the hybridization of a completely identical sequence to a target molecule. Inhibition of hybridization of the completely identical sequence can be assessed using hybridization assays that are well known in the art (e.g., Southern (DNA) blot, Northern (RNA) blot, solution hybridization, or the like, see Sambrook, et al., Molecular Cloning: A Laboratory Manual, Second Edition, (1989) Cold Spring Harbor, N.Y.). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a reference nucleic acid sequence, and then by selection of appropriate conditions the probe and the reference sequence selectively hybridize, or bind, to each other to form a duplex molecule. A nucleic acid molecule that is capable of hybridizing selectively to a reference sequence under moderately stringent hybridization conditions typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/reference sequence hybridization, where the probe and reference sequence have a specific degree of sequence identity, can be determined as is known in the art (see, for example, Nucleic Acid Hybridization: A Practical Approach, editors B. D. Hames and S. J. Higgins, (1985) Oxford; Washington, D.C.; IRL Press). Conditions for hybridization are well-known to those of skill in the art.

Hybridization stringency refers to the degree to which hybridization conditions disfavor the formation of hybrids containing mismatched nucleotides, with higher stringency correlated with a lower tolerance for mismatched hybrids. Factors that affect the stringency of hybridization are well-known to those of skill in the art and include, but are not limited to, temperature, pH, ionic strength, and concentration of organic solvents such as, for example, formamide and dimethylsulfoxide. As is known to those of skill in the art, hybridization stringency is increased by higher temperatures, lower ionic strength and lower solvent concentrations. With respect to stringency conditions for hybridization, it is well known in the art that numerous equivalent conditions can be employed to establish a particular stringency by varying, for example, the following factors: the length and nature of the sequences, base composition of the various sequences, concentrations of salts and other hybridization solution components, the presence or absence of blocking agents in the hybridization solutions (e.g., dextran sulfate, and polyethylene glycol), hybridization reaction temperature and time parameters, as well as, varying wash conditions. A particular set of hybridization conditions may be selected following standard methods in the art (see, for example, Sambrook, et al., Molecular Cloning: A Laboratory Manual, Second Edition, (1989) Cold Spring Harbor, N.Y.).

### EXAMPLES

The following examples are included to demonstrate preferred embodiments of the invention.

### Example 1. Tagging endogenous α-tubulin isoform 1B protein.

The endogenous α-tubulin isoform 1 B protein was tagged with GFP using ZFN-induced homologous recombination. In short, ZFNs were used to introduce a double-stranded break in the chromosome region encoding α-tubulin isoform 1 B encoded by the TUBA1B locus. The double stranded break induces homologous recombination with a donor polynucleotide comprising the GFP coding sequence flanked by nucleic acid sequences homologous to the TUBA1B locus chromosome region, and resulting in the integration of the GFP coding region into the chromosome. The donor polynucleotide was constructed to fuse the GFP tag in-frame with the α-tubulin isoform 1 B coding sequence to produce a protein tagged with GFP at the N-terminus. GFP-tagged α-tubulin isoform 1 B protein was expressed under the control of the endogenous Tubulin promoter.

A pair of ZFNs was designed for the targeted integration of a tag into TUBA1B target site. For more information see Science (2009) 325:433, herein incorporated by reference in its entirety. The frequency of targeted ZFN pair double stranded break generation in ZFN-treated pools of cells was determined by using the Cel-1 nuclease assay. This assay detects alleles of the target locus that deviate from wild type as a result of non-homologous end joining (NHEJ)-mediated imperfect repair of ZFN-induced DNA double strand breaks. PCR amplification of the targeted region from a pool of ZFN-treated cells generates a mixture of WT and mutant amplicons. Melting and reannealing of this mixture results in mismatches forming between heteroduplexes of the WT and mutant alleles. A DNA "bubble" formed at the site of mismatch is cleaved by the surveyor nuclease Cel-1, and the cleavage products can be resolved by gel electrophoresis. The relative intensity of the cleavage products compared with the parental band is a measure of the level of Cel-1 cleavage of the heteroduplex. This, in turn, reflects the frequency of ZFN-mediated cleavage of the endogenous target locus that has subsequently undergone imperfect repair by NHEJ. For the ZFN pair used to tag α-tubulin isoform 1 B protein, one ZFN was designed to bind the 5' CTTCGCCTCCTAATC 3' (SEQ ID NO:1) sequence, and the other ZFN was designed to bind the 5' CACTATGGTGAGTAA 3' (SEQ ID NO:2) sequence **(****Fig. 1A****).** Capped, polyadenylated mRNAs encoding the ZFN pair was then produced using known molecular biology techniques. Upon binding, the ZFN pair introduces a double-stranded break in the CCTAGC chromosome sequence between the recognition sites **(****Fig. 1A and 1B**) to induce homologous recombination.

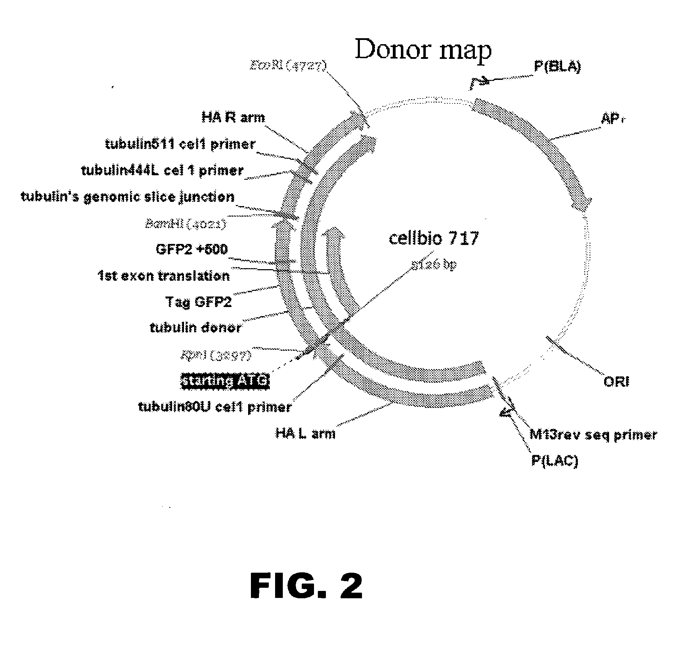

A plasmid **(****Fig. 2****)** was constructed as a polynucleotide donor for the targeted integration of a GFP tag into the TUBA1 B locus of the U2OS human cell line. The plasmid comprised the GFP coding sequence flanked by 1 Kb and 700 base pairs of TUBA1 B locus sequence upstream and downstream of the cut site introduced by the ZFN pair **(****Fig. 1C and D****).** The tag sequence in the plasmid was fused to the upstream and downstream the TUBA1B locus in such a manner that, when the TUBA1 B locus is expressed, a α-tubulin isoform 1 B protein fused to the GFP tag at the N-terminus is produced as detailed in **Fig 1E****.** The GFP-Tubulin fusion was also designed such that the splice signal of the first exon of the TUBA1 B locus, where the GFP coding sequence was introduced, was kept intact.

### Tagging of Tubulin in U2OS cells.

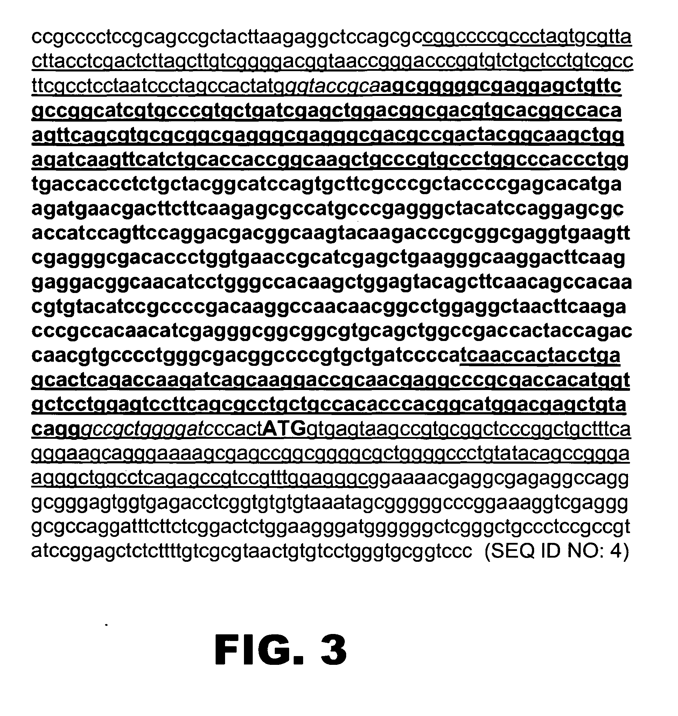

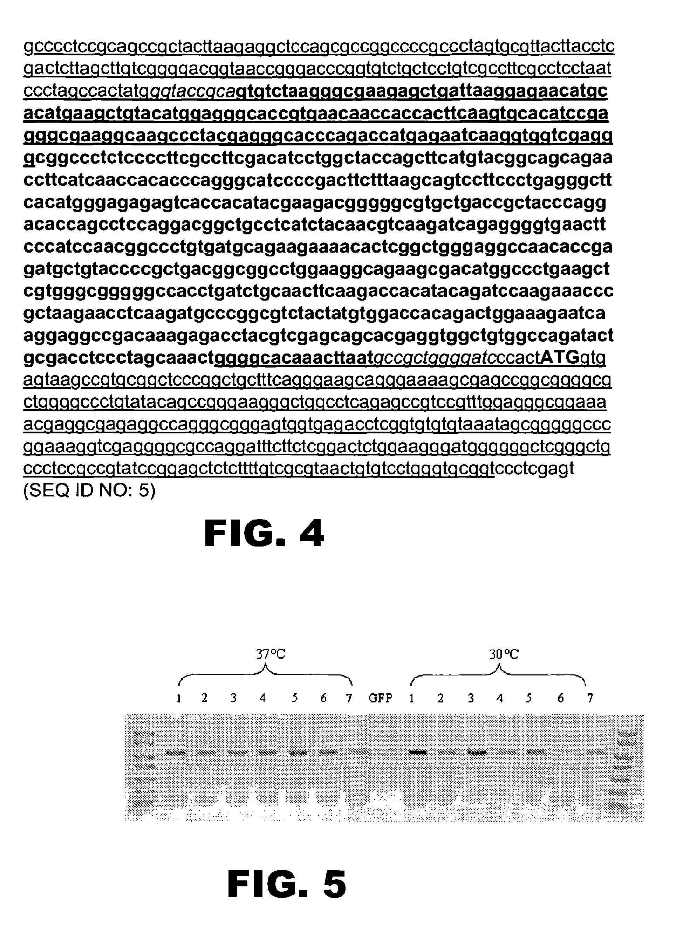

The donor plasmid and the pair of RNAs encoding ZFNs were transfected into U2OS, A549, K562, HEK293, MCF10a, or HEK293T cells. The nucleic acid mixture comprised one part donor DNA to one part ZFN RNAs. The transfected cells were then cultured and individual cell clones were analyzed. Junction PCR performed at 37°C and 30°C was used to confirm the donor DNA was integrated in the Tubulin TUBA1 B locus. Sequence analysis confirmed that the GFP2 sequence was integrated into the TUBA1 B locus in USOS cells, as shown in **Fig. 3** (SEQ ID NO: 4). The confirmed integration of RFP sequence in the TUBA1 B region in U2OS cells is shown in **Fig. 4** (SEQ ID NO: 5).

PCR analysis using primers that flanked the right junction confirmed integration. For this, 100 ng of template DNA was amplified in a 25 µl reaction mixture (26 cycles of 95°C, 5 min; 95°C, 30 sec; 51°C, 30 sec; 70°C, 1.1 min; 70°C, 7 min; 4°C, hold). **Fig 5** shows that fourteen cell clones comprised the PCR fragment size indicative of GFP integration. Fluorescent microscopy was then used to visualize the GFP-tagged α-tubulin isoform 1 B protein U2OS cells **(****Fig. 6A-C****),** A549 cells **(****FIG. 6D-E****),** K562 cells **(****FIG. 6F****),** and HEK293 cells **(****FIG. 6G-H****).**

### Tagging of Tubulin in MCF10a cell line.

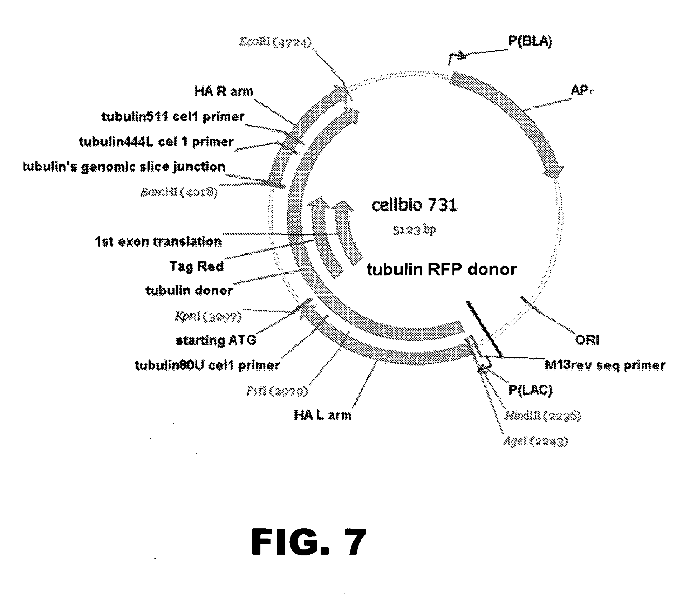

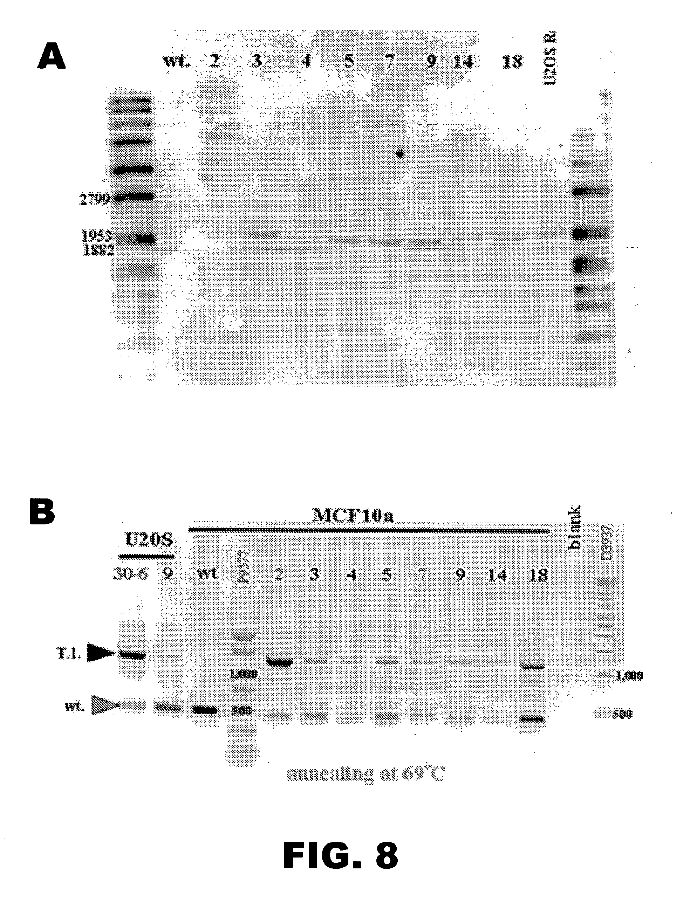

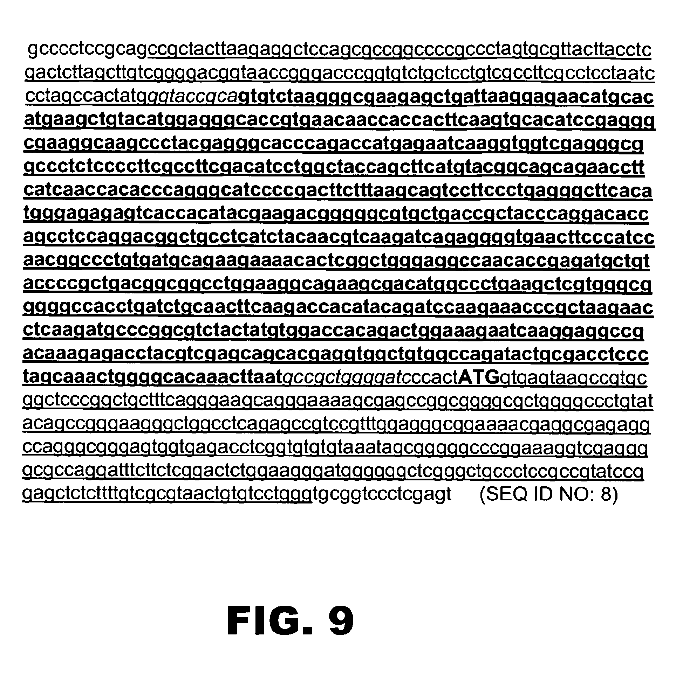

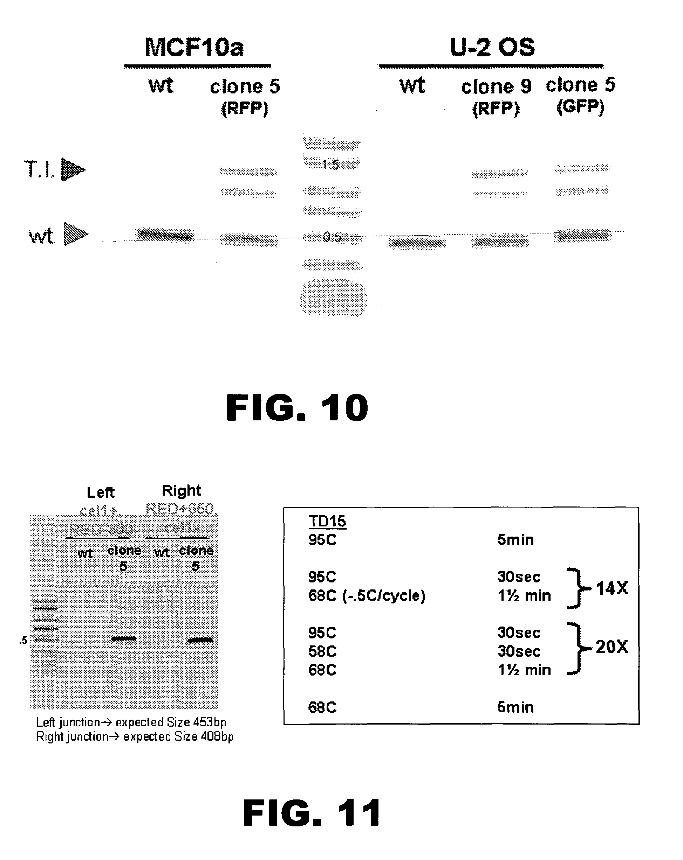

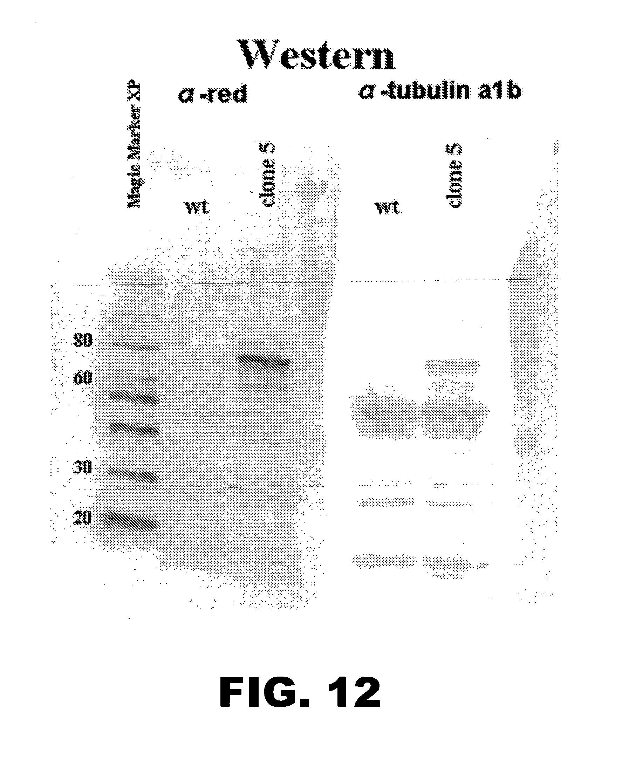

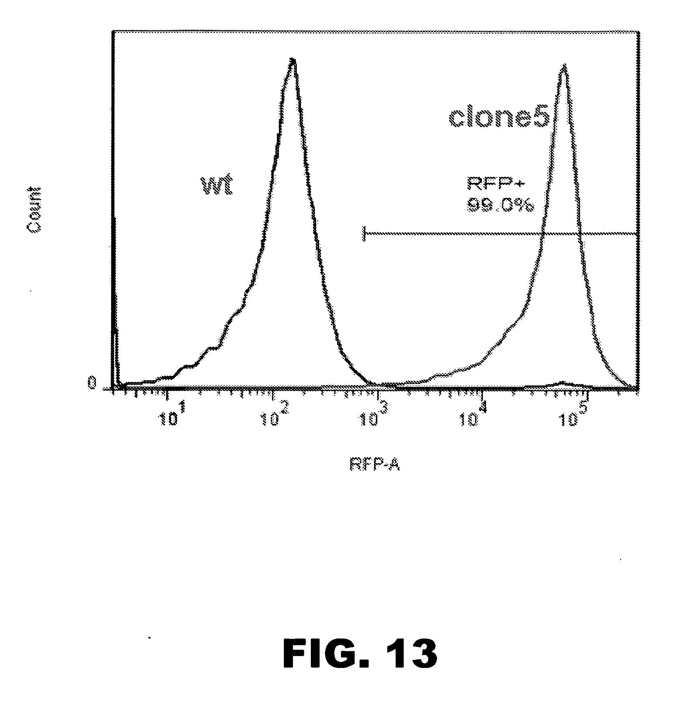

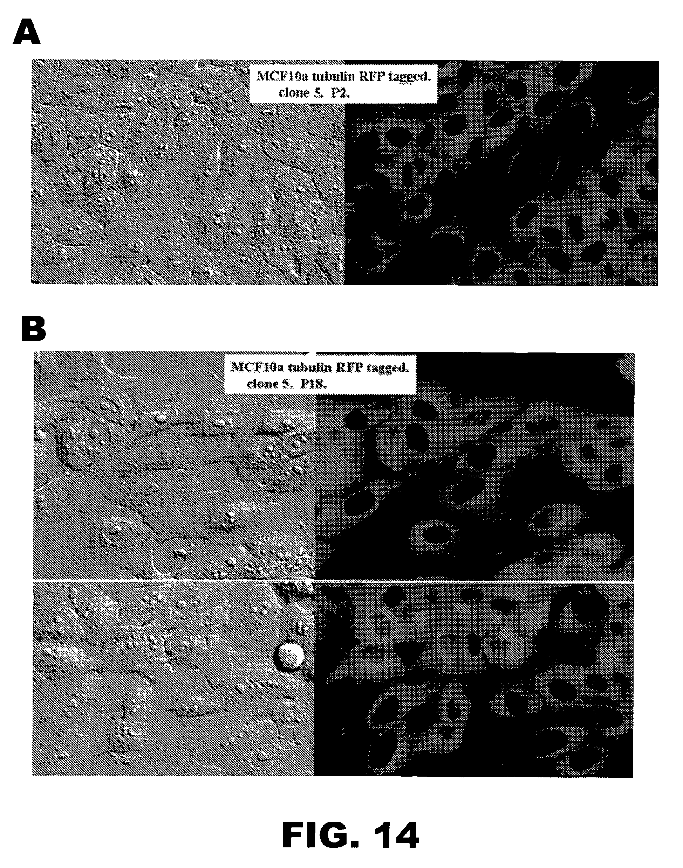

A plasmid **(****Fig. 7****)** was constructed as a polynucleotide donor for the targeted integration of a RFP tag into the TUBA1 B locus of the MCF10a human cell line. The RFP tag integration into the TUBA1 B locus MCF10a cells was verified by genomic PCR and junction PCR using Tubulin primers: 5' CCCCTCCGCAGCCGCTACT 3' (SEQ ID NO:6; tub80U) and 5' GGACCGCACCCAGGACACAGT 3' (SEQ ID NO:7; tub511 L). Genomic PCR and Southern blotting indicated the integration of RFP tag into TUBA1B in several clones **(****Fig. 8****).** Sequence analysis confirming integration of the tag sequence into the TUBA1 B locus in MCF10a cell line is shown in **Fig. 9** (SEQ ID NO: 8). Clone 5 of transfected MCF10a was selected for further verification **(****Fig. 10****)**.In the Jumpstart PCR verification of the RFP integration: 95 ng of genomic DNA (wild-type and clone 5 of MCF10a cells, and wild-type and clones 9, 5 of U2OS cells) was amplified (35X, annealing at 69°C and extension at 72°C using tub80U and tub522L primers). Transfected MCF10a Clone 5 was confirmed to have the integrated sequence (see FIG. 10). The sizes of the left and right junctions of MCF10a clone 5 were confirmed using RFP-specific and tubulin-specific primers, and were found to be the expected sizes of 452 base pairs and 408 base pairs, respectively **(****Fig. 11****).** Expression of RFP-tubulin protein was verified through Western blotting **(****Fig. 12****).** Blots were probed with either anti-RFP or anti-tubulin antibodies. The RFP expression was also observed with fluorescent microscopy and it was observed to co-localize with the endogenous TUBA1B expression **(****Fig 13****).** The growth characteristics of the transfected MCF10a cells were compared to the parental cell line. The doubling time of transfected MCF10a cells was +/- 20% of that of parental cell line. The phenotype stability of the transfected MCF10a cells was assessed. It was observed that after 8 weeks and 16 splits, 99% of the cells maintained RFP signal **(Table 1).** Fluorescence microscopy confirmed the expression of RFP-tagged tubulin in MCF10a clone 5 cells **(****Fig. 14****).**

| **Table 1** | | |
|---|---|---|
| | RFP clone5 | Wt MCF10a |
| Viability at P0 | 90% | 100% |
| Viability at P10 | 99% | 98% |
| Viability at P17 | 98% | 99% |
| Flow analysis of "RED" at P17 | 99% | 2% |

### Example 2. Attempt to tag signal transducer and activator of transcription 3 protein encoded by STAT3.

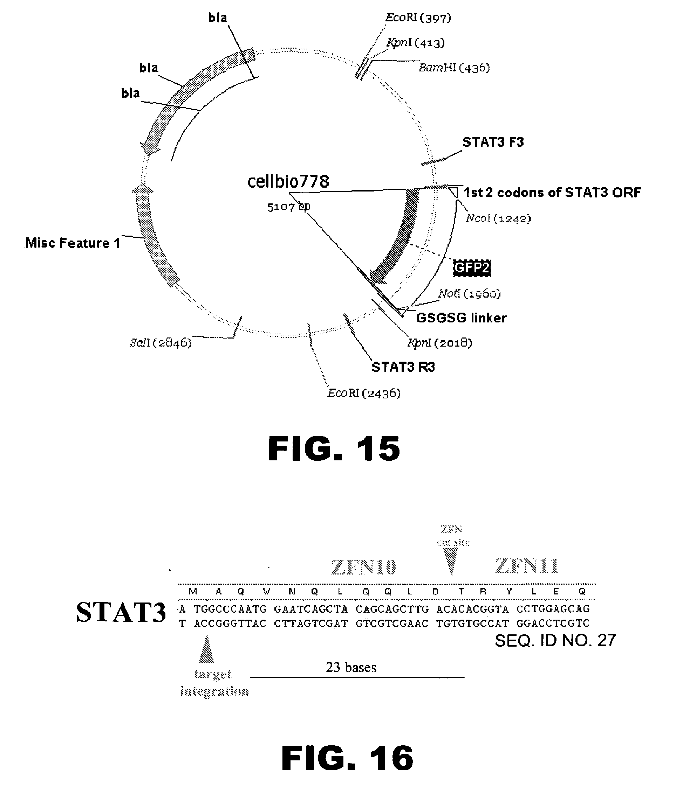

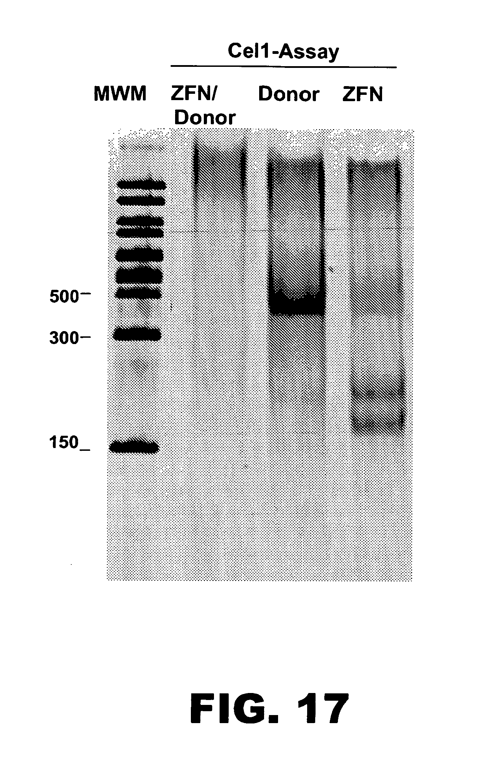

An attempt to produce a GFP or RFP-tagged signal transducer and activator of transcription 3 protein encoded by STAT3 was not successful. A donor plasmid comprising upstream and downstream STAT3 locus sequences flanking a polynucleotide encoding GFP or RFP fused to the N-terminus of the signal transducer protein was produced **(****Fig. 15****).** ZFNs were designed as described in the example above. One ZFN was designed to bind the 5' AGCTACAGCAGCTTG 3' (SEQ ID NO:9) sequence, and another ZNF was designed to bind the 5' CGGTACCTGGAGCAG 3' (SEQ ID NO:10) sequence comprising the STAT3 locus **(****Fig. 16****).** The Cel-1 assay described above was used to confirm the ZFN pair efficiently cut the STAT3 locus at the proper site **(****Fig. 17****).**

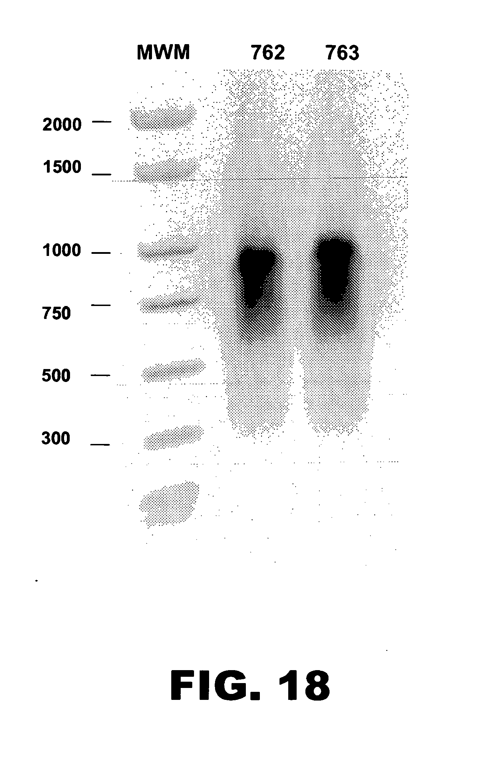

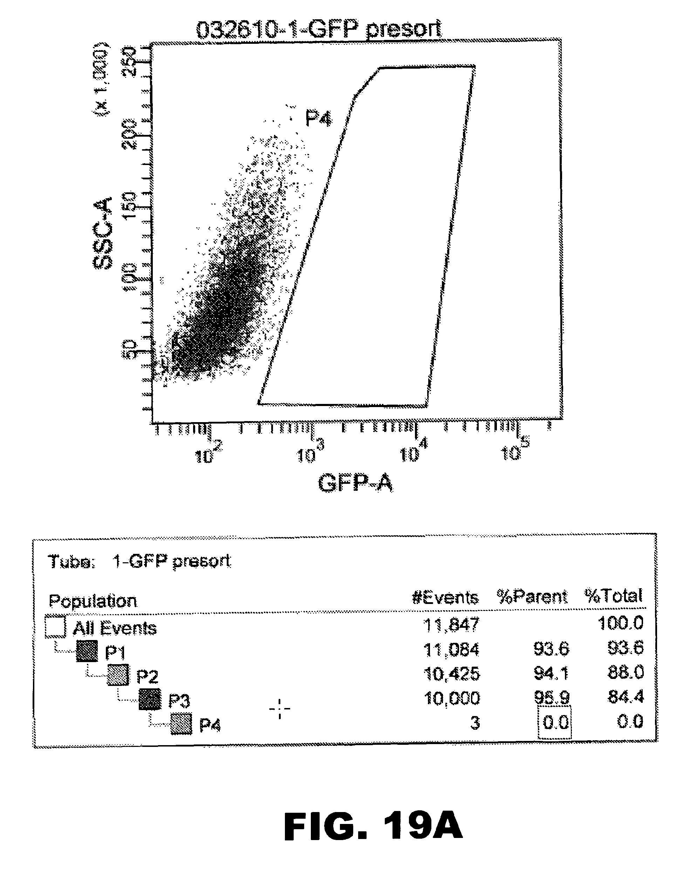

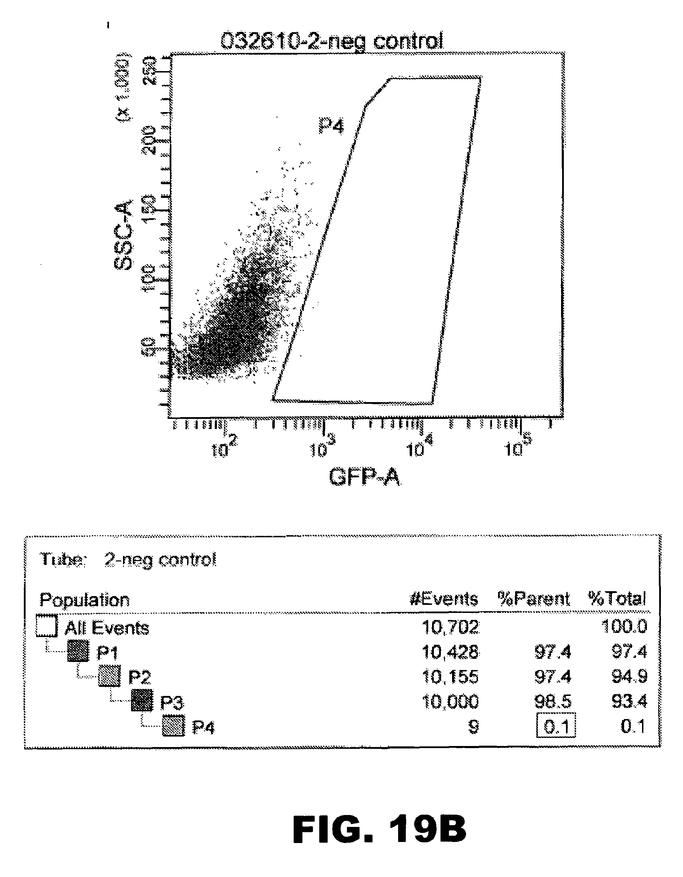

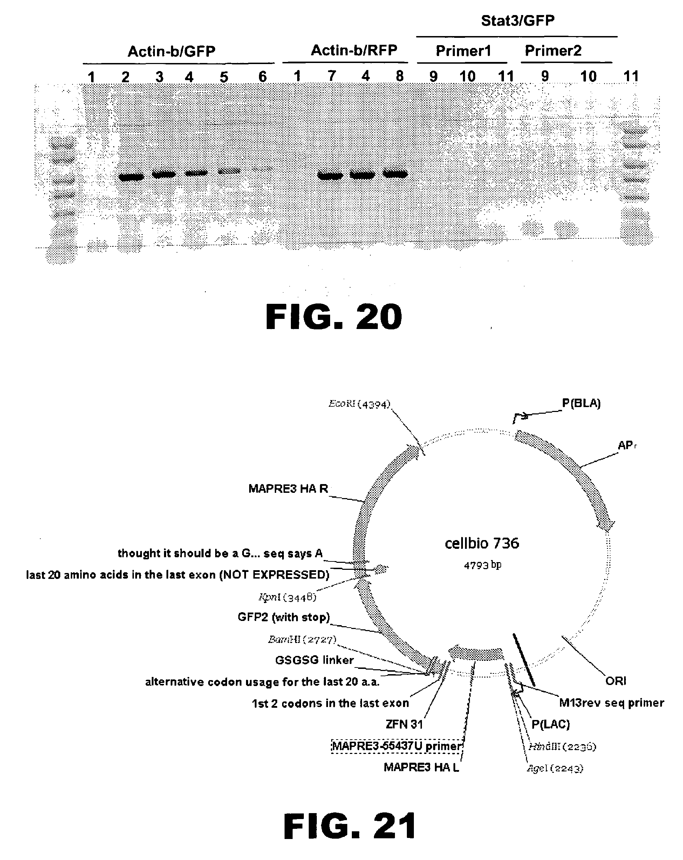

The donor plasmid and the pair of RNAs encoding ZFNs **(****Fig. 18****)** were transfected into cells. Fluorescent activated cell sorting (FACS) analysis showed that no fluorescent signal was detected, and therefore the targeted integration was not successful **(****Fig. 19****).** These results were confirmed by junction PCR analysis which failed to detect any targeted integration of GFP within the STAT3 locus, while detecting targeted integration of tag sequences encoding GFP and RFP at the ACTB locus encoding β-actin protein. **(****Fig. 20****).**

Therefore, even though the ZFN pair designed was able to introduce a double-stranded break into the correct chromosomal location, integration of the GFP tag was not achieved.

### Example 3. Attempt to tag microtubule-associated protein RP/EB family member 3 encoded by MAPRE3.

An attempt to produce a GFP-tagged microtubule-associated protein RP/EB family member 3 encoded by MAPRE3 was not successful.

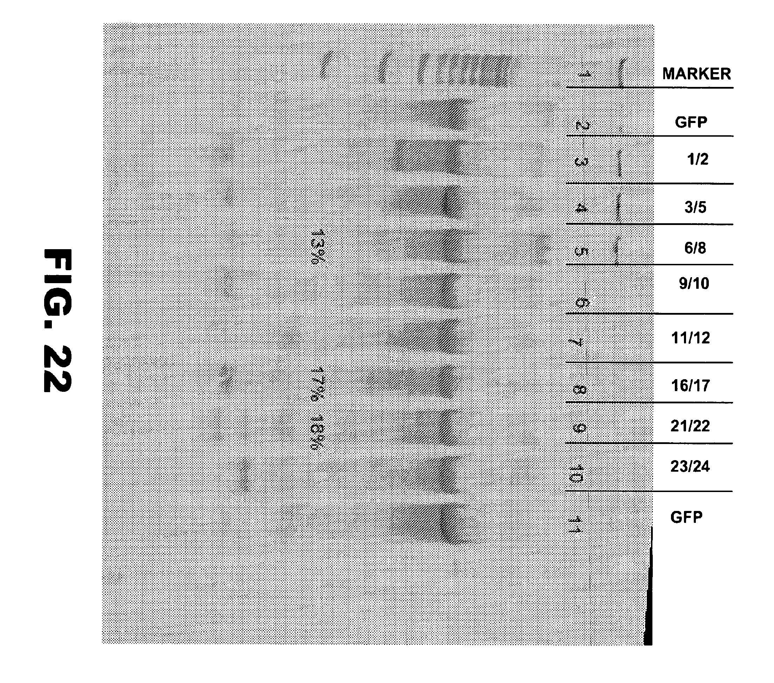

First, tagging the microtubule associated protein at the N-terminus was attempted. Multiple ZFNs were designed as described in Example 1 above to integrate tag sequences at the N-terminus of the microtubule-associated protein. ZFNs that successfully cut the chromosomal DNA near the MAPRE3 N-terminus were found (Pair 6/8 and 16/17; **Fig. 22** **and Table 2**). However, none of the ZFN pairs cut the chromosome at a location that was suitable for producing the desired tagged fusion protein.

| **Table 2** | | | | | | |
|---|---|---|---|---|---|---|
| ZFN pair | Total | Parent | Band one | Band two | % efficiency | |
| 1/2 | 3282 | 2495 | 787 | 0 | 24% | Extra bands outside of expected |
| 3/5 | 10187 | | | | 0 | No bands detected via densitometry |
| 6/8 | 3210 | 2803 | 210 | 197 | 13% | |
| 9/10 | | | | | 0 | No bands detected via densitometry |
| 11/12 | | | | | 0 | No bands detected via densitometry |
| 16/17 | 2647 | 2185 | 131 | 331 | 17% | |
| 21/22 | 2496 | 2056 | 160 | 280 | 18% | Slight multiple banding |
| 23/24 | | | | | 0 | No bands detected via densitometry |

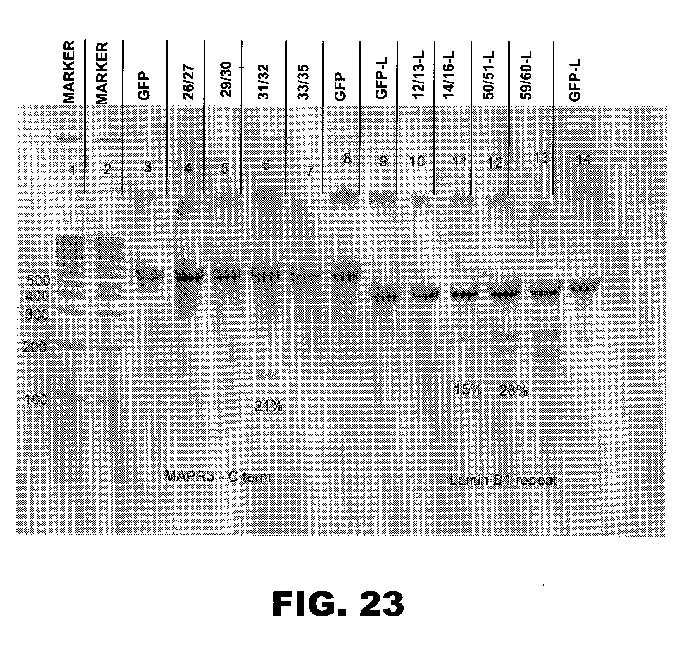

Since tagging the microtubule associated protein at the N-terminus was not successful, tagging the protein at the C-terminus was then attempted. Multiple ZFN pairs were designed to integrate tag sequences at the C-terminus of the microtubule-associated protein. As a control, ZFN pairs were also designed to integrate tag sequences at the N-terminus of a Lamin protein **(****Fig. 23** **and Table 4).** One ZFN pair that successfully cut the chromosomal DNA at or near the MAPRE3 C-terminus was found (pair 31/32; **Fig. 23** **and Table 3**). In this pair, one ZFN was designed to bind the 5' TTCCTCTCTCTCCCAC 3' (SEQ ID NO:11) sequence, and another ZNF was designed to bind the 5' AGGAAGGATTCGCAC 3' (SEQ ID NO:12) sequence comprising the MAPRE3 locus.

| **Table 3** | | | | | | |
|---|---|---|---|---|---|---|
| ZFN pair | Total | Parent | Band one | Band two | % efficiency | |
| 26/27 | | | | | 0 | No bands detected via densitometry |
| 29/30 | | | | | 0 | No bands detected via densitometry |
| 31/32 | 3448 | 2728 | 579 | 141 | 21% | Smaller band easily detected, 317 bp band barely detected |
| 33/35 | | | | | 0 | No bands detected via densitometry |

| **Table 4** | | | | | | |
|---|---|---|---|---|---|---|
| ZFN pair | Total | Parent | Band one | Band two | % efficiency | |
| 12/13-L | 5476 | 5172 | 304 | | 0 | No bands detected via densitometry |
| 14/16-L | 4093 | 3463 | 377 | 253 | 015% | No bands detected via densitometry |
| 50/51-L | 4722 | 3512 | 726 | 484 | 26% | Smaller band easily detected, 317 bp band barely detected |
| 59/60-L | 5726 | 4022 | 983 | 721 | 30% | No bands detected via densitometry |

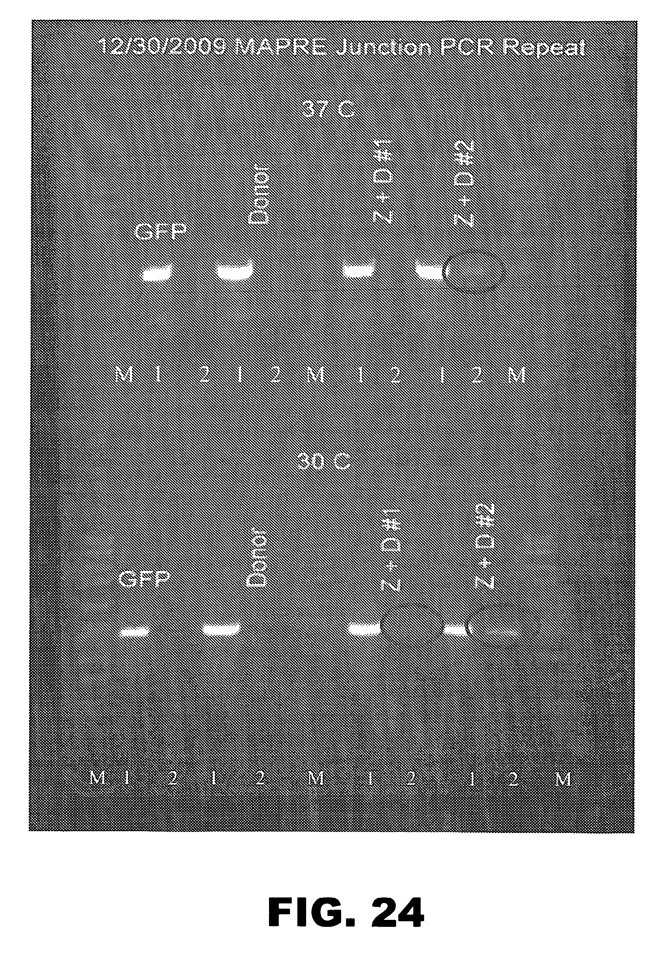

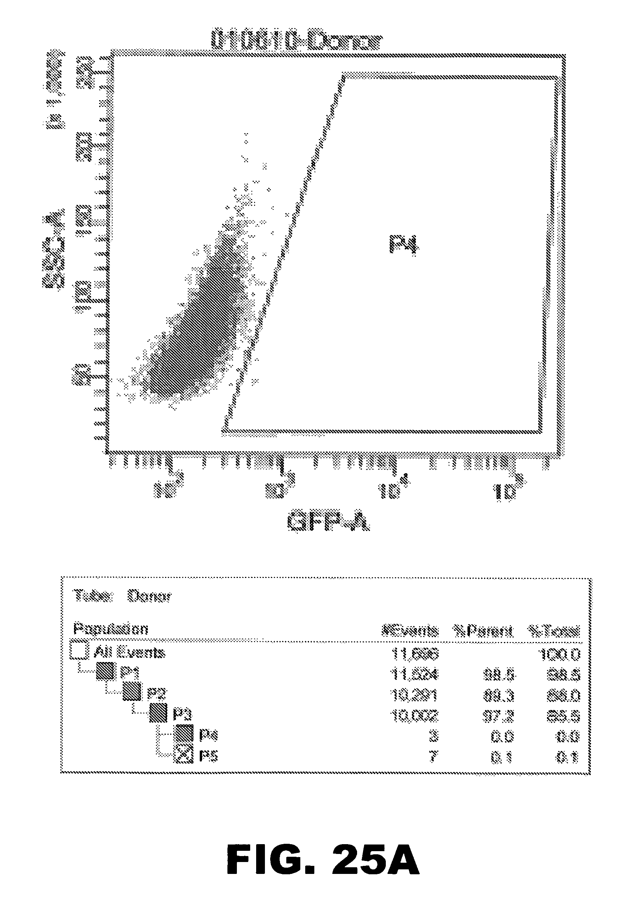

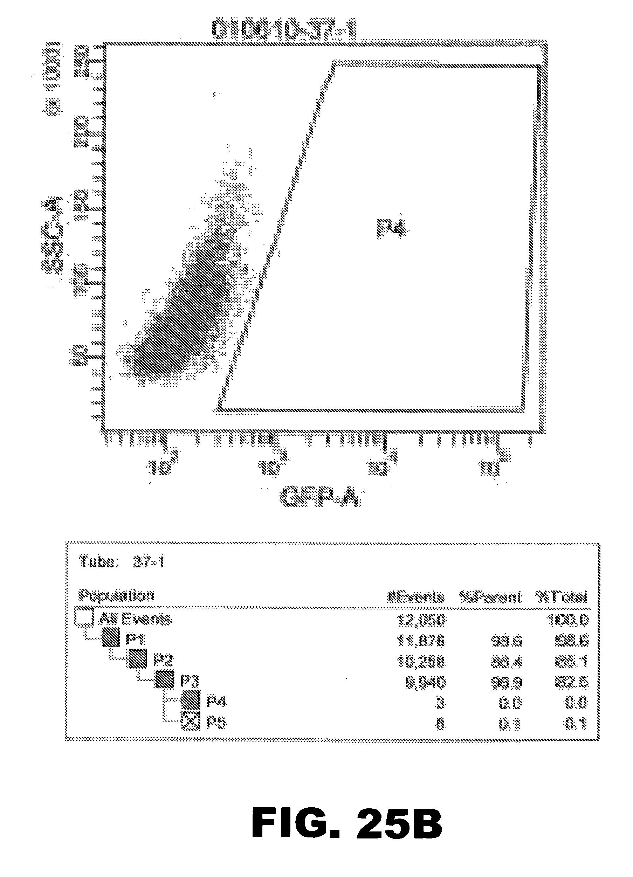

A donor plasmid comprising upstream and downstream MAPRE3 locus sequences flanking a polynucleotide encoding GFP was produced **(****Fig. 21****).** The donor plasmid and the 31/32 pair of RNAs encoding ZFNs were transfected into cells, and junction PCR showed a possible insertion of the GFP tag into the MAPRE3 locus **(****Fig. 24****).** However, FACS analysis showed that no fluorescent signal was detected, and therefore the targeted integration was not successful **(****Fig. 25****).**

### Example 4. Tagging endogenous β-actin protein.

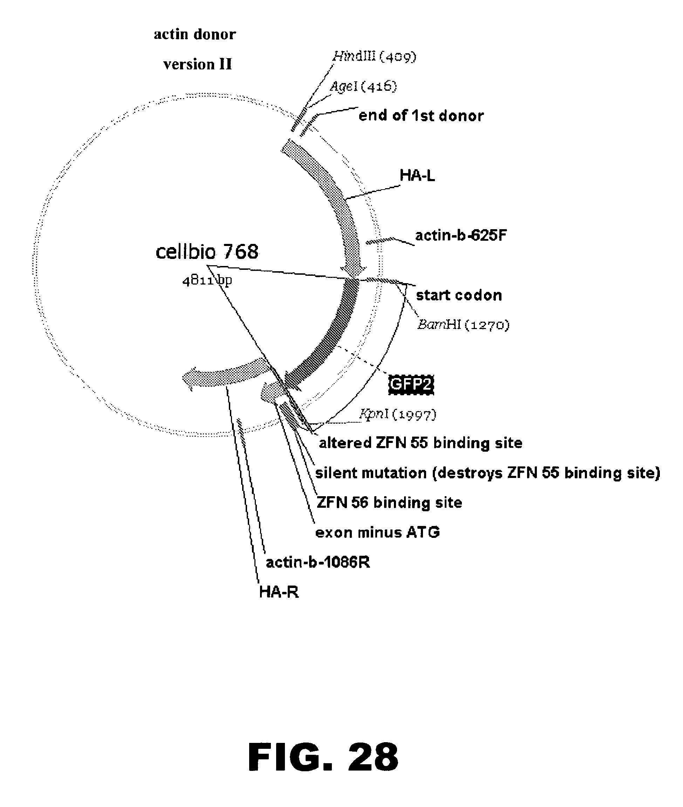

The endogenous β-actin protein was tagged with GFP using ZFN-induced homologous recombination. In short, ZFNs were used to introduce a double-stranded break in the chromosome region encoding β-actin encoded by the ACTB locus. The double stranded break induces homologous recombination with a donor polynucleotide comprising the GFP coding sequence flanked by nucleic acid sequences homologous to the ACTB locus chromosome region, and resulting in the integration of the GFP coding region into the chromosome. The donor polynucleotide **(****Fig. 28****)** was constructed to integrate the GFP tag in-frame with the β-actin coding sequence **(****Fig. 26****,** "v.2") to produce a protein tagged with GFP at the N-terminus **(****Fig. 26D****).** GFP-tagged β-actin protein was expressed under the control of the endogenous Actin promoter.

A pair of ZFNs was designed for the targeted integration of a tag into the ACTB target site, as detailed above.. For the ZFN pair used to tag β-actin, protein, one ZFN was designed to bind the 5' GTCGTCGACAACGGCTCC 3' (SEQ ID NO:13) sequence, and the other ZFN was designed to bind the 5' TGCAAGGCCGGCTTCGCGG 3' (SEQ ID NO:14) sequence **(****Fig. 26A****).** Upon binding, the ZFN pair introduces a double-stranded break in the GGCATG chromosome sequence between the recognition sites **(****Fig. 26A and 26B****)** to induce homologous recombination. Capped, polyadenylated mRNAs encoding the ZFN pair was then produced using known molecular biology techniques.

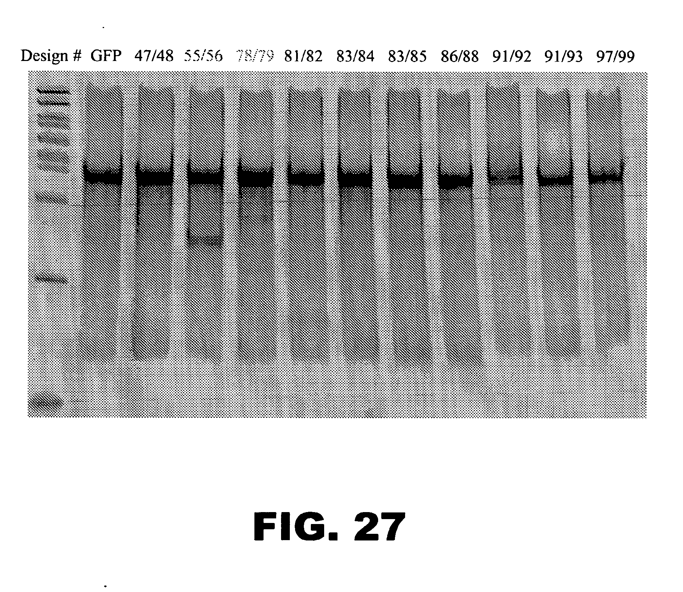

The frequency of targeted ZFN pair double stranded break generation in ZFN-treated pools of cells was determined by using the Cel-1 nuclease assay **(****Fig. 27****).** This assay detects alleles of the target locus that deviate from wild type as a result of non-homologous end joining (NHEJ)-mediated imperfect repair of ZFN-induced DNA double strand breaks. PCR amplification of the targeted region from a pool of ZFN-treated cells generates a mixture of WT and mutant amplicons. Melting and reannealing of this mixture results in mismatches forming between heteroduplexes of the WT and mutant alleles. A DNA "bubble" formed at the site of mismatch is cleaved by the surveyor nuclease Cel-1, and the cleavage products can be resolved by gel electrophoresis. The relative intensity of the cleavage products compared with the parental band is a measure of the level of Cel-1 cleavage of the heteroduplex. This, in turn, reflects the frequency of ZFN-mediated cleavage of the endogenous target locus that has subsequently undergone imperfect repair by NHEJ.

A plasmid **(****Fig. 28****)** was constructed as a polynucleotide donor for the targeted integration of a GFP tag into the ACTB locus of the human cell line. The plasmid comprised the GFP coding sequence flanked by 861 and 593 nucleotides of ACTB locus sequences upstream and downstream of the cut site introduced by the ZFN pair **(****Fig. 26C****).** The tag sequence in the plasmid was fused to the upstream and downstream sequences of the ACTB locus in such a manner that, when the ACTB locus is expressed, a β-actin protein fused to the GFP tag at the N-terminus is produced as detailed in **Fig 26D****.** The GFP-Actin fusion was also designed such that the splice signal of the first exon of the ACTB locus, where the GFP coding sequence was introduced, was kept intact.

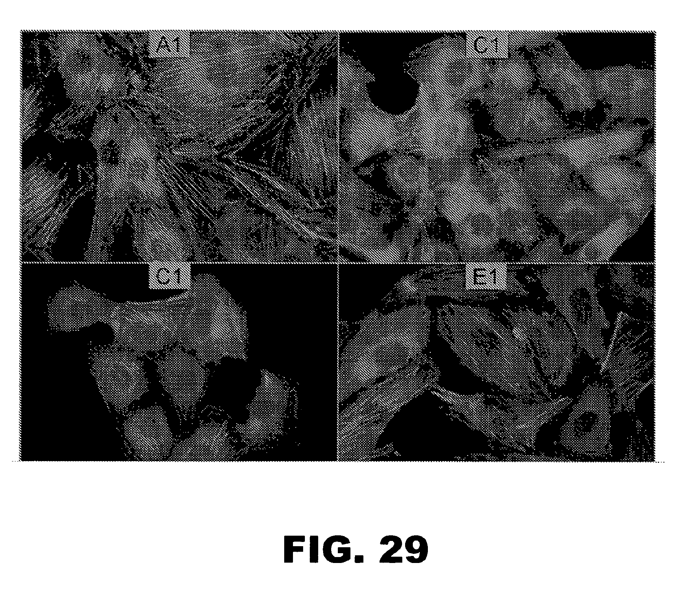

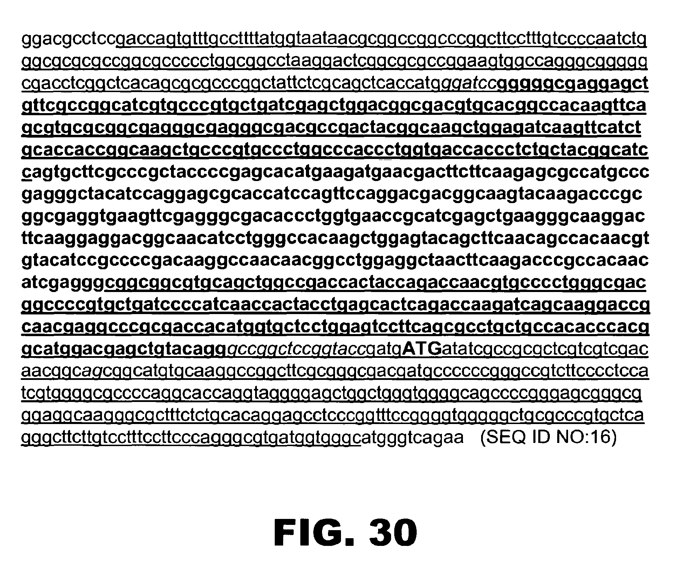

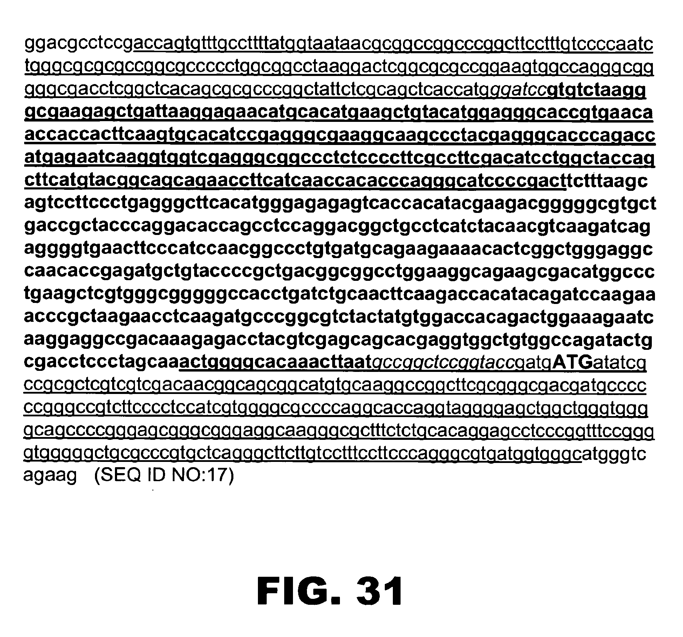

The donor plasmid, and the pair of RNAs encoding ZFNs were transfected into cells. The nucleic acid mixture comprised one part donor DNA to one part ZFN RNAs. The transfected cells were then cultured and individual cell clones were analyzed. Fluorescent microscopy was used to visualize the GFP-tagged β-actin protein **(****Fig. 29****).** The confirmed sequence of the ACTB locus with the GFP2 integration in U2OS cells is shown in **Fig. 30** (SEQ ID NO: 16). The confirmed sequence of the ACTB locus with the RFP integration in U2OS cells is shown in **Fig. 31** (SEQ ID NO: 17).

### Example 5. GFP-tagged β-actin utilizing the 2A peptide.

β-actin was also tagged at the N-terminus with GFP while simultaneously replacing the nucleic acid sequence encoding the first 15 amino acids of β-actin with a nucleic acid sequence with alternate codon usage.

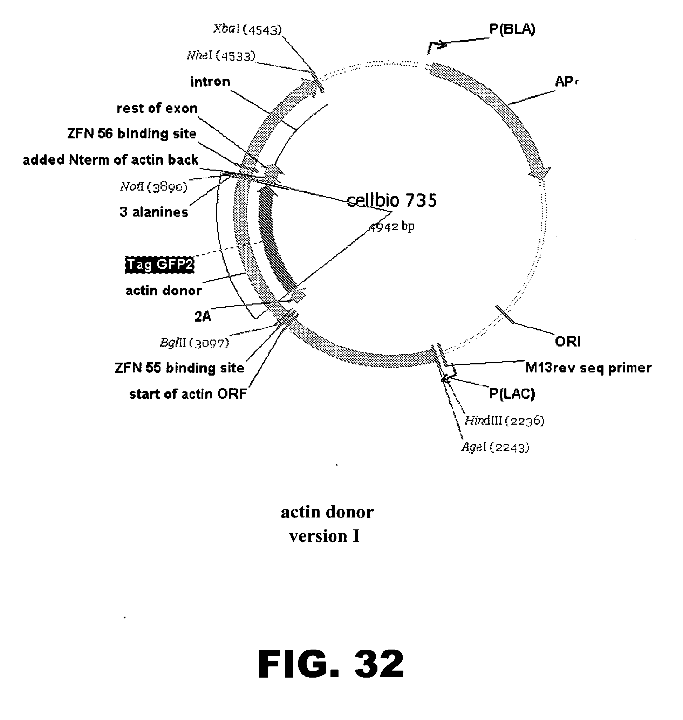

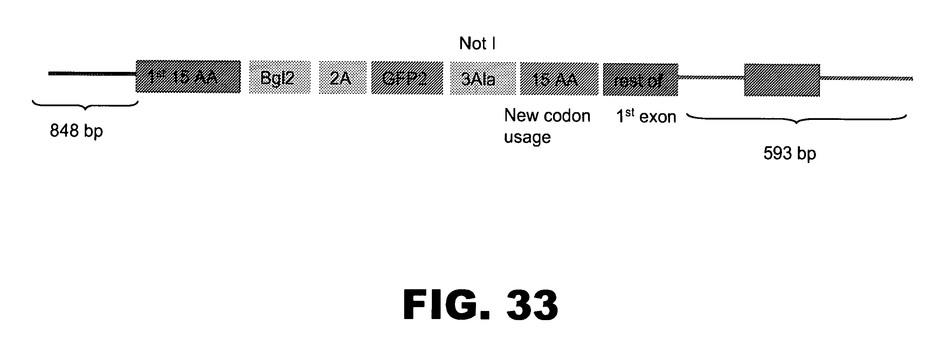

To integrate a tag sequence near the ZFN cut site (**Fig. 26****,** "v.1") which would result in full length β-actin translationally fused to GFP, a new donor plasmid was created in which the first 15 amino acids of β-actin were changed **(****Fig. 32****).** The donor plasmid comprised upstream and downstream ACTB locus sequences flanking a polynucleotide encoding 2a peptide fused to GFP which was in turn fused through a 3 alanine amino acid residue linker to the first 15 amino acids of β-actin encoded by alternate codons **(****Fig. 33****).** Co-translational cleavage of the 2a peptide removes the first 15 amino acids of β-actin encoded by the new codons, producing a β-actin protein tagged with GFP at the N-terminus **(****Fig. 26D****).**

ZFNs were as described in Example 4. The donor plasmid, and the pair of RNAs encoding ZFNs were transfected into cells. The nucleic acid mixture comprised one part donor DNA to one part ZFN RNAs. The transfected cells were then cultured and individual cell clones were analyzed. Fluorescent microscopy was used to confirm expression of the GFP-tagged β-actin protein **(****Fig. 29****).**

### Example 6. Tagging endogenous Lamin B1 protein.

The endogenous Lamin B1 protein was tagged with GFP using ZFN-induced homologous recombination. In short, ZFNs were used to introduce a double-stranded break in the chromosome region encoding Lamin B1 encoded by the LMNB1 locus. The double stranded break induces homologous recombination with a donor polynucleotide comprising the GFP coding sequence flanked by nucleic acid sequences homologous to the LMNB1 locus chromosome region, and resulting in the integration of the GFP coding region into the chromosome. The donor polynucleotide was constructed to fuse the GFP tag in-frame with the Lamin B1 coding sequence to produce a protein tagged with GFP at the N-terminus. GFP-tagged Lamin B1 protein was expressed under the control of the endogenous Lamin promoter.

A pair of ZFNs was designed as described above. The frequency of targeted ZFN pair double stranded break generation in ZFN-treated pools of cells was determined by using the Cel-1 nuclease assay. For the ZFN pair used to tag Lamin B1 protein, one ZFN was designed to bind the 5' CCTCGCCGCCCCGCT 3' (SEQ ID NO:18) sequence, and the other ZFN was designed to bind the 5' GCCGCCCGCCATGGCG 3' (SEQ ID NO:19) sequence **(****Fig. 34A****).** Upon binding, the ZFN pair introduces a double-stranded break in the GTCTCC chromosome sequence between the recognition sites **(****Fig. 34A and 34B****)** to induce homologous recombination. Capped, polyadenylated mRNAs encoding the ZFN pair was then produced using known molecular biology techniques.

A plasmid was constructed as a polynucleotide donor for the targeted integration of a GFP tag into the LMNB1 locus of the U2OS human cell line. The plasmid comprised the GFP coding sequence flanked by 633Kb and 629 base pairs of LMNB1 locus sequence upstream and downstream of the cut site introduced by the ZFN pair **(****Fig. 34C and 34D****).** The tag sequence in the plasmid was fused to the upstream and downstream the LMNB1 locus in such a manner that, when the LMNB1 locus was expressed, a Lamin B1 protein fused to the GFP tag at the N-terminus was produced as detailed in **Fig 34E****.**

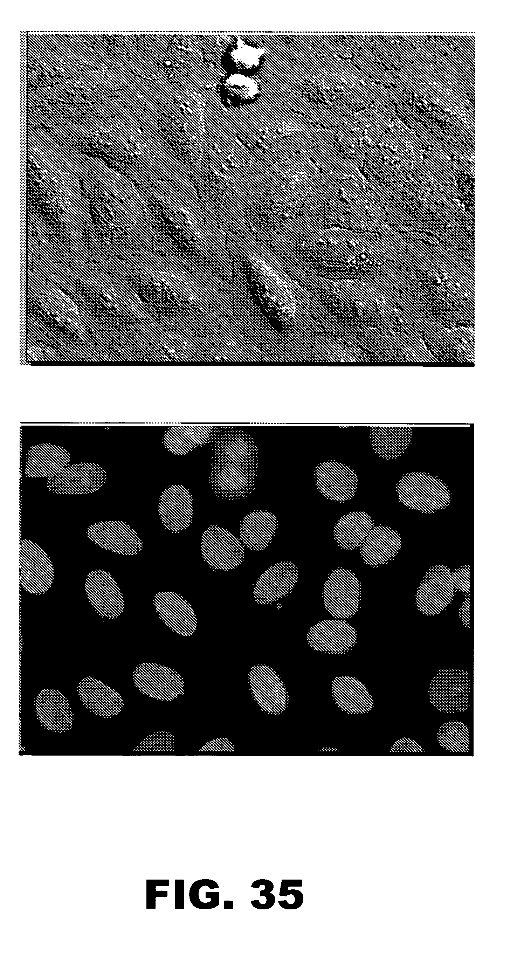

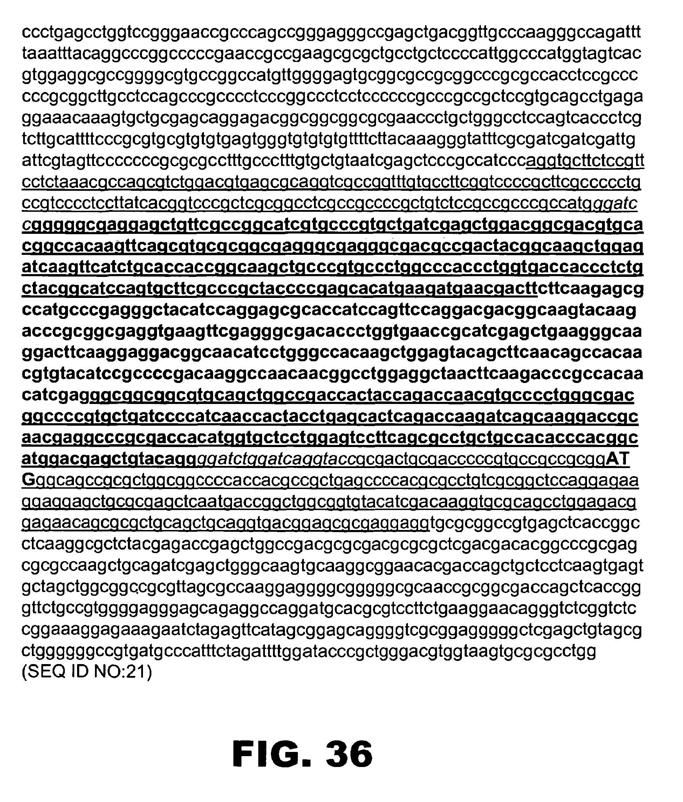

The donor plasmid, and the pair of RNAs encoding ZFNs were transfected into cells. The nucleic acid mixture comprised one part donor DNA to one part ZFN RNAs. The transfected cells were then cultured and individual cell clones were analyzed. Junction PCR performed at 37°C and 30°C was used to confirm the donor DNA was integrated in the Lamin LMNB1 locus. Fluorescent microscopy was then used to visualize the GFP-tagged Lamin B1 protein **(****Fig. 35****).** The confirmed sequence at the site of integration of GFP2 in the lamin coding region in U2OS cells is shown in **Fig. 36** (SEQ ID NO: 21).

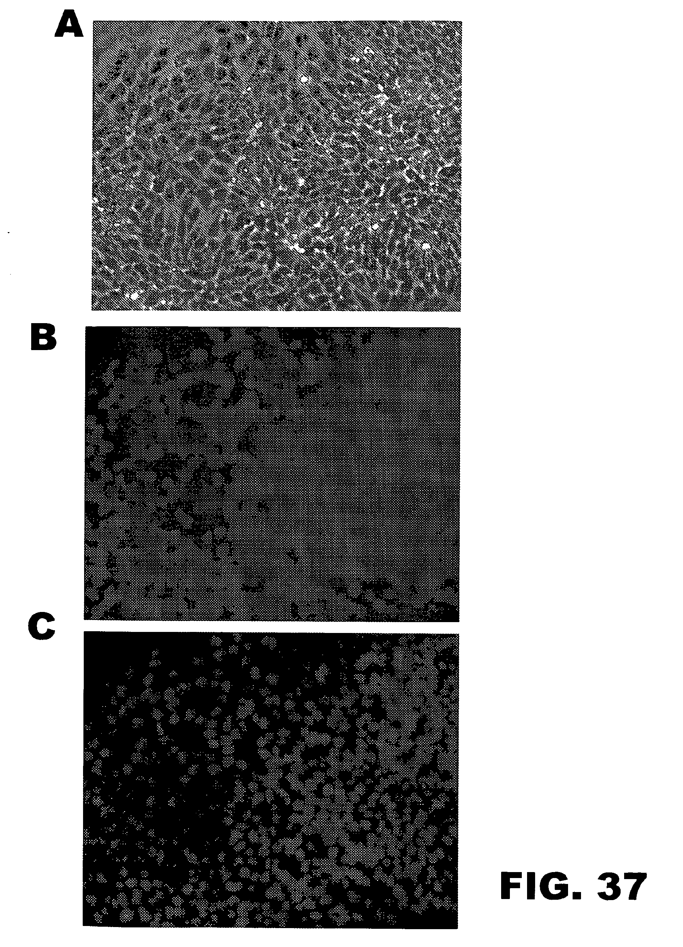

A donor plasmid comprising RFP coding sequence and flanking lamin sequences, and the pair of RNAs encoding ZFNs were also transfected into iPS cells, which are induced pluripotent stem cells generated from fibroblasts or other cell types. Images of iPS cells comprising RFP-tagged lamin are shown in **Fig 37****.**

### Example 7. Tagging endogenous HER2 protein.

The endogenous HER2 protein was tagged with GFP using ZFN-induced homologous recombination. In short, ZFNs were used to introduce a double-stranded break in the chromosome region encoding HER2 encoded by the ERBB2 gene locus. The double stranded break induces homologous recombination with a donor polynucleotide comprising the GFP coding sequence flanked by nucleic acid sequences homologous to the ERBB2 locus chromosome region, and resulting in the integration of the GFP coding region into the chromosome. The donor polynucleotide was constructed to fuse the GFP tag in-frame with the HER2 coding sequence to produce a protein tagged with GFP at the N-terminus. GFP-tagged HER2 protein was expressed under the control of the endogenous ERBB2 promoter.

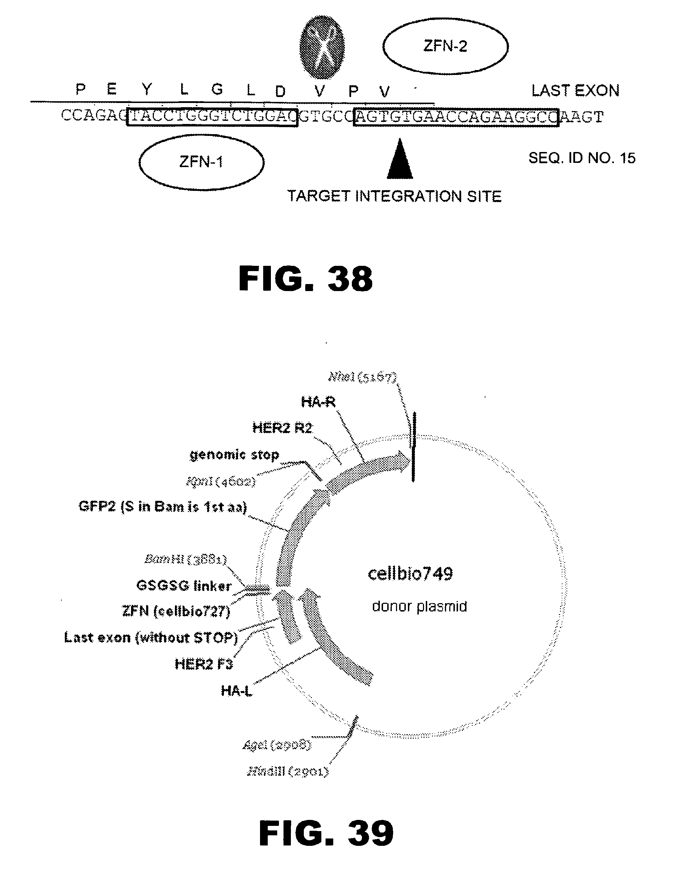

A pair of ZFNs was designed as described above. The frequency of targeted ZFN pair double stranded break generation in ZFN-treated pools of cells was determined by using the Cel-1 nuclease assay. For the ZFN pair used to tag HER2 protein, one ZFN was designed to bind the 5' TACCTGGGTCTGGAC 3' (SEQ ID NO:22) sequence, and the other ZFN was designed to bind the 5' AGTGTGAACCAGAAGGCC 3' (SEQ ID NO:23) sequence. Upon binding, the ZFN pair introduces a double-stranded break in the GTGCC chromosome sequence between the recognition sites **(****Fig. 38****)** to induce homologous recombination. Capped, polyadenylated mRNAs encoding the ZFN pair was then produced using known molecular biology techniques.

A plasmid was constructed as a polynucleotide donor for the targeted integration of a GFP tag into the ERBB2 locus **(****Fig. 39****).** The tag sequence in the plasmid was fused to the upstream and downstream the ERBB2 locus in such a manner that, when the ERBB2 locus was expressed, a HER2 protein fused to the GFP tag at the N-terminus was produced.

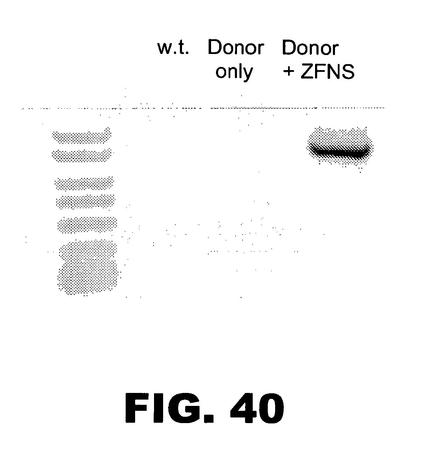

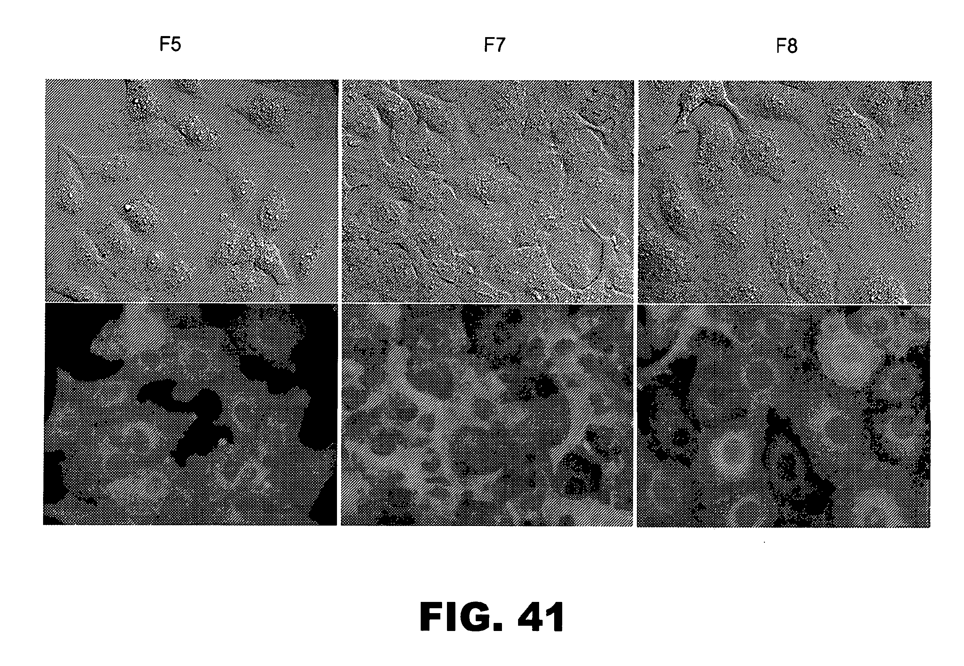

The donor plasmid, and the pair of RNAs encoding ZFNs were transfected into SKOV3 cells The nucleic acid mixture comprised one part donor DNA to one part ZFN RNAs. The transfected cells were then cultured and individual cell clones were analyzed. Junction PCR performed at 37°C and 30°C was used to confirm the donor DNA was integrated in the ERBB2 locus in transfected SKOV3 cells **(****Fig. 40****).** Fluorescent microscopy was then used to visualize the GFP-tagged HER2 protein **(****Fig. 41****).**

### Example 8. Tagging endogenous HMGA protein.

The HMGA protein was tagged with GFP using ZFN-induced homologous recombination. In short, ZFNs were used to introduce a double-stranded break in the chromosome region encoding HMGA encoded by the HMGA1 locus. The double stranded break induces homologous recombination with a donor polynucleotide comprising the GFP coding sequence flanked by nucleic acid sequences homologous to the HMGA1 locus chromosome region, and resulting in the integration of the GFP coding region into the chromosome. The donor polynucleotide was constructed to fuse the GFP tag in-frame with the HMGA1 coding sequence to produce a protein tagged with GFP at the N-terminus. GFP-tagged HMGA1 protein was expressed under the control of the endogenous HMGA1 promoter.

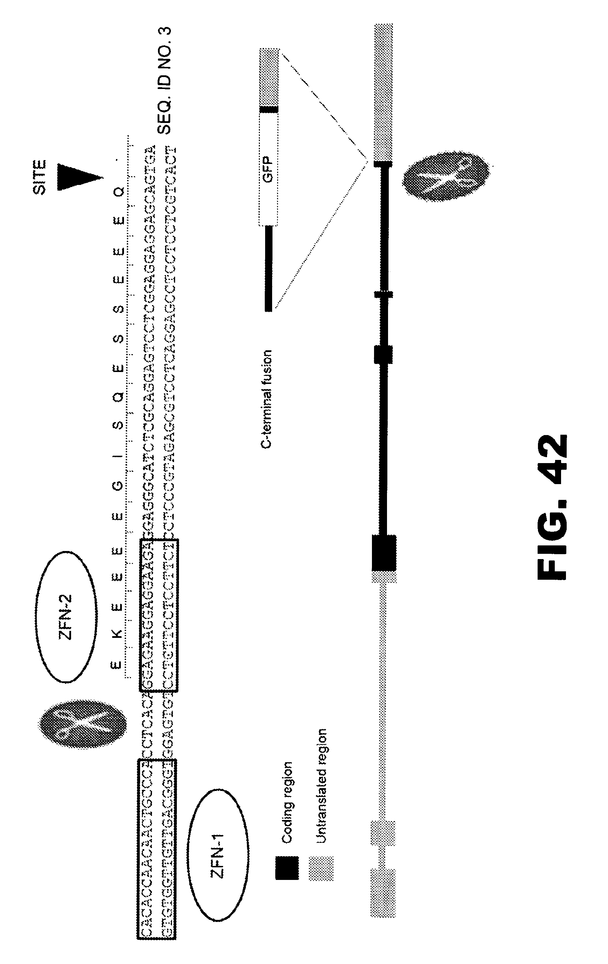

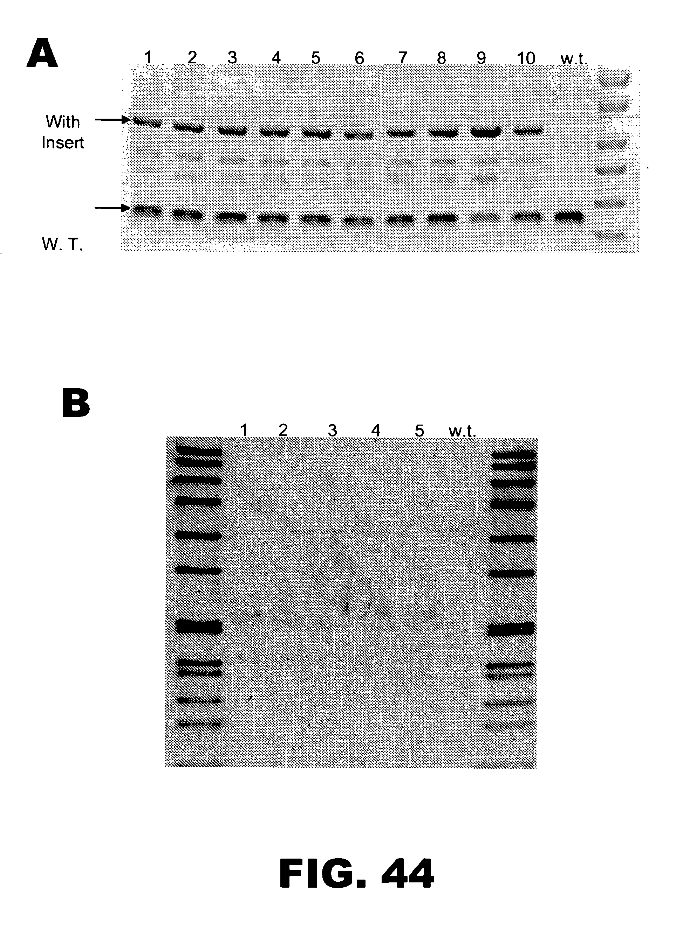

A pair of ZFNs was designed as described above. to tag the endogenous HMG1 protein. One ZFN was designed to bind the 5' CACACCAACAACTGCCCA 3' (SEQ ID NO:25) sequence, and the other ZFN was designed to bind the 5' GGAGAAGGAGGAAGA 3' (SEQ ID NO:26) sequence **(****Fig. 42****).** Upon binding, the ZFN pair introduces a double-stranded break in the CCTCACA chromosome sequence between the recognition sites **(****Fig. 44****)** to induce homologous recombination. Capped, polyadenylated mRNAs encoding the ZFN pair was then produced using known molecular biology techniques.

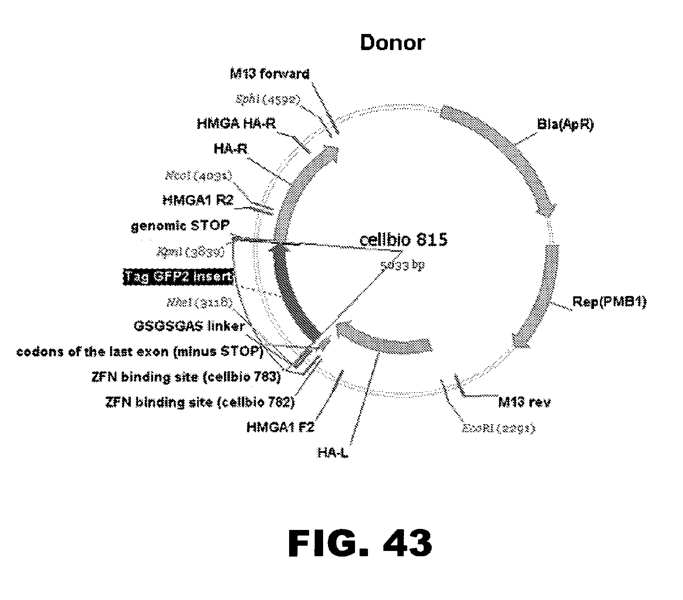

A plasmid was constructed as a polynucleotide donor for the targeted integration of a GFP tag into the HMGA1 locus **(****Fig. 43****).** The plasmid comprised the GFP coding sequence flanked by 806 base pairs and 747 base pairs of HMGA1 locus sequence upstream and downstream of the cut site introduced by the ZFN pair **(****Fig. 43****).** The tag sequence in the plasmid was fused to the upstream and downstream the HMGA1 locus in such a manner that, when the HMGA1 locus was expressed, a HMGA protein fused to the GFP tag at the N-terminus was produced.

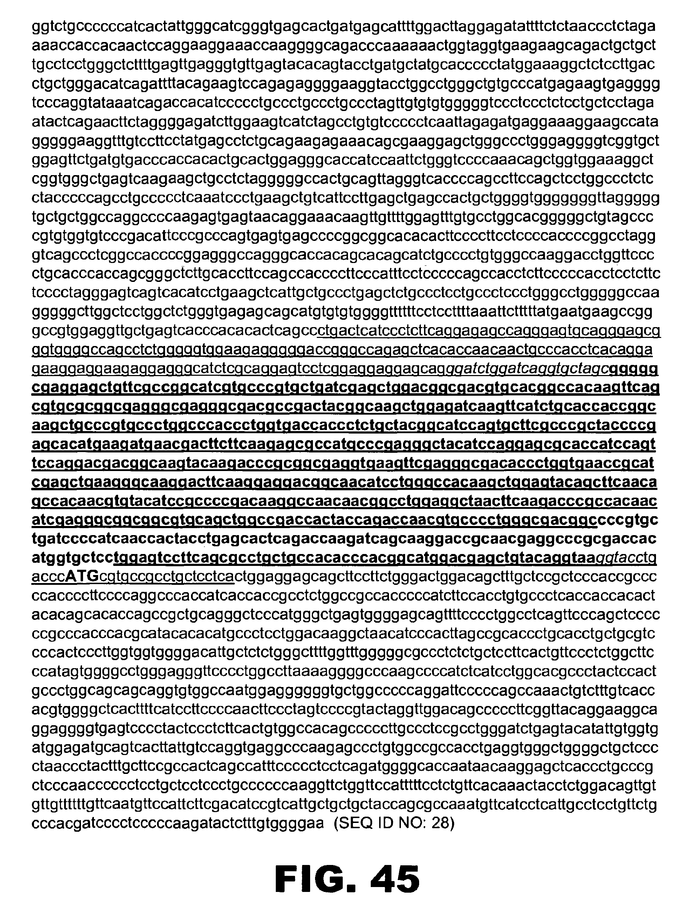

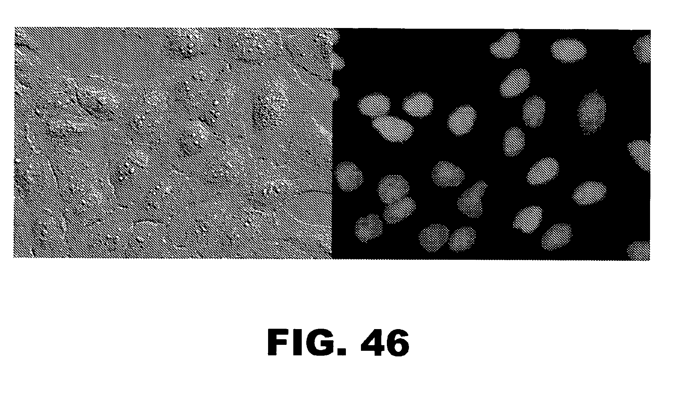

The donor plasmid, and the pair of RNAs encoding ZFNs were transfected into U2OS cells. The nucleic acid mixture comprised one part donor DNA to one part ZFN RNAs. The transfected cells were then cultured and individual cell clones were analyzed. Genomic PCR and Southern blotting indicated the integration of the tag sequence into the HMGA1 locus in selected clones **(****Fig. 44A and Fig. 44B****)** Sequence analysis confirmed integration into the targeted chromosomal region **(****Fig. 45****)** (SEQ ID NO: 28). Fluorescent microscopy was then used to visualize the GFP-tagged HMGA1 protein **(****Fig. 46****).**

## Claims

1. A method for tagging at least one endogenous protein, the method comprising:
a) introducing into a mammalian cell (i) at least one targeting endonuclease or nucleic acid encoding a targeting endonuclease, the targeting endonuclease binding a target site and able to cleave a cleavage site in a chromosomal sequence encoding the endogenous protein, and (ii) at least one donor polynucleotide comprising a tag sequence, the tag sequence being flanked by an upstream sequence and a downstream sequence, the upstream sequence and the downstream sequence sharing substantial sequence identity with either side of the cleavage site in the chromosomal sequence; and
b) maintaining the cell under conditions such that a double-stranded break introduced at the cleavage site by the targeting endonuclease is repaired by a homology-directed process such that the tag sequence in the donor polynucleotide is integrated in-frame into the chromosomal sequence encoding the endogenous protein such that a tagged endogenous protein is produced;
wherein the endogenous protein is chosen from actin, tubulin, lamin, human epidermal growth factor receptor 2 (HER2), and high-mobility group A protein (HMGA).

2. The method of claim 1, wherein the targeting endonuclease is a zinc finger nuclease.

3. The method of claim 1, wherein the cell is a human U2OS cell, a human MCF10A cell, a human SKOV3 cell, or a human iPS cell.

4. The method of claim 1, wherein the endogenous protein is tagged at the C terminus or at the N terminus.

5. The method of claim 2, wherein the zinc finger nuclease binds to a sequence having at least about 80% sequence identity to a sequence chosen from SEQ ID NO:13, 14, 1, 2, 18, 19, 22, 23, 25 and 26.

6. A mammalian cell comprising at least one tag sequence integrated in-frame into a chromosomal sequence encoding an endogenous protein, such that the cell expresses at least one tagged endogenous protein, wherein the endogenous protein is chosen from actin, tubulin, lamin, human epidermal growth factor receptor 2 (HER2), and high-mobility group A protein (HMGA).

7. The cell of claim 6, wherein the cell is a human U2OS cell, a human MCF10A cell, a human SKOV3 cell, or a human iPS cell.

8. The cell of claim 6, wherein the endogenous protein is tagged at the C terminus or at the N terminus.

9. The cell of claim 6, wherein the cell expresses one or more fluorescently tagged endogenous proteins.

10. The cell of claim 6, wherein the cell is produced by:
a) introducing into a parental cell (i) at least one targeting endonuclease or nucleic acid encoding a targeting endonuclease, the targeting endonuclease binding a target site and able to cleave a cleavage site in a chromosomal sequence encoding the endogenous protein, and (ii) at least one donor polynucleotide comprising a tag sequence, the tag sequence being flanked by an upstream sequence and a downstream sequence, the upstream sequence and the downstream sequence sharing substantial sequence identity with either side of the cleavage site in the chromosomal sequence; and
b) maintaining the cell under conditions such that a double-stranded break introduced at the cleavage site by the targeting endonuclease is repaired by a homology-directed process such that the tag sequence in the donor polynucleotide is integrated in-frame into the chromosomal sequence encoding the endogenous protein.

11. The cell of claim 10, wherein the targeting endonuclease is a zinc finger nuclease.

12. The cell of claim 11, wherein the zinc finger nuclease binds to a sequence having at least about 80% sequence identity to a sequence chosen from SEQ ID NO:13, 14, 1, 2, 18, 19, 22, 23, 25 and 26.

13. The method of claim 5 or the cell of claim 12, wherein the sequence identity is about 85%, 90%, 95%, 99%, or 100%.

## Patentansprüche

1. Verfahren zum Markieren wenigstens eines endogenen Proteins, wobei das Verfahren umfasst:
a) Einführen in eine Säugerzelle von (i) zumindest einer zielgerichteten Endonuklease oder einer eine zielgerichtete Endonuklease kodierenden Nukleinsäure, wobei die zielgerichtete Endonuklease eine Zielstelle bindet und in der Lage ist, eine Spaltungsstelle in eine das endogene Protein kodierende chromosomale Sequenz zu schneiden, und (ii) zumindest einem Donor-Polynukleotid, welches eine Markierungssequenz umfasst, wobei die Markierungssequenz flankiert wird von einer stromaufwärts liegenden Sequenz und einer stromabwärts liegenden Sequenz, wobei die stromaufwärts liegende Sequenz und die stromabwärts liegende Sequenz mit beiden Seiten der Spaltungsstelle in der chromosomalen Sequenz eine wesentliche Sequenzidentität aufweisen; und
b) Halten der Zelle unter derartigen Bedingungen, dass ein von der zielgerichteten Endonuklease an der Spaltungsstelle eingeführter Doppelstrangbruch derart von einem Homologie-gelenkten Prozess repariert wird, dass die Markierungssequenz in dem Donor-Polynukleotid in-frame in die das endogene Protein kodierende chromosomale Sequenz so eingefügt wird, dass ein markiertes endogenes Protein erzeugt wird;
wobei das endogene Protein ausgewählt ist aus Actin, Tubulin, Lamin, humanem epidermalem Wachstumsfaktor-Rezeptor 2 (HER2), und High-Mobility Group A-Protein (HMGA).

2. Verfahren nach Anspruch 1, wobei die zielgerichtete Endonuklease eine Zinkfingernuklease ist.

3. Verfahren nach Anspruch 1, wobei die Zelle eine humane U2OS-Zelle, eine humane MCF10A-Zelle, eine humane SKOV3-Zelle oder eine menschliche iPS-Zelle ist.

4. Verfahren nach Anspruch 1, wobei das endogene Protein am C-Terminus oder am N-Terminus markiert wird.

5. Verfahren nach Anspruch 2, wobei die Zinkfingernuklease an eine Sequenz bindet, welche mindestens etwa 80 % Sequenzidentität mit einer aus SEQ ID NO:13, 14, 1, 2, 18, 19, 22, 23, 25 und 26 ausgewählten Sequenz aufweist.

6. Säugerzelle, umfassend zumindest eine Markierungssequenz, welche in-frame in eine ein endogenes Protein kodierende chromosomale Sequenz so eingefügt wurde, dass die Zelle zumindest ein markiertes endogenes Protein exprimiert, wobei das endogene Protein ausgewählt ist aus Actin, Tubulin, Lamin, humanem epidermalem Wachstumsfaktor-Rezeptor 2 (HER2) und High-Mobility Group A-Protein (HMGA).

7. Zelle nach Anspruch 6, wobei die Zelle eine humane U2OS-Zelle eine humane MCF10A-Zelle, eine humane SKOV3-Zelle oder eine humane iPS-Zelle ist.

8. Zelle nach Anspruch 6, wobei das endogene Protein am C-Terminus oder am N-Terminus markiert ist.

9. Zelle nach Anspruch 6, wobei die Zelle ein oder mehrere floureszenzmarkierte endogene Proteine exprimiert.

10. Zelle nach Anspruch 6, wobei die Zelle hergestellt ist durch:
a) Einführen in eine Mutterzelle von (i) zumindest einer zielgerichteten Endonuklease oder einer eine zielgerichtete Endonuklease kodierenden Nukleinsäure, wobei die zielgerichtete Endonuklease eine Zielstelle bindet und in der Lage ist, eine Spaltungsstelle in eine das endogene Protein kodierende chromosomale Sequenz zu schneiden, und (ii) zumindest einem Donor-Polynukleotid, welches eine Markierungssequenz umfasst, wobei die Markierungssequenz flankiert wird von einer stromaufwärts liegenden Sequenz und einer stromabwärts liegenden Sequenz, wobei die stromaufwärts liegende Sequenz und die stromabwärts liegende Sequenz mit beiden Seiten der Spaltungsstelle in der chromosomalen Sequenz eine wesentliche Sequenzidentität aufweisen; und
b) Halten der Zelle unter derartigen Bedingungen, dass ein von der zielgerichteten Endonuklease an der Spaltungsstelle eingeführter Doppelstrangbruch derart von einem Homologie-gelenkten Prozess repariert wird, dass die Markierungssequenz in dem Donor-Polynukleotid in-frame in die das endogene Protein kodierende chromosomale Sequenz eingefügt wird.

11. Zelle nach Anspruch 10, wobei die zielgerichtete Endonuklease eine Zinkfingernuklease ist.

12. Zelle nach Anspruch 11, wobei die Zinkfingernuklease an eine Sequenz bindet, welche mindestens etwa 80 % Sequenzidentität mit einer aus SEQ ID NO:13, 14, 1, 2, 18, 19, 22, 23, 25 und 26 ausgewählten Sequenz aufweist.

13. Verfahren nach Anspruch 5 oder Zelle nach Anspruch 12, wobei die Sequenzidentität ungefähr 85 %, 90 %, 95 %, 99 % oder 100 % beträgt.

## Revendications

1. Procédé d'étiquetage d'au moins une protéine endogène, le procédé comprenant :
a) l'introduction dans une cellule de mammifère de (i) au moins une endonucléase de ciblage ou un acide nucléique codant pour une endonucléase de ciblage, l'endonucléase de ciblage se liant à un site cible et étant capable de cliver un site de clivage dans une séquence chromosomique codant pour la protéine endogène, et de (ii) au moins un polynucléotide donneur comprenant une séquence d'étiquette, la séquence d'étiquette étant encadrée par une séquence amont et une séquence aval, la séquence amont et la séquence aval partageant une identité de séquence substantielle avec l'un ou l'autre côté du site de clivage dans la séquence chromosomique ; et
b) le maintien de la cellule dans des conditions telles qu'une coupure double brin introduite dans le site de clivage par l'endonucléase de ciblage est réparée par un processus homologique de façon homologue que la séquence d'étiquette dans le polynucléotide donneur soit intégrée dans le cadre de lecture de la séquence chromosomique codant pour la protéine endogène et qu'une protéine endogène étiquetée soit produite ;
dans lequel la protéine endogène est choisie parmi l'actine, la tubuline, la lamine, le récepteur 2 du facteur de croissance épidermique humain (HER2), et une protéine à haute mobilité du groupe A (HMGA).

2. Procédé selon la revendication 1, dans lequel l'endonucléase de ciblage est une nucléase à doigt de zinc.

3. Procédé selon la revendication 1, dans lequel la cellule est une cellule U2OS humaine, une cellule MCF10A humaine, une cellule SKOV3 humaine, ou une cellule iPS humaine.

4. Procédé selon la revendication 1, dans lequel la protéine endogène est étiquetée à son extrémité C-terminale ou à son extrémité N-terminale.

5. Procédé selon la revendication 2, dans lequel la nucléase à doigt de zinc se lie à une séquence présentant une identité de séquence d'au moins environ 80 % avec une séquence choisie parmi SEQ ID No. : 13, 14, 1, 2, 18, 19, 22, 23, 25 et 26.

6. Cellule de mammifère comprenant au moins une séquence d'étiquetée intégrée dans le cadre de lecture d'une séquence chromosomique codant pour une protéine endogène, de façon que la cellule exprime au moins une protéine endogène étiquetée, la protéine endogène étant choisie parmi l'actine, la tubuline, la lamine, le récepteur 2 du facteur de croissance épidermique humain (HER2), et une protéine à haute mobilité du groupe A (HMGA).

7. Cellule selon la revendication 6, dans laquelle la cellule est une cellule U2OS humaine, une cellule MCF10A humaine, une cellule SKOV3 humaine, ou une cellule iPS humaine.

8. Cellule selon la revendication 6, dans laquelle la protéine endogène est étiquetée à son extrémité C-terminale ou à son extrémité N-terminale.

9. Cellule selon la revendication 6, dans laquelle la cellule exprime une ou plusieurs protéines endogènes étiquetées par fluorescence.

10. Cellule selon la revendication 6, dans laquelle la cellule est produite par :
a) introduction dans une cellule parentale de (i) au moins une endonucléase de ciblage ou un acide nucléique codant pour une endonucléase de ciblage, l'endonucléase de ciblage se liant à un site cible et étant capable de cliver un site de clivage dans une séquence chromosomique codant pour la protéine endogène, et de (ii) au moins un polynucléotide donneur comprenant une séquence d'étiquette, la séquence d'étiquette étant encadrée par une séquence amont et une séquence aval, la séquence amont et la séquence aval partageant une identité de séquence substantielle avec l'un ou l'autre côté du site de clivage dans la séquence chromosomique ; et
b) maintien de la cellule dans des conditions telles qu'une cassure double brin introduite dans le site de clivage par l'endonucléase de ciblage est réparée par un processus homologique de façon que la séquence d'étiquette dans le polynucléotide donneur soit intégrée dans le cadre de lecture de la séquence chromosomique codant pour la protéine endogène.

11. Cellule selon la revendication 10, dans laquelle l'endonucléase de ciblage est une nucléase à doigt de zinc.

12. Cellule selon la revendication 11, dans laquelle la nucléase à doigt de zinc se lie à une séquence présentant une identité de séquence d'au moins environ 80 % avec une séquence choisie parmi SEQ ID No. : 13, 14, 1, 2, 18, 19, 22, 23, 25 et 26.

13. Procédé selon la revendication 5 ou cellule selon la revendication 12, dans lequel ou laquelle l'identité de séquence est d'environ 85, 90, 95, 99, ou 100 %.
